# EUROPEAN PATENT APPLICATION

(11) **EP 4 464 378 A2**
(43) Date of publication of application: **20.11.2024**
(21) Application number: 24188941.9
(22) Date of filing: 02.03.2020
(51) Int. Cl.: A61P 29/00

(54) **LEUCINE, ACETYL LEUCINE, AND RELATED ANALOGS FOR TREATING DISEASE**

(30) Priority: 02.03.2019 US 201962812987 P; 02.05.2019 US 201962842296 P; 19.08.2019 US 201962888894 P; 03.09.2019 US 201962895144 P
(62) Divisional of application: 20712029.6
(71) Applicant: IntraBio Ltd, London NW3 6BP (GB)
(72) Inventor: FACTOR, Mallory, Mayfair, W1J 5LF (GB); FIELDS, Taylor, Mayfair, W1J 5LF (GB)
(74) Representative: Maiwald GmbH

(57) **Abstract**

The present disclosure provides methods of treating diseases, disorders, conditions, or syndromes, e.g., traumatic brain injury, in a patient in need thereof by administering a therapeutically effective amount of DL-leucine, or a pharmaceutically acceptable salt thereof, L-leucine, or a pharmaceutically acceptable salt thereof, D-leucine, or a pharmaceutically acceptable salt thereof, L-leucine ethyl ester, or a pharmaceutically acceptable salt thereof, acetyl-DL-leucine, or a pharmaceutically acceptable salt thereof, acetyl-D-leucine, or a pharmaceutically acceptable salt thereof, or acetyl-L-leucine, or a pharmaceutically acceptable salt thereof.

## Description

### BACKGROUND OF THE INVENTION

### Field of the Invention

The present disclosure provides methods of treating diseases, disorders, conditions, or syndromes, e.g., traumatic brain injury, in a patient in need thereof by administering a therapeutically effective amount of DL-leucine, or a pharmaceutically acceptable salt thereof, D-leucine, or a pharmaceutically acceptable salt thereof, L-leucine, or a pharmaceutically acceptable salt thereof, L-leucine ethyl ester, or a pharmaceutically acceptable salt thereof, acetyl-DL-leucine, or a pharmaceutically acceptable salt thereof, acetyl-D-leucine, or a pharmaceutically acceptable salt thereof, or acetyl-L-leucine, or a pharmaceutically acceptable salt thereof.

### Background

Acetyl-DL-leucine has been used in France to treat acute vertigo since 1957. A FDG-µPET study in a rat model of an acute unilateral labyrinthectomy (Zwergal et al. (2016) Brain Struct Funct; 221(1): 159-70) showed a significant effect of an L-enantiomer, N-acetyl-L-leucine, on postural compensation by activation of the vestibulo-cerebellum and a deactivation of the posterolateral thalamus (Gunther et al. (2015) PLoS One; 10(3): e0120891). The symptomatic improvement of cerebellar ataxia using acetyl-DL-leucine was shown in a case series with cerebellar patients (Strupp et al. (2013) J Neurol; 260(10): 2556-61). Another case series did not find benefit (Pelz et al. (2015) J Neurol; 262(5): 1373-5). Quantitative gait analysis showed that acetyl-DL-leucine improved temporal gait variability in patients with cerebellar ataxia (Schniepp et al. (2015) Cerebellum; 3:8). In a one-month study involving 12 patients with Niemann-Pick Type C (NPC), symptomatic improvement of ataxia was shown (Bremova et al. (2015) Neurology; 85(16): 1368-75). Further, a PET study in patients with ataxia given acetyl-DL-leucine demonstrated an increased metabolism in the midbrain and lower brainstem in responders (Becker-Bense et al. (2015) Abstract EAN)*.*

WO 2018/029657 and WO 2018/029658 describe the use of leucine and acetyl-leucine for treating certain neurodegenerative diseases such as Alzheimer's disease (AD), multiple sclerosis (MS), Parkinson's disease (PD), and amyotrophic lateral sclerosis (ALS), and cerain lysosomal storage disorders (LSDs). In WO 2018/029658, a Niemann-Pick Disease Type C (NPC) mouse model showed that the NPC1 mouse brain exhibited accumulation of LC3-II, and the administration of acetyl-DL-leucine was associated with a lowering of LC3-II, indicative of a partial restoration of autophagic flux.

Inflammation caused by the production of pro-inflammatory cytokines, e.g., IL-1β, and chemokines is the cornerstone of various neurodegenerative, neuroinflammatory, mitochondrial and other diseases. *See, e.g.*, Chakrabarti et al., Curr Alzheimer Res. 15(10):894-904 (2018); Cherry et al., Journal of Neuroinflammation 11:98 (2014); Inácio et al., Journal of Neuroinflammation 12:211 (2015); Lappalainen et al., Am J Respir Cell Mol Biol 32:311-318 (2005); Coccia et al., J. Exp. Med. 209;1595-1609 (2012); Ray, Journal of the American College of Cardiology 63:1735-1438 (2014); Ren et al., Brain Res Rev. 60(1):57-64 (2009); Frank-Cannon et al., Molecular Neurodegeneration 4:47 (2009); DiSabato et al., J Neurochem 139(Suppl 2):136-153 (2016); Amor et al., Immunology 142:151-166 (2013); Chen et al., Molecular Medicine Reports 13:3391-3396 (2016); Tuttolomondo et al., Drug Design, Development and Therapy 8:2221-2239 (2014); Hong et al., Int Neurourol J 20 Suppl 1:S2-7 (2016); Gu et al., Journal of Biological Regulators & Homeostatic Agents 29:787-791 (2015); Belarbi et al., Journal of Neuroinflammation 9:23 (2012); Abdulkhaleq et al., Veterinary World, EISSN: 2231-0916 (available atwww.veterinaryworld.org/Vol.11/May-2018/9.pdf); Frankola et al., CNS Neurol Disord Drug Targets 10(3):391-403 (2011); Habbas et al., Cell 163:1730-1741 (2015); Debray et al., Current Opinion in Pediatrics 20:471-482 (2008); Niyazov et al., Mol Syndromol 7:122-137 (2016).

For example, traumatic brain injury ("TBI") is a devastating disorder associated with significant morbidity and mortality. Incidences of TBI continue to rise in view of population growth and increases in injury-related events such as traffic accidents. Other emergencies such as natural disasters, sports injuries, falls, assaults, and military conflict significantly contribute to cases of TBI. Within the United States alone, it is estimated that more than 1.5 million people sustain a TBI each year. TBI varies in severity, ranging from mild, to moderate, to severe, and may be focal or diffuse. Focal injuries occur in a specific location whereas diffuse injuries are associated with potentially widespread axonal dysfunction and vascular damage.

Damage to the brain from TBI occurs in two phases. The initial primary phase is the head trauma event itself, which is irreversible and amenable only to preventative measures to minimize the extent of damage, followed by an ongoing secondary phase, which begins at the time of injury and continues in the ensuing days to weeks to years. This secondary phase leads to a variety of physiological, cellular, and molecular responses aimed at restoring the homeostasis of the damaged tissue, which may lead to secondary brain injury.

There exists a need in the art for pharmaceutical agents to treat diseases responsive to the modulation of pro-inflammatory mediator expression.

### BRIEF SUMMARY OF THE INVENTION

Applicant has unexpectedly found that acetyl-L-leucine modulates the expression of pro-inflammatory mediators in a control cortical impact induced (CCI) traumatic brain injury model in mice. Thus, in one aspect, the present disclosure provides methods of treating a disease, disorder, condition, or syndrome wherein the modulation of one or more pro-inflammatory mediators provides a benefit. Foremost among these diseases, disorders, conditions, or syndromes are neuroinflammatory, neurological, autoimmune, neurodegenerative proteinopathic, psychiatric, and mitochondrial diseases, disorders, conditions, or syndromes.

Without wishing to be bound by any particular theory, it is believed that leucine, acetyl-DL-leucine, acetyl-D-leucine, and acetyl-L-leucine modulate neuroinflammation which is a pathway to combat the molecular cascades contributing to neuroinflammatory and other diseases, disorders, conditions, or syndromes. For example, secondary brain injury in TBI patients may be characterized by a hyper-reactive response, where an excess of pro-inflammatory cytokines may play a role in maintenance of brain function as well as repair after TBI. Head-trauma induced brain injury may trigger excessive and/or uncontrolled release of these cytokines, particularly interleukin (IL)-1β, IL-6, and tumour necrosis factor (TNF)-α, which may result in significant brain damage. Pro-inflammatory cvtokines are reported to play a role in the pathophysiology of neurodegenerative diseases such as AD, MS, PD, and ALS (Wei-Wei et al., Mol. Med. Rep. 2016; 13(4):3391-3396), and have been identified to be a hallmark of genetic-neurodegenerative disorders such as GM1 and GM2 gangliodises (Jeyakumar et al., Brain 2003; 126(Pt 4):974-87). In addition, disruption of normal lysosomal function in the lysosomal storage diseases (LSDs) has been associated with abnormalities in inflammation, such as abnormally elevated pro-inflammatory cytokines. (Simonaro, Journal of Inborn Errors of Metabolism & Screening, Vol. 4: 1-8 (2016)).

Also, without wishing to be bound by any particular theory, it is also believed according to the present disclosure that DL-leucine, L-leucine, D-leucine, L-leucine ethyl ester, acetyl-DL-leucine, acetyl-D-leucine, and acetyl-L-leucine modulates microtubule-associated protein Tau (which is an autophagy substrate) and/or the microtubule-associated protein 1A/1B-light chain 3-phosphatidylethanolamine conjugate (LC3-II), which may be markers of TBI. LC3-II is a marker of autophagosome formation, and increased levels of LC3-II can reflect impaired clearance of autophagic vacuoles. Autophagosomes are formed, but are not cleared. Autophagy is impaired in AD, and AD brains exhibit increased levels of LC3-II.

In one aspect, the present disclosure provides methods of treating a disease, disorder, condition, or syndrome in a subject in need thereof, or treating a symptom of a disease, disorder, condition, or syndrome in a subject in need thereof, the method comprising administering a therapeutically effective amount of a Compound of the Disclosure to the subject, wherein the diseases, disorders, conditions, or syndromes are any one or more of the diseases listed in Tables 1-17, see below. A "Compound of the Disclosure" collectively refers to DL-leucine ("DLL"), L-leucine ("LL"), D-leucine ("DL"), L-leucine ethyl ester ("LLE" or "LEE"), acetyl-DL-leucine ("ADLL"), acetyl-D-leucine ("ADL"), or acetyl-L-leucine ("ALL," "NAL", or "NALL"), and the pharmaceutically acceptable salts thereof.

In another aspect, the present disclosure provides methods of treating TBI in a subject in need thereof, or treating a symptom of TBI in a subject in need thereof, the method comprising administering a therapeutically effective amount a Compound of the Disclosure to the subject.

In another aspect, the present disclosure provides a Compound of the Disclosure for use in treating a disease, disorder, condition, or syndrome in a subject in need thereof, or for use in treating a symptom of a disease, disorder, condition, or syndrome in a subject in need thereof, wherein the diseases, disorders, conditions, or syndromes are any one or more of the diseases listed in Tables 1-17, see below.

In another aspect, the present disclosure provides a Compound of the Disclosure for use in treating TBI in a subject in need thereof, or for use in treating a symptom of TBI in a subject in need thereof.

In another aspect, the present disclosure provides the use of a Compound of the Disclosure for the manufacture of a medicament for treating a disease, disorder, condition, or syndrome in a subject in need thereof, or for the manufacture of a medicament for treating a symptom of a disease, disorder, condition, or syndrome in a subject in need thereof, wherein the diseases, disorders, conditions, or syndromes are any one or more of the diseases listed in Tables 1-17, see below.

In another aspect, the present disclosure provides the use of a Compound of the Disclosure for the manufacture of a medicament for treating TBI in a subject in need thereof, or for the manufacture of a medicament for treating TBI in a subject in need thereof.

In another aspect, the present disclosure provides a pharmaceutical composition comprising a Compound of the Disclosure and a pharmaceutically acceptable carrier for use in treating a disease, disorder, condition, or syndrome in a subject in need thereof, or for use in treating a symptom of a disease, disorder, condition, or syndrome in a subject in need thereof, wherein the diseases, disorders, conditions, or syndromes are any one or more of the diseases listed in Tables 1-17, see below.

In another aspect, the present disclosure provides a method of modulating the expression of pro-inflammatory mediators, e.g., pro-inflammatory cytokines and pro-inflammatory chemokines in a subject in a subject in need thereof, the method comprising administering a therapeutically effective amount a Compound of the Disclosure to the subject. In another aspect, the pro-inflammatory mediators include, but are not limited to, NOS2, IL-18, IFNb, IL-1β, TNFα, NOX2, NLRP3, SOCS3, ARG1, IL-10, IL-4ra, and/or YM1.

It is to be understood that both the foregoing summary and the following detailed description are exemplary and explanatory only, and are not restrictive of the invention as claimed.

### DETAILED DESCRIPTION OF DRAWINGS

Fig. 1 is a schematic showing the NAL dosing and experimental plan for the cortical impact induced TBI model. Mice were orally fed with NAL and cortical tissues were collected at days 1, 3, and 7 after TBI for biochemical analyses.
Fig. 2 is a bar graph showing the body weight change in mice 7 days after NAL (N-acetyl-L-leucine or acetyl-L-leucine) treatment following TBI.
Fig. 3 is bar graph showing the spatial memory assessment in mice 7 days after NAL treatment following TBI using the Y-maze test.
Fig. 4 is a bar graph showing IL-16 expression in mice 3 days after NAL treatment following CCI.
Fig. 5 is a bar graph showing IL-1β expression in mice 3 days after NAL treatment following CCI.
Fig. 6 is a bar graph showing INFb expression in mice 3 days after NAL treatment following CCI.
Fig. 7 is a bar graph showing Arg1 expression in mice 3 days after NAL treatment following CCI.
Fig. 8 is a bar graph showing SOCS3 expression in mice 3 days after NAL treatment following CCI.
Fig. 9 is a bar graph showing TNF expression in mice 3 days after NAL treatment following CCI.
Fig. 10 is a bar graph showing IL-18 expression in mice 3 days after NAL treatment following CCI.
Fig. 11 is a Western blot analysis assessment of autophagy and cell death after NAL treatment following CCI.
Fig. 12 is a bar graph showing the fodrin/b-actin levels after NAL treatment following CCI.
Fig. 13 is a bar graph showing the LC3-II/b-actin levels after NAL treatment following CCI.
Fig. 14 is a histogram of the ratio of LC3-II expression to βIII-tubulin in the cerebellum of mouse NPC1 brains at 9-12 weeks of age treated with ADLL, ALL, ADL, DLL, LL, DL, LLE, and miglustat (miglu.) for 3 weeks relative to untreated NPC1 cerebellum (n = 4, mean ± S.E.M).
Fig. 15 is a histogram of the ratio of APP-CTFs: full-length APP expression in the cerebellum of mouse NPC1 brains at 9-12 weeks of age treated with ADLL, ALL, ADL, DLL, LL, DL, LLE, and miglustat (miglu.) for 3 weeks relative to untreated NPC1 cerebellum (n = 4, mean ± S.E.M).
Fig. 16 is a histogram of the ratio of APP-CTF-6: full-length APP expression in the cerebellum of mouse NPC1 brains at 9-12 weeks of age treated with ADLL, ALL, ADL, DLL, LL, DL, and LLE for 3 weeks relative to untreated NPC1 cerebellum.
Fig. 17 is a histogram of the ratio of APP-CTF-7: full-length APP expression in the cerebellum of mouse NPC1 brains at 9-12 weeks of age treated with ADLL, ALL, ADL, DLL, LL, DL, and LLE for 3 weeks relative to untreated NPC1 cerebellum (n = 4, mean ± S.E.M).
Fig. 18. is a histogram of the ratio of LC3-II normalised to βIII tubulin (expressed as a percentage of untreated (n =1)) in rat primary cortical neurons that were treated with U18666A (2 µg/ml, 24 hr) in the absence or presence of ADLL, ALL, ADL, DLL, LL, DL, and LLE (1 mM, 24 hr).

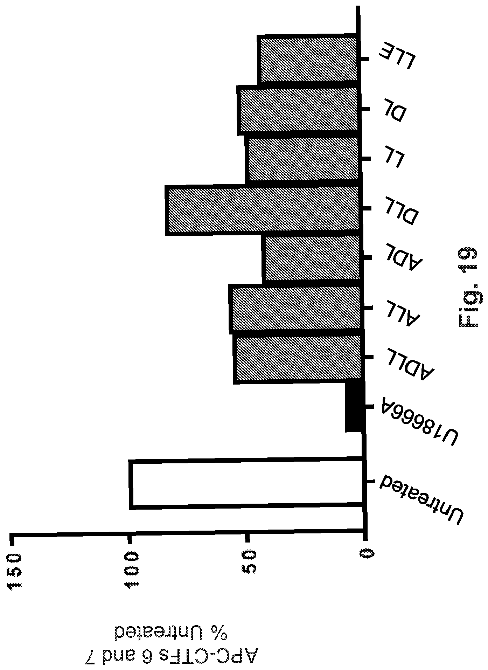

Fig. 19 is histogram of the ratio of APP-CTFs -6 and -7 normalised to their respective CTFs in untreated cells (expressed as a percentage of untreated (n =1) in rat primary cortical neurons that were treated with U18666A (2 µg/ml, 24 hr) in the absence or presence of ADLL, ALL, ADL, DLL, LL, DL, and LLE (1 mM, 24 hr).
Fig. 20 is eight Western blots of tau (5E2), synaptophysin and βIII-tubulin in rat primary cortical neurons (neuron-enriched (NE) and neuron-glia (NG) cultures) in the absence and presence of ADLL, ALL, ADL, DLL, LL, DL, and LLE (1 mM, 5 days).
Fig. 21 is a histogram of the ratio of full-length tau normalised to βIII-tubulin in NE cultures at DIV14 in the absence and presence of ADLL, ALL, ADL, DLL, LL, DL, and LLE (1 mM, 5 days).
Fig. 22 is a histogram of the ratio of truncated tau (Clone 5E2) normalised to βIIItubulin in NG cultures at DIV14 in the absence and presence of ADLL, ALL, ADL, DLL, LL, DL, and LLE (1 mM, 5 days).
Fig. 23 is a histogram of the ratio of synaptophysin normalized to βIII-tubulin in NE cultures at DIV14 (expressed as a percentage of untreated control lysates; n = 4, mean ± S.E.M) in the absence and presence of ADLL, ALL, ADL, DLL, LL, DL, and LLE (1 mM, 5 days).
Fig. 24 is a histogram of the ratio of synaptophysin normalized to βIII-tubulin in NG cultures at DIV14 (expressed as a percentage of untreated control lysates; n = 4, mean ± S.E.M) in the absence and presence of ADLL, ALL, ADL, DLL, LL, DL, and LLE (1 mM, 5 days).
Fig. 25 is a bar graph showing the lysomal volume in wild type and NPC1 null Chinese hamster ovary cells treated with ALL for seven days at the concentrations indicated (n = 4).
Fig. 26 is a bar graph showing the lysomal volume in wild type and NPC1 null Chinese hamster ovary cells treated with LEE for seven days at the concentrations indicated (n = 4).
Fig. 27 is a bar graph showing the lysomal volume in wild type and NPC1 null Chinese hamster ovary cells treated with LL for seven days at the concentrations indicated (n = 3).
Fig. 28 is a Western blot analysis assessment of cortical tissue lysate of sham and TBI mice fed with NALL or vehicle to detect α-fodrin breakdown products.
Fig. 29 is a bar graph showing the densitometric analysis of α-fodrin with respect to actin. Data are presented as mean ± SEM. n=5, **p<0.01 (Two-way ANOVA with Bonferroni posttests).
Fig. 30 is a series of four images of vehicle or NALL fed TBI mouse cortical brain sections stained for TUNEL. Data are presented as mean ± SEM. n=3 for vehicle fed and 4 for NALL fed TBI mice
Fig. 31 is a bar graph showing the area quantification for the TBI mouse cortical brain sections stained for TUNEL. Data are presented as mean ± SEM. n=3 for vehicle fed and 4 for NALL fed TBI mice.
Fig. 32 is a bar graph showing the cell quantification for the TBI mouse cortical brain sections stained for TUNEL. Data are presented as mean ± SEM. n=3 for vehicle fed and 4 for NALL fed TBI mice.
Fig. 33 is a Western blot analysis assessment of cortical tissue lysate of sham and TBI mice fed with NALL or vehicle for the autophagosomal marker LC3 and autophagic cargo proteins p62/SQSTM1.
Fig. 34 is a bar graph showing the densitometric analysis of LC3-II with respect to actin. Data are presented as mean ± SEM. n=5, *p<0.05 (Two-way ANOVA with Bonferroni posttests).
Fig. 35 is a bar graph showing the densitometric analysis of p62 with respect to actin. Data are presented as mean ± SEM. n=5, *p<0.05 (Two-way ANOVA with Bonferroni posttests).
Fig. 36 is a bar graph showing the relative mRNA levels of *NOS2* in the cortices of sham and TBI mice fed with NALL or vehicle. Data are presented as mean ± SEM. n=5, ***p<0.001, *p<0.05 (Two-way ANOVA with Bonferroni posttests).
Fig. 37 is a bar graph showing the relative mRNA levels of *NLRP3* in the cortices of sham and TBI mice fed with NALL or vehicle. Data are presented as mean ± SEM. n=5, ***p<0.001, *p<0.05 (Two-way ANOVA with Bonferroni posttests).
Fig. 38 is a bar graph showing the relative mRNA levels of *IL-1β* in the cortices of sham and TBI mice fed with NALL or vehicle. Data are presented as mean ± SEM. n=5, ***p<0.001, *p<0.05 (Two-way ANOVA with Bonferroni posttests).
Fig. 39 is a bar graph showing the relative mRNA levels of *TNF-α* in the cortices of sham and TBI mice fed with NALL or vehicle. Data are presented as mean ± SEM. n=5, ***p<0.001, *p<0.05 (Two-way ANOVA with Bonferroni posttests).
Fig. 40 is a bar graph showing the relative mRNA levels of *IFN-β* in the cortices of sham and TBI mice fed with NALL or vehicle. Data are presented as mean ± SEM. n=5, ***p<0.001, *p<0.05 (Two-way ANOVA with Bonferroni posttests).
Fig. 41 is a bar graph showing the relative mRNA levels of *NOX2* in the cortices of sham and TBI mice fed with NALL or vehicle. Data are presented as mean ± SEM. n=5, ***p<0.001, *p<0.05 (Two-way ANOVA with Bonferroni posttests).
Fig. 42 is a bar graph showing the relative mRNA levels of *SOCS3* in the cortices of sham and TBI mice fed with NALL or vehicle. Data are presented as mean ± SEM. n=5, ***p<0.001, *p<0.05 (Two-way ANOVA with Bonferroni posttests).
Fig. 43 is a bar graph showing the relative mRNA levels of *YM-1* in the cortices of sham and TBI mice fed with NALL or vehicle. Data are presented as mean ± SEM. n=5, ***p<0.001, *p<0.05 (Two-way ANOVA with Bonferroni posttests).
Fig. 44 is a bar graph showing the relative mRNA levels of *IL-4ra* in the cortices of sham and TBI mice fed with NALL or vehicle. Data are presented as mean ± SEM. n=5, ***p<0.001, *p<0.05 (Two-way ANOVA with Bonferroni posttests).
Fig. 45 is a bar graph showing the relative mRNA levels of *Arg-1* in the cortices of sham and TBI mice fed with NALL or vehicle. Data are presented as mean ± SEM. n=5, ***p<0.001, *p<0.05 (Two-way ANOVA with Bonferroni posttests).
Fig. 46 is an image showing cerebellar Purkinje cell loss progressing from anterior to posterior lobes and microglial activation in untreated *Npc1*^{*-*/*-*} mice and *Npc1*^{*-*/*-*} mice treated with ADLL, ALL, and ADL. Cerebellums were stained with calbindin-b (Purkinje cells) or CD68 (activated microglia).
Fig. 47 is a scatter graph showing Purkinje cell survival at 59 days of age in untreated *Npc1*^{*-*/*-*} mice and *Npc1*^{*-*/*-*} mice treated with ADLL, ALL, and ADL.
Fig. 48 is a scatter graph showing frequency of CD68 positive activated microglia at 59 days of age in untreated *Npc1*^{*-*/*-*} mice and *Npc1*^{*-*/*-*} mice treated with ADLL, ALL, and ADL. CD68 cell density was measured in the cerebellum. n=5 animals per group. Mean ± SD, *** *p*<0.0009 (one-way ANOVA).
Fig. 49 is a line graph showing the motor function (beam walk) outcome in the controlled cortical impact TBI model with mice treated with NAL.

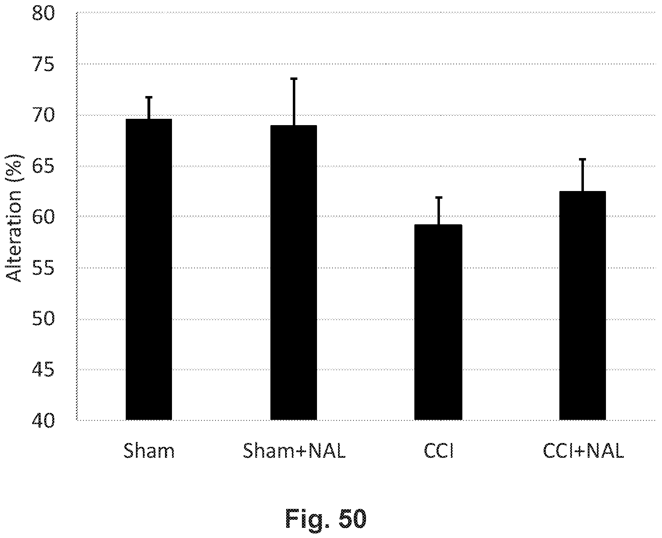

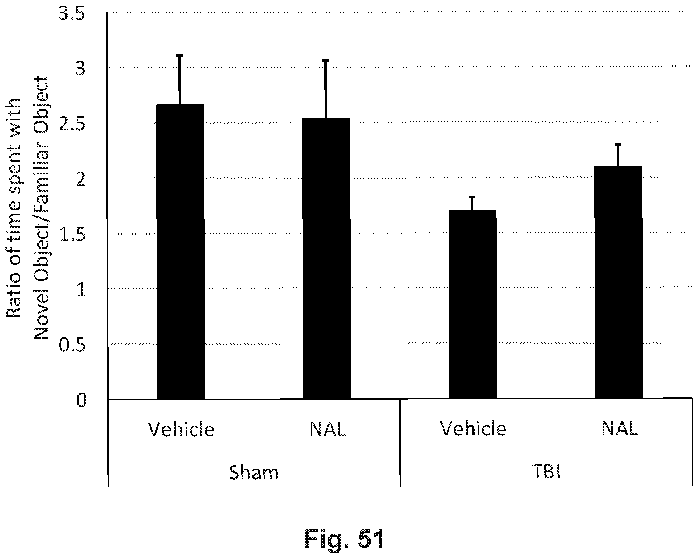

Fig. 50 is bar graph showing the spatial memory (Y-maze) outcome in the controlled cortical impact TBI model with mice treated with NAL.
Fig, 51 is a bar 'graph showing the novel object recognition (NOR) outcome in the controlled cortical impact TBI model with mice treated with NAL.

### DETAILED DESCRIPTION OF THE INVENTION

A "subject," as used herein, may be a vertebrate, mammal or domestic animal. Hence, compositions according to the disclosure may be used to treat any mammal, for example livestock (e.g. a horse, cow, sheep or pig), pets (e.g. a cat, dog, rabbit or guinea pig), a laboratory animal (e.g. a mouse or rat), or may be used in other veterinary applications. In one embodiment, the subject is a human being. "Subject" and "patient" are used interchangeably.

As used herein, the singular forms "a," "an," and "the" include plural reference.

The terms "approximately" and "about" mean to be nearly the same as a referenced number or value including an acceptable degree of error for the quantity measured given the nature or precision of the measurements.

As used herein, the terms "approximately" and "about" should be generally understood to encompass ± 20% of a specified amount, frequency or value. Numerical quantities given herein are approximate unless stated otherwise, meaning that term "about" or "approximately" can be inferred when not expressly stated.

The terms "administer," "administration," or "administering" as used herein refer to (1) providing, giving, dosing and/or prescribing by either a health practitioner or his authorized agent or under his direction, a Compound of the Disclosure and (2) putting into, taking or consuming by the patient or person himself or herself, a Compound of the Disclosure. Any reference to "leucine," "acetyl-DL-leucine," and "acetyl-L-leucine," or any other Compound of the Disclosure, include pharmaceutically acceptable salts of the same, even if not expressly stated.

Unless otherwise indicated, the term "leucine" refers to L-leucine.

A "pharmaceutically acceptable salt" as referred to herein, is any salt preparation that is appropriate for use in a pharmaceutical application. Pharmaceutically acceptable salts include, but are not limited to, amine salts, such as N,N'-dibenzylethylenediamine, chloroprocaine, choline, ammonia, diethanolamine and other hydroxyalkylamines, ethylenediamine, N-methylglucamine, procaine, N-benzylphenethylamine, 1-para-chlorobenzyl-2-pyrrolidin-1'-ylmethylbenzimidazole, diethylamine and other alkylamines, piperazine, tris(hydroxymethyl)aminomethane and the like; alkali metal salts, such as lithium, potassium, sodium and the like; alkali earth metal salts, such as barium, calcium, magnesium and the like; transition metal salts, such as zinc, aluminum and the like; other metal salts, such as sodium hydrogen phosphate, disodium phosphate and the like; mineral acids, such as hydrochlorides, sulfates and the like; and salts of organic acids, such as acetates, lactates, malates, tartrates, citrates, ascorbates, succinates, butyrates, valerates, fumarates and the like.

A Compound of the Disclosure may be formulated and administered to a subject in accordance with known teachings in the art. For example, DL-leucine, L-leucine, D-leucine, L-leucine ethyl ester, acetyl-DL-leucine, acetyl-D-leucine, or acetyl-L-leucine may be formulated as a pharmaceutical composition. The pharmaceutical composition may comprise DL-leucine, L-leucine, D-leucine, L-leucine ethyl ester, acetyl-DL-leucine, acetyl-D-leucine, or acetyl-L-leucine, and a pharmaceutically acceptable carrier. Reference to the pharmaceutical composition encompasses the active agent alone, i.e., DL-leucine, L-leucine, D-leucine, L-leucine ethyl ester, acetyl-DL-leucine, acetyl-D-leucine, or acetyl-L-leucine, or in the form of a pharmaceutical composition.

The pharmaceutical composition may take any of a number of different forms depending, in particular, on the manner in which it is to be used. Thus, for example, it may be in the form of a powder, tablet, capsule, liquid, ointment, cream, gel, hydrogel, aerosol, spray, micellar solution, transdermal patch, liposome suspension or any other suitable form that may be administered to a person or animal in need of treatment.

A "pharmaceutically acceptable carrier" as referred to herein, is any known compound or combination of known compounds, e.g., excipients, carriers, etc., that are known to those skilled in the art to be useful in formulating pharmaceutical compositions. It will be appreciated that the carrier of the pharmaceutical composition should be one which is tolerated by the subject to whom it is given.

In one embodiment, the pharmaceutically acceptable carrier may be a solid, and the composition may be in the form of a powder or tablet. A solid pharmaceutically acceptable carrier may include, but is not limited to, one or more substances which may also act as flavouring agents, buffers, lubricants, stabilisers, solubilisers, suspending agents, wetting agents, emulsifiers, dyes, fillers, glidants, compression aids, inert binders, sweeteners, preservatives, dyes, coatings, or tablet-disintegrating agents. The carrier may also be an encapsulating material. In powders, the carrier may be a finely divided solid that is in admixture with the finely divided active agents according to the invention. In tablets, the active agent may be mixed with a carrier having the necessary compression properties in suitable proportions and compacted in the shape and size desired. The powders and tablets may, for example, contain up to 99% of the active agents. Suitable solid carriers include, for example, calcium phosphate, magnesium stearate, talc, sugars, lactose, dextrin, starch, gelatin, cellulose, polyvinylpyrrolidine, low melting waxes and ion exchange resins. In another embodiment, the pharmaceutically acceptable carrier may be a gel and the composition may be in the form of a cream or the like.

The carrier may include, but is not limited to, one or more excipients or diluents. Examples of such excipients are gelatin, gum arabicum, lactose, microcrystalline cellulose, starch, sodium starch glycolate, calcium hydrogen phosphate, magnesium stearate, talcum, colloidal silicon dioxide, and the like.

In another embodiment, the pharmaceutically acceptable carrier may be a liquid. In one embodiment, the pharmaceutical composition is in the form of a solution. Liquid carriers are used in preparing solutions, suspensions, emulsions, syrups, elixirs and pressurized compositions. A Compound of the Disclosure may be dissolved or suspended in a pharmaceutically acceptable liquid carrier such as water, an organic solvent, a mixture of both or pharmaceutically acceptable oils or fats. The liquid carrier may contain other suitable pharmaceutical additives such as solubilisers, emulsifiers, buffers, preservatives, sweeteners, flavouring agents, suspending agents, thickening agents, colours, viscosity regulators, stabilizers or osmo-regulators. Suitable examples of liquid carriers for oral and parenteral administration include water (partially containing additives as above, e.g. cellulose derivatives, such as sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the carrier may also be an oily ester such as ethyl oleate and isopropyl myristate. Sterile liquid carriers are useful in sterile liquid form compositions for parenteral administration. The liquid carrier for pressurised compositions may be a halogenated hydrocarbon or other pharmaceutically acceptable propellant.

Liquid pharmaceutical compositions, which are sterile solutions or suspensions, may be utilised by, for example, intramuscular, intrathecal, epidural, intraperitoneal, intravenous and subcutaneous injection. The active agent may be prepared as a sterile solid composition that may be dissolved or suspended at the time of administration using sterile water, saline, or other appropriate sterile injectable medium.

The compositions may be administered orally in the form of a sterile solution or suspension containing other solutes or suspending agents (for example, enough saline or glucose to make the solution isotonic), bile salts, acacia, gelatin, sorbitan monoleate, polysorbate 80 (oleate esters of sorbitol and its anhydrides copolymerized with ethylene oxide) and the like. The compositions may also be administered orally either in liquid or solid composition form. Compositions suitable for oral administration include solid forms, such as pills, capsules, granules, tablets, and powders, and liquid forms, such as solutions, syrups, elixirs, and suspensions. Forms useful for parenteral administration include sterile solutions, emulsions, and suspensions.

Compositions may alternatively be administered by inhalation (e.g. intranasally). Compositions may also be formulated for topical use. For instance, creams or ointments may be applied to the skin.

A Compound of the Disclosure may be incorporated within a slow- or delayed-release device. Such devices may, for example, be inserted on or under the skin, and the medicament may be released over weeks or even months. Such devices may be advantageous when long-term treatment with a Compound of the Disclosure used according to the present disclosure is required and which may require frequent administration (e.g. at least daily administration).

In one embodiment, the pharmaceutical composition is a solid oral dosage form, such as a tablet. In tablets, the active agent may be mixed with a vehicle, such as a pharmaceutically acceptable carrier, having the necessary compression properties in suitable proportions and compacted in the shape and size desired. The tablets may contain up to 99% by weight of the active agents.

Pharmaceutical compositions in solid oral dosage form, such as tablets, may be prepared by any method known in the art of pharmacy. Pharmaceutical compositions are usually prepared by mixing the active agent with conventional pharmaceutically acceptable carriers.

A tablet may be formulated as is known in the art. Tanganil^{®}, for example, includes wheat starch, pregelatinised maize (corn) starch, calcium carbonate and magnesium stearate as excipients. The same, or similar, excipients, for example, may be employed with the present disclosure.

The composition of each 700 mg Tanganil^{®} tablet is as follows: 500 mg acetyl-DL-leucine, 88 mg wheat starch, 88 mg pregelatinised maize (corn) starch, 13 mg calcium carbonate and 11 mg magnesium stearate. The same tablets, for example, may be employed in the methods of the present disclosure.

As discussed above, a Compound of the Disclosure may be formulated and administered as a pharmaceutical composition taking any number of different forms. For example, a Compound of the Disclosure may be formulated as a pharmaceutical composition to facilitate its delivery across the blood-brain barrier. As a further example, a Compound of the Disclosure may be formulated as a pharmaceutical composition for bypassing the blood-brain barrier. Formulations that facilitate delivery across the blood-brain barrier or that are suitable for administration in a manner that bypasses the blood-brain barrier may be used to prepare and administer leucine (not acetylated) as described herein.

In one embodiment, the pharmaceutical composition, e.g., comprising acetyl-L-leucine, or salt thereof, is formulated for nanodelivery, e.g., colloidal drug-carrier systems. Suitable examples include but are not limited to liposomes, nanoparticles (e.g., polymeric, lipid and inorganic nanoparticles), nanogels, dendrimers, micelles, nanoemulsions, polymersomes, exosomes, and quantum dots. See, e.g., Patel et al., "Crossing the Blood-Brain Barrier: Recent Advances in Drug Delivery to the Brain," CNS Drugs 31:109-133 (2017); Kabanov et al., "New Technologies for Drug Delivery across the Blood Brain Barrier," Curr Pharm Des., 10(12):1355-1363 (2004); Cheng et al., "Highly Stabilized Curcumin Nanoparticles Tested in an In Vitro Blood-Brain Barrier Model and in Alzheimer's Disease Tg2576 Mice," The AAPS Journal, vol. 15, no. 2, pp. 324-336 (2013); Lähde et al. "Production of L-Leucine Nanoparticles under Various Conditions Using an Aerosol Flow Reactor Method," Journal of Nanomaterials, vol. 2008, article ID 680897 (2008).

In one embodiment, the pharmaceutical composition, e.g., comprising acetyl-L-leucine, or salt thereof, is formulated for direct delivery to the central nervous system (CNS), such as by injection or infusion. Formulations for and methods of direct delivery to the CNS are known in the art. *See, e.g.*, U.S. Patent No. 9,283,181. Examples of such administration include but are not limited to intranasal, intraventricular, intrathecal, intracranial, and delivery via nasal mucosal grafting.

In one embodiment, the pharmaceutical composition is formulated for (and administered by) intranasal delivery. See, e.g., Hanson et al., "Intranasal delivery bypasses the blood-brain barrier to target therapeutic agents to the central nervous system and treat neurodegenerative disease," BMC Neurosci. 9(Suppl 3):S5 (2008). In one embodiment, the pharmaceutical composition is formulated for (and administered by) delivery via a nasal mucosal graft. In one embodiment, the pharmaceutical composition is formulated for (and administered by) intracerebroventricular injection or infusion. In another embodiment, the pharmaceutical composition is formulated for (and administered by) intrathecal intracisternal injection or infusion. In one embodiment, the pharmaceutical composition is formulated for (and administered by) intrathecal lumbar injection or infusion.

Various techniques may be used including, without limitation, injection through a burrhole or cisternal or lumbar puncture or the like as known in the art. Various devices, whether internal (e.g., implanted) or external, may be used for delivery as known in the art, such as pumps, catheters, reservoirs, etc. In one embodiment, the administration interval is once every two weeks.

In one embodiment, the administration interval is once every month. In one embodiment, the administration interval is once every two months. In one embodiment, the administration interval is twice per month. In one embodiment, the administration interval is once every week. In one embodiment, the administration interval is twice or several times per week. In one embodiment, the administration interval is daily. In one embodiment, the administration is continuous, such as continuous infusion.

In one embodiment, the dose or amount equivalent of a Compound of the Disclosure may adjusted to account for either its direct delivery to the CNS or its delivery across the blood-brain barrier.

The present disclosure describes a Compound of the Disclosure, including pharmaceutical compositions thereof, for treating various diseases, disorders, conditions, and syndromes in a subject in need thereof.

As used herein, "traumatic brain injury" refers to any injury to the brain having an initial phase consisting of at least one head-trauma event (i.e., an object entering the brain and/or load(s) or force(s) on the brain causing the brain to move rapidly and/or unnaturally within the subject's skull) and an ensuing secondary phase that comprises physiological, cellular, and/or molecular disturbances resulting from the head trauma. Examples of head trauma include objects penetrating the skull, such as, bullets, arrows, and other physical objects which pass through the skull and enter the brain; impact loads or forces applied to the head or other portions of the patient's body; surgically induced trauma; tremors created by explosions; tremors created by nonexplosive means, such as sports injuries, vehicular accidents, collapse of buildings and earthquakes, for example. The results of a TBI may be of various types, and all forms of TBI are within the scope of the present disclosure. For example, TBI can range in severity from a brief change in mental status or consciousness to an extended period of unconsciousness or memory loss. In one embodiment, the TBI was inflicted by a concussion. In one embodiment, the TBI was inflicted by a contusion. In one embodiment, the TBI was inflicted by an open head injury. In another embodiment, the TBI was inflicted by a closed head injury. In one embodiment, the TBI was inflicted by a focal injury. In another embodiment, the TBI was inflicted by a diffuse injury. In one embodiment, the form of TBI is mild TBI. In another embodiment, the form of TBI is moderate TBI. In another embodiment, the form of TBI is severe TBI.

A "subject in need thereof" as used herein may be any subject who has a disease, disorder, condition, or syndrome, e.g., a disease, disorder, condition, or syndrome provided in any one of Tables 1-17. The subject may or may not have been diagnosed with the disease, disorder, condition, or syndrome. For example, the subject may not yet have a diagnosis (clinical or otherwise) of TBI, but may have one or more symptoms of TBI. The subject may also have a biochemical or other similar identifiable marker of a TBI.

A "therapeutically effective amount" of Compound of the Disclosure is any amount which, when administered to a subject, is the amount that is needed to produce the desired effect, which, for the present disclosure, can be therapeutic and/or prophylactic. The dose may be determined according to various parameters, such as the specific Compound of the Disclosure used; the age, weight and condition of the patient to be treated; the route of administration; and the required regimen. A physician will be able to determine the required route of administration and dosage for any particular patient. For example, a daily dose may be from about 10 to about 225 mg per kg, from about 10 to about 150 mg per kg, or from about 10 to about 100 mg per kg of body weight.

As used herein, "treating" or "treatment" refers to any indicia of success in preventing, arresting, or ameliorating a disease, disorder, condition, or syndrome, e.g., a disease, disorder, condition, or syndrome provided in any one of Tables 1-17, in a subject, and/or preventing, arresting, or ameliorating any one or more symptoms a disease, disorder, condition, or syndrome, e.g., a disease, disorder, condition, or syndrome provided in any one of Tables 1-17, in a subject, including any objective or subjective parameter such as abatement; remission; preventing, diminishing, inhibiting, or eliminating one or more symptoms; making the disease, disorder, condition, or syndrome more tolerable to the subject; slowing in the worsening of the disease, disorder, condition, or syndrome; or improving the physical or mental well-being of the subject in need thereof.

The terms "treating" or "treatment" also encompasses, e.g., inducing inhibition, regression, or stasis of the disease, disorder, condition, or syndrome. For example, treatment of a patient or subject in need of treatment for TBI includes reducing a symptom of TBI in the subject, inducing clinical response, inhibiting or reducing progression of TBI, or inhibiting or reducing a complication of TBI.

Preventing, arresting, or ameliorating an injury or pathology of a disease, disorder, condition, or syndrome, such as preventing, diminishing, inhibiting, or eliminating one or more symptoms of disease, disorder, condition, or syndrome can be based on objective and/or subjective parameters, including, e.g., the results of physical examination(s), neurological examination(s), and/or psychiatric evaluation(s). The success of treatment for certain diseases listed in Tables 1-17, e.g., TBI, may be measured or evaluated by, for example, comparing the severity of the disease (e.g., objective and/or subjective parameters of TBI) before treatment with a Compound of the Disclosure is initiated, with the severity of the disease (e.g., objective and/or subjective parameters of TBI) following the initiation of treatment with a Compound of the Disclosure. For example, the severity of TBI may be assessed using a scale, index, rating, or score. In one embodiment, the treatment described herein improves such an assessment from a value or degree characteristic of a symptomatic subject to a value or degree characteristic of a non-symptomatic subject. In one embodiment, the treatment described herein improves such an assessment compared to a baseline. The baseline may be, for example, the subject's condition before initiating any treatment for the disease, e.g., TBI, or before initiating treatment for the disease with a Compound of the Disclosure. Alternatively, the baseline may be, for example, the subject's condition after a certain time period on treatment for the disease. In one embodiment, treatment with a Compound of the Disclosure as described herein improves the subject's assessment (e.g., scale, index, rating, or score of objective and/or subjective parameters) compared to a baseline by at least 10%, at least 20%, at least 30%, at least 40%, or at least 50%. In one embodiment, assessment is improved by at least 60%, at least 70%, at least 80%, at least 90%, or 100%.

For example, in one embodiment, the severity of the patient's disease, e.g., TBI, may be quantified by the Glasgow Coma Scale (GCS). The GCS is the cumulative score of three areas of examination: Eye, Verbal and Motor function. For the Eye exam, the patient is graded from 1 to 4 as follows: 1-no eye opening to any stimulation, 2-eye opening only in response to pain, 3-eye opening to speech, and 4-eyes are open spontaneously. For the Verbal exam, the patient is graded from 1 to 5 as follows: 1-no verbal response, 2-incomprehensible sounds, 3- inappropriate words, 4-confused, and 5-oriented. For the Motor exam, the patient is graded from 1 to 6 as follows: 1-no motor response, 2-extension to pain, 3-abnormal flexion to pain, 4- withdrawal to pain, 5-localizes to pain, and 6-obeys commands. The GCS score is the sum of the three scores received for the Eye, Verbal and Motor responses. In general, TBI is classified as mild, moderate or severe based on the Glasgow Coma Scale as follows: Mild: GCS > 13, Moderate: GCS 9 - 12, and Severe: GCS < 8. The level of recovery of TBI patients may be quantified by the Glasgow Outcome Scale (GOS). The Glasgow Outcome Scale is a 5- level score: 1-dead, 2-vegetative state, 3-severely disabled, 4- moderately disabled, and 5-good recovery. The GOS is frequently divided into "favorable" outcomes (moderately disabled and good recovery) and "unfavorable" outcomes (dead, vegetative state, and severely disabled).

A "symptom" of a disease, disorder, condition, or syndrome includes any clinical or laboratory manifestation associated with the disease, disorder, condition, or syndrome and is not limited to what the subject can feel or observe. For example, symptoms of certain diseases listed in Tables 1-17, e.g., TBI, include, but are not limited to, loss of consciousness, confusion, restlessness, agitation, disorientation, loss of memory, headache, lightheadedness, dizziness, blurred vision or tired eyes, fatigue or lethargy, change in sleep pattern, behavioural or mood swings, problems with memory, concentration, attention, and/or thinking, repeated nauseua or vomiting, convulsions or seizures, inability to awaken from sleep, slurred speech, weakness or numbness in the extremities, and loss of coordination.

In one embodiment, a Compound of the Disclosure may be administered, for example, at a dose ranging from about 500 mg to about 30 g per day or ranging from about 500 mg to about 15 g per day, such as ranging from about 1.5 g to about 10 g per day, optionally by solid oral or liquid oral route. A Compound of the Disclosure, may be administered, for example, in a dose according to that of Tanganil^{®}, which is prescribed to adults in a dose of 1.5 g to 2 g per day, 3-4 tablets in two doses, morning and evening.

If a single enantiomer, i.e., L-leucine, acetyl-D-leucine, or acetyl-L-leucine, is administered the doses may be reduced accordingly. For instance if only acetyl-L-leucine or if only acetyl-D-leucine is administered, the dose may range from about 250 mg to about 15 g per day, range from about 250 mg to about 10 g per day, or range from about 250 mg to about 5 g per day, such as from about 0.75 g to about 5 g per day.

In one embodiment, the administered dose ranges from about 1 g to about 30 g per day, from about 1 g to about 15 g per day, from about 1 g to about 10 g per day, or from about 1.5 g to about 7 g per day, from 15.1 g to about 30 g per day, 16 g to about 30 g per day, 17 g to about 30 g per day, 18 g to about 30 g per day, 19 g to about 30 g per day, or 20 g to about 30 g per day. It may be from about 2, 3, 4, 5, 6, 7, 8, 9, 10, 11, 12, 13, or 14 g to about 15 g per day. It may be from about 2, 3, 4, 5, 6, 7, 8 or 9 g to about 10 g per day. It may be from 15.1, 16, 17, 18, 19, 20, 21, 22, 23, 24, 25, 25, 27, 28, or 29 g to about 30 g per day. It may be more than about 1.5 g per day, but less than about 15, 14, 13, 12, 11, 10, 9, 8, 7, 6 or 5 g per day. In one embodiment, the dose ranges from about 4 g to about 6 g per day. In one embodiment, the dose ranges from about 4 g to about 5 g per day. In one embodiment, the dose is about 4.5 g per day. In one embodiment, the dose is about 5 g per day. In one embodiment, the dose is about 1 g per day, about 2 g per day, about 3 g per day, about 4 g per day, about 5 g per day, about 6 g per day, about 7 g per day, about 8 g per day, about 9 g per day, about 10 g per day, about 11g per day, about 12 g per day, about 13 g per day, about 14 g per day, or about 15 g per day. In another embodiment, the dose is about 16 g per day, about 17 g per day, about 18 g per day, about 19 g per day, or about 20 g per day. In another embodiment, the dose is about 21 g per day, about 22 g per day, about 23 g per day, about 24 g per day, about 25 g per day, about 26 g per day, about 27 g per day, about 28 g per day, about 29 g per day, or about 30 g per day. In one embodiment, these doses are administered in a solid oral dosage form, notably tablets. In another embodiment, these doses are for acetyl-leucine when in its racemic form. Doses for acetyl-leucine when an enantiomeric excess is present may be lower, for example, around 50% lower. The above recited dose-ranges when halved are thus also explicitly encompassed by the present disclosure.

In one embodiment, the total daily dose may be spread across multiple administrations, i.e. administration may occur two or more times a day to achieve the total daily dose. As an example, the required number of tablets to provide the total daily dose of a Compound of the Disclosure may be split across two administrations (for example, in the morning and evening) or three administrations (for example, in the morning, noon and evening). Each dose may be suitably administered with or without food. For example, acetyl-L-leucine or acetyl-DL-leucine may be dosed by about 1 or about 2 hours before meals, such as at least about 20 minutes, at least about 30 minutes, at least about 40 minutes, or at least about 1 hour before meals, or may be dosed by about 1, about 2, or about 3 hours after meals, such as waiting at least about 20 minutes, at least about 30 minutes, at least about 1 hour, at least about 1.5 hours, at least about 2 hours, or at least about 2.5 hours after meals. For example, a total daily dose of 4.5 g acetyl-DL-leucine may be administered as three Tanganil^{®} (or equivalent) tablets before, with, or after breakfast, three further tablets before, with, or after lunch and three further tablets before, with, or after dinner.

In one embodiment, with respect to TBI, administration of a Compound of the Disclosure in accordance with the present disclosure is initiated at or around (e.g., within 24 hours) the time of head trauma. In one embodiment, the administration is initiated after a period of time (e.g., days, weeks, months, or even years) following head trauma. In one embodiment, the administration is initiated after the subject is diagnosed (clinically or otherwise) to have TBI. In one embodiment, the administration is initiated after the subject is diagnosed to have TBI using the Glasgow Coma Scale (GCS). In one embodiment, the subject had a GCS score of 13-15. In another embodiment, the subject had a GCS score of 9-12. In another embodiment, the subject had a GCS score of 3-8. In one embodiment, the administration is initiated in advance of head trauma (or in advance of a subsequent head trauma if one or more head traumas have already occurred) to a subject at risk of suffering TBI. In one embodiment, the administration is initiated at, around, or after the time a subject is found to have a biochemical, or other similar identifiable marker of TBI. In one embodiment, the administration is initiated at, around, or after the time a subject experiences one or more symptoms of TBI.

Treatment duration for any of the diseases listed in Tables 1-17 may be, for example, about seven days or more, about two weeks or more, about three weeks or more, about one month or more, about six weeks or more, about seven weeks or more, or about two months or more. In one embodiment, it is about three months or more, about four months or more, about five months or more or about six months or more. The treatment duration may be about 1 year or more, about 2 years or more, about 4 years or more, about 5 years or more, or about 10 years or more. The treatment duration may be the lifetime of the subject.

Any and all combinations of dosage form, dose amount, dosing schedule and treatment duration are envisaged and encompassed by the disclosure. In one embodiment, the dose is from about 4 g to about 10 g per day, taken across one, two, or three administrations per day, for a treatment duration of about two months or more. In another embodiment, the dose is more than 4 g but no more than 5 g per day, taken across one, two, or three administrations per day, for a treatment duration of about six months or more. The dosage form may be a solid oral dosage form, notably tablets.

A Compound of the Disclosure may be used as a monotherapy (e.g., use of the active agent alone) for treating a disease, disorder, condition, or syndrome, e.g., a disease, disorder, condition, or syndrome provided in any one of Tables 1-17, in a subject. Alternatively, a Compound of the Disclosure may be used as an adjunct to, or in combination with, other known therapies for treating a disease, disorder, condition, or syndrome, e.g., a disease, disorder, condition, or syndrome provided in any one of Tables 1-17.

Also disclosed is a kit for treating a disease, disorder, condition, or syndrome, e.g., a disease, disorder, condition, or syndrome provided in any one of Tables 1-17, in a subject, comprising a means for diagnosing or prognosing the disease, disorder, condition, or syndrome, and a Compound of the Disclosure.

The kit may further comprise buffers or aqueous solutions. The kit may further comprise instructions for using the Compound of the Disclosure according to a method of the present disclosure.

All of the features described herein (including any accompanying claims, abstract and drawings), and/or all of the steps of any method so disclosed, may be combined with any of the above aspects in any combination, except combinations where at least some of such features and/or steps are mutually exclusive.

The phrase "a disease, disorder, condition, or syndrome wherein modulation of one or more pro-inflammatory mediators provides a benefit" and the like refers to (i): a disease, disorder, condition, or syndrome in which one or more pro-inflammatory mediators are important or necessary, e.g., for the onset, progress, or expression of that disease, disorder, condition, or syndrome, or; (ii) a disease, disorder, condition, or syndrome which is known to be responsive to the modulation of a pro-inflammatory mediator. Examples of such diseases include, but are not limited to, neuroinflammatory diseases, neurological diseases, autoimmune diseases, neurodegenerative proteinopathic, psychiatric conditions, e.g., depression, and mitochondrial diseases. Pro-inflammatory mediators are well-known in the art. *See, e.g.*, "Really Essential Medical Immunology," 2nd ed., Rabson, Roitt, Delves (Blackwell, 2005), Exemplary, non-limiting pro-inflammatory mediators include cytokines, e.g., interleukin (IL)-1β, IL-8, tumor necrosis factor alpha (TNF-α), IL-6, and IL-12, chemokines, and growth factors.

In one embodiment, the present disclosure provides a method of treating a disease, disorder, condition, or syndrome in a subject in need thereof, or treating a symptom of a disease, disorder, condition, or syndrome in a subject in need thereof, the method comprising administering a therapeutically effective amount a Compound of the Disclosure to the subject, wherein the diseases, disorders, conditions, or syndromes are any one or more of the diseases, disorders, conditions, or syndromes provided in Table 1.

**Table 1**

| | |
|---|---|
| accute optic neuritis | Klippel-Feil syndrome |
| acoustic neuroma | Kufor Rakeb disease |
| acute confusion disorders | Kufs' disease |
| acute confusion disorders in which apoptotic necrocytosis plays a part | labrynthitis |
| acute disseminated encephalomyelitis | Lambert-Eaton myasthenic syndrome (LEMS) |
| addiction | lattice corneal dystrophy |
| adrenoleukodystrophy | lead enphalapathy |
| Adult and juvenile Still disease | Leber hereditary optic neuropathy (LHON) |
| age-related macular degeneration (ARMD) | Leigh syndrome |
| agryophilic grain disease | Lennox Gastaut syndromes |
| AIDS | Lesch-Nyhan disease |
| AIDS-related dementia | lewy body dementia |
| Alpers-Huttenlocher syndrome | lewy body disease |
| Alzheimer's Disease | lewy body mutant of Alzheimer' s disease |
| amyotrophic lateral sclerosis | lipofuscinosis |
| anencephaly | lupus nephritis |
| ankylosing spondylitis | Lyme disease |
| antisynthetase syndrome | macrophage activation syndrome (MAS) |
| aortic medial amyloid | major depression |
| apathy | malnutrition |
| ataxia neuropathy syndromes (ANS), e.g., mitochondrial recessive ataxia syndrome (MIRAS), spinocerebellar ataxia with epilepsy (SCAE), sensory ataxia neuropathy, dysarthria, ophthalmoplegia (SANDO), myoclonic epilepsy myopathy sensory ataxia (MEMSA) | mania |
| atherosclerosis | maple syrup urine disease |
| attention deficit disorder (ADD) | medullary carcinoma of the thyroid |
| attention deficit hyperactivity disorder (ADHD) | meningioangiomatosis |
| autism | Menkes disease |
| autism spectrum disorder (ASD) | microglial hyper-activation |
| autoimmune thrombocytopenia | migraine |
| autoimmune vasculitis | mild cognitive impairment |
| B12 deficiency | mitochondrial disorders |
| batten disease | mitochondrial encephalomyopathy with lactic acidosis and stroke-like episodes (MELAS) |
| Behçet disease | Morbus Parkinson |
| bipolar disorder | Moyamoya disease |
| Blau syndrome; Sweet syndrome | multi-infarct dementia |
| bovine spongiform encephalopathy | multiple sclerosis |
| brain inflammation | multiple system atrophy |
| brain lesions | myasthenia gravis |
| brain neoplasms | myoclonic epilepsy with ragged-red fibers (MERRF) |
| cancer cachexia | myoneurogenic gastrointestinal encephalopathy (MNGIE) |
| cardiac arrythmias | myotonic dystrophy |
| cardiovascular disease | nerve trauma |
| catatonia | neurodegeneration with brain iron accumulation type I |
| central nervous system (CNS) vasculitis | neurofibromatosis |
| central nervous system regeneration | neurogenic weakness with ataxia and retinitis pigmentosa (NARP) |
| cerebral amyloid angiopathy (and Icelandic type) | neuroinflammation |
| cerebral haemorrhage with amyloidosis | Neuromyelitis Optica |
| cerebral palsy | neuropsychiatric disorders |
| cerebrovascular disease | neurosarcoidosis |
| Charcot-Marie-Tooth type 2K | neurosyphillis |
| chorea in children and adults | niacin deficiency |
| chromosome 8P | obsessive compulsive disorder |
| Chronic obstructive pulmonary disease | olivopontocerebellar atrophy (OPCA) |
| chronic progressive external ophthalmoplegia (CPEO) | osteoarthritis |
| Churg-Struass disease | osteoporosis |
| cognitive impairment due to a history of drug abuse | pain |
| cognitive impairment due to chemotherapy | panic disorder |
| cognitive impairment due to electroconvulsive shock therapy | pantothenate kinase associated neurodegeneration |
| cognitive impairment during waking hours due to sleep apnea | Parkinson's disease |
| complications from intracerebral hemorrhage | Parkinson's disease dementia |
| complications post anoxia | Pearson syndrome |
| concussions | pediatric Guillain - Barre syndrome |
| congenital SMA with arthrogryposis | periodic fever, aphthous stomatitis, pharyngitis, adenitis syndrome |
| conus and cauda equina medullaris syndromes | periodontal disease (PD) |
| corticobasal degeneration | phenylketonuria |
| Crohn's disease | Pick's disease |
| cryopyrin-associated periodic syndromes (CAPS) | polymyalgia rheumatica |
| deficiency in IL-1 receptor antagonist (DIRA) | post coronary artery by-pass graft surgery |
| dementia | Postmyocardial infarction heart failure |
| dementia pugilistica | post-polio syndrome (PPS) |
| dementia with Lewy bodies | post-traumatic stress disorder |
| dentatorubropallidouysian atrophy | prion disease (Creutzfeldt-Jakob disease) |
| depression | progressive bulbar palsy |
| diabetes mellitus type 1 | progressive muscular atrophy |
| diabetes mellitus type 2 | progressive supranuclear palsy |
| dialysis related amyloidosis | prolactinomas |
| diffuse Lewy body disease | protein and lipid accumulation due to normal aging |
| dopamine responsive dystonia | pseudobulbar palsy primary lateral sclerosis (PLS) |
| Down's syndrome | psoriasis |
| drop foot | pyogenic arthritis, pyoderma gangrenosum, acne (PAPA) |
| dyslexia | Rasmussen's encephalitis |
| Emory Dreifuss muscular dystrophy | recurrent idiopathic pericarditis |
| encephalitis (infectious encephalitis or autoimmune encephalitis) | relapsing chondritis |
| epilepsy | Rett's syndrome |
| Erdheim-Chester syndrome (histiocytosis) | Reynaud's syndrome |
| facroscapulohumerol dystrophy | rheumatoid arthritis |
| familial amyloid polyneuropathy | rosacea |
| familial Mediterranean fever (FMF) | schizophrenia |
| Fazio-Londe disease | Schnitzler syndrome |
| Finnish amyloidosis | senile dementia |
| folic acid deficiency | Sjorgen's syndrome and glomerulonephritis. |
| fragile X associated tremor/ataxia syndrome | sleep disorder |
| fragile X syndrome | smoldering multiple myeloma |
| fragile XE mental retardation | social phobia |
| Friedreich's ataxia | spinal muscular atrophy |
| frontal lobe syndrome | spinal muscular atrophy (SMA including SMA type I SMA type II and SMA type III) |
| frontotemporal dementia | spinobulbar muscular atrophy (Kennedy's disease) |
| frontotemporal dementia with Parkinsonism linked to chromosome 17 | spinocerebellar ataxis (types 1-8 10-14 16-29) |
| frontotemporal lobar degeneration | sporadic inclusion body myositis |
| ganglioglioma | steel-Richard syndrome |
| generalized anxiety | stiff person syndrome |
| glaucoma | storage diseases |
| hallervorden-spatz disease | stroke |
| hepatic conditions | subacute sclerosing panencephalitis |
| hereditary non-neuropathic systemic amyloidosis | syphillis |
| Huntington's disease | systemic AL amyloidosis |
| hydrocephalus | systemic lupus erythematosus |
| hyper IgD syndrome (HIDS) | tardive dyskinesia |
| hyperalgesia or sensory disorders | thiamine deficiency |
| hypercalcemia | thrombotic throbocytopenic purpura (TTP) |
| hyperkinesias | TNF receptor-associated periodic syndrome (TRAPS) |
| hypoglycemia | Tourette's syndrome |
| hypothyroidism | transmissible spongiform encephalopathy |
| idiopathic thrombocytopenic purpura (ITP) | transverse myelitis |
| IgA nephropathy | traumatic brain injury |
| IgM polyneuropathies | tuberous sclerosis |
| immune response/metabolic shifts | type 2 diabetes |
| inclusion body myositis | Urate crystal arthritis (gout) |
| Infantile myopathy and lactic acidosis (fatal & non-fatal forms) | Urticarial vasculitis |
| infantile scoliosis | vascular amyloidosis |
| infectious vasculitis | vascular dementia |
| inflammatory bowel disease | vasculitis |
| isolated atrial amyloidosis | Wegener's disease |
| juvenile arthritis | West disease |
| Kearns-Sayre syndrome (KSS) | Wilson's disease |
| Kennedy disease | |

In another embodiment, the present disclosure provides a method of treating a disease, disorder, condition, or syndrome in a subject in need thereof, or treating a symptom of a disease, disorder, condition, or syndrome in a subject in need thereof, the method comprising administering a therapeutically effective amount of a Compound of the Disclosure to the subject, wherein the diseases, disorders, conditions, or syndromes are any one or more of diseases, disorders, conditions, or syndromes provided in Table 2.

**Table 2**

| |
|---|
| accute optic neuritis |
| acute disseminated encephalomyelitis |
| addiction |
| adult and juvenile Still disease |
| age-related macular degeneration (ARMD) |
| ankylosing spondylitis |
| antisynthetase syndrome |
| Behçet disease |
| Blau syndrome; Sweet syndrome |
| cancer cachexia |
| cardiovascular disease |
| cardiovascular diseases |
| central nervous system (CNS) vasculitis |
| central nervous system regeneration |
| chronic obstructive pulmonary disease (COPD) |
| concussions |
| Crohn's disease |
| cryopyrin-associated periodic syndromes (CAPS) |
| deficiency in IL-1 receptor antagonist (DIRA) |
| diabetes |
| epilepsy |
| Erdheim-Chester syndrome (histiocytosis) |
| familial Mediterranean fever (FMF) |
| hyper IgD syndrome (HIDS) |
| immune response/metabolic shifts |
| inflammatory bowel disease |
| juvenile arthritis |
| macrophage activation syndrome (MAS) |
| microglial hyper-activation |
| migraine |
| neuroinflammation |
| neuromyelitis Optica |
| neurosarcoidosis |
| osteoarthritis |
| osteoporosis |
| pain |
| periodic fever, aphthous stomatitis, pharyngitis, adenitis syndrome (PFAPA) |
| periodontal disease (PD) |
| psoriasis (all skin disorders) |
| pyogenic arthritis, pyoderma gangrenosum, acne (PAPA) |
| Rasmussen's encephalitis |
| recurrent idiopathic pericarditis |
| relapsing chondritis |
| rheumatoid arthritis |
| rosacea |
| schizophrenia |
| Schnitzler syndrome |
| smoldering multiple myeloma |
| stroke |
| TNF receptor-associated periodic syndrome (TRAPS) |
| transverse Myelitis |
| traumatic brain injury |
| urate crystal arthritis (gout) |
| urticarial vasculitis |
| vasculitis |

In one embodiment, the present disclosure provides a method of treating a disease, disorder, condition, or syndrome in a subject in need thereof, or treating a symptom of a disease, disorder, condition, or syndrome in a subject in need thereof, the method comprising administering a therapeutically effective amount of a Compound of the Disclosure to the subject, wherein the diseases, disorders, conditions, or syndromes are any one or more of the diseases, disorders, conditions, or syndromes provided in Table 3.

**Table 3**

| |
|---|
| accute optic neuritis |
| acute disseminated encephalomyelitis |
| adrenoleukodystrophy |
| adult and juvenile Still disease |
| age-related macular degeneration (ARMD) |
| agryophilic grain disease |
| AIDS |
| AIDS-related dementia |
| Alzheimer's Disease |
| amyotrophic lateral sclerosis |
| ankylosing spondylitis |
| attention deficit disorder (ADD) |
| attention deficit hyperactivity disorder (ADHD) |
| autism |
| autoimmune thrombocytopenia |
| autoimmune vasculitis |
| Behcet disease |
| bovine spongiform encephalopathy |
| brain inflammation |
| Central nervous system (CNS) vasculitis |
| central nervous system regeneration |
| cerebral amyloid angiopathy (and Icelandic type) |
| cerebral haemorrhage with amyloidosis |
| cerebrovascular disease |
| chronic obstructive pulmonary disease |
| cognitive impairment due to a history of drug abuse |
| cognitive impairment due to chemotherapy |
| cognitive impairment due to electroconvulsive shock therapy |
| cognitive impairment during waking hours due to sleep apnea |
| complications post anoxia |
| concussions |
| corticobasal degeneration |
| Crohn's disease |
| deficiency in IL-1 receptor antagonist (DIRA) |
| dementia |
| dementia with Lewy bodies |
| dentatorubropallidouysian atrophy |
| depression |
| diffuse Lewy body disease |
| Down's syndrome |
| dyslexia |
| epilepsy |
| fragile X syndrome |
| fragile XE mental retardation |
| frontal lobe syndrome |
| frontotemporal dementia with Parkinsonism linked to chromosome 17 |
| hallervorden-spatz disease |
| Huntington's disease |
| hyperalgesia or sensory disorders |
| inclusion body myositis |
| juvenile arthritis |
| lewy body dementia: already covered |
| lewy body disease |
| lewy body mutant of Alzheimer' s disease |
| microglial hyper-activation |
| mild cognitive impairment |
| mitochondrial disorders |
| multiple sclerosis |
| myotonic dystrophy |
| neuroinflammation |
| neuromyelitis optica |
| obsessive compulsive disorder |
| pain |
| panic disorder |
| Parkinson's disease dementia |
| Pick's disease: Fronto-temporal dementia |
| progressive supranuclear palsy |
| protein and lipid accumulation due to normal aging: antiaging! |
| pseudobulbar palsy primary lateral sclerosis (PLS) |
| psoriasis |
| pyogenic arthritis, pyoderma gangrenosum, acne (PAPA) |
| sleep disorder, e.g., difficulty with falling asleep and/or staying asleep, fatigue, drowsiness, sleep apnea, narcolepsy, and/or sleepiness, e.g., excessive daytime sleepiness |
| steel-Richard syndrome |
| pransverse Myelitis |
| traumatic brain injury |
| vascular dementia |

In another embodiment, the present disclosure provides a method of treating a neuroinflammatory disease, disorder, condition, or syndrome in a subject in need thereof, or treating a symptom of a neuroinflammatory disease, disorder, condition, or syndrome in a subject in need thereof, the method comprising administering a therapeutically effective amount of a Compound of the Disclosure to the subject.

In another embodiment, the present disclosure provides a method of treating a disease, disorder, condition, or syndrome in a subject in need thereof, or treating a symptom of a disease, disorder, condition, or syndrome in a subject in need thereof, the method comprising administering a therapeutically effective amount of a Compound of the Disclosure, to the subject, wherein the diseases, disorders, conditions, or syndromes are any one or more of the diseases, disorders, conditions, or syndromes provided in Table 4.

**Table 4**

| |
|---|
| accute optic neuritis |
| acute disseminated encephalomyelitis |
| addiction |
| adult and juvenile Still disease |
| age-related macular degeneration (ARMD) |
| Alzheimer's Disease |
| amyotrophic lateral sclerosis |
| ankylosing spondylitis |
| antisynthetase syndrome |
| autism |
| Behçet disease |
| Blau syndrome; Sweet syndrome |
| cancer cachexia |
| cardiovascular disease |
| central nervous system (CNS) vasculitis |
| central nervous system regeneration |
| Chronic obstructive pulmonary disease |
| concussions |
| Crohn's disease |
| cryopyrin-associated periodic syndromes (CAPS) |
| deficiency in IL-1 receptor antagonist (DIRA) |
| depression |
| diabetes |
| epilepsy |
| Erdheim-Chester syndrome (histiocytosis) |
| damilial Mediterranean fever (FMF) |
| frontotemporal dementia |
| Huntington's disease |
| hyper IgD syndrome (HIDS) |
| immune response/metabolic shifts |
| inflammatory bowel disease |
| juvenile arthritis |
| lewy body dementia |
| macrophage activation syndrome (MAS) |
| microglial hyper-activation |
| migraine |
| mitochondrial disorders |
| multiple sclerosis |
| neuroinflammation |
| neuromyelitis Optica |
| neuropsychiatric disorders |
| neurosarcoidosis |
| osteoarthritis |
| osteoporosis |
| pain |
| Parkinson's disease |
| periodic fever, aphthous stomatitis, pharyngitis, adenitis syndrome |
| periodontal disease (PD) |
| postmyocardial infarction heart failure |
| prion disease |
| psoriasis |
| pyogenic arthritis, pyoderma gangrenosum, acne (PAPA) |
| Rasmussen's encephalitis |
| recurrent idiopathic pericarditis |
| relapsing chondritis |
| rheumatoid arthritis |
| rosacea |
| schizophrenia |
| schnitzler syndrome |
| smoldering multiple myeloma |
| stroke |
| TNF receptor-associated periodic syndrome (TRAPS) |
| transverse Myelitis |
| traumatic brain injury |
| type 2 diabetes |
| urate crystal arthritis (gout) |
| urticarial vasculitis |
| vasculitis |

In another embodiment, the present disclosure provides a method of treating a disease, disorder, condition, or syndrome in a subject in need thereof, or treating a symptom of a disease, disorder, condition, or syndrome in a subject in need thereof, the method comprising administering a therapeutically effective amount of a Compound of the Disclosure to the subject, wherein the diseases, disorders, conditions, or syndromes are any one or more of the diseases, disorders, conditions, or syndromes provided in Table 5. In another embodiment, the present disclosure provides a method of treating traumatic brain injury.

**Table 5**

| |
|---|
| accute optic neuritis |
| acute disseminated encephalomyelitis |
| adult and juvenile Still disease |
| amyotrophic lateral sclerosis |
| ankylosing spondylitis |
| antisynthetase syndrome |
| autism |
| Behcet disease |
| Blau syndrome; Sweet syndrome |
| cancer cachexia |
| central nervous system (CNS) vasculitis |
| central nervous system regeneration |
| chronic obstructive pulmonary disease |
| concussions |
| Crohn's disease |
| cryopyrin-associated periodic syndromes (CAPS) |
| deficiency in IL-1 receptor antagonist (DIRA) |
| epilepsy |
| Erdheim-Chester syndrome (histiocytosis) |
| damilial Mediterranean fever (FMF) |
| hyper IgD syndrome (HIDS) |
| immune response/metabolic shifts |
| inflammatory bowel disease |
| juvenile arthritis |
| macrophage activation syndrome (MAS) |
| microglial hyper-activation |
| migraine |
| neuroinflammation |
| neuromyelitis optica |
| neurosarcoidosis |
| osteoarthritis |
| osteoporosis |
| pain |
| Parkinson's disease |
| periodic fever, aphthous stomatitis, pharyngitis, adenitis syndrome |
| periodontal disease (PD) |
| postmyocardial infarction heart failure |
| prion disease |
| psoriasis |
| pyogenic arthritis, pyoderma gangrenosum, acne (PAPA) |
| Rasmussen's encephalitis |
| recurrent idiopathic pericarditis |
| relapsing chondritis |
| rheumatoid arthritis |
| rosacea |
| schizophrenia |
| schnitzler syndrome |
| smoldering multiple myeloma |
| stroke |
| TNF receptor-associated periodic syndrome (TRAPS) |
| transverse Myelitis |
| traumatic brain injury |
| type 2 diabetes |
| urate crystal arthritis (gout) |
| urticarial vasculitis |
| vasculitis |

In another embodiment, the present disclosure provides a method of treating a neurological disease, disorder, condition, or syndrome in a subject in need thereof, or treating a symptom of a neurological disease, disorder, condition, or syndrome in a subject in need thereof, the method comprising administering a therapeutically effective amount of a Compound of the Disclosure to the subject.

In another embodiment, the present disclosure provides a method of treating a disease, disorder, condition, or syndrome in a subject in need thereof, or treating a symptom of a disease, disorder, condition, or syndrome in a subject in need thereof, the method comprising administering a therapeutically effective amount of a Compound of the Disclosure to the subject, wherein the diseases, disorders, conditions, or syndromes are any one or more of the diseases, disorders, conditions, or syndromes provided in Table 6.

**Table 6**

| |
|---|
| acute confusion disorders in which apoptotic necrocytosis plays a part |
| acute confusion disorders |
| adrenoleukodystrophy |
| agryophilic grain disease |
| AIDS |
| AIDS-related dementia |
| Alzheimer's disease |
| amyotrophic lateral sclerosis |
| aortic medial amyloid |
| apathy |
| atherosclerosis |
| attention deficit disorder (ADD) |
| attention deficit hyperactivity disorder (ADHD) |
| autism |
| autoimmune vasculitis |
| B12 deficiency |
| bipolar disorder |
| bovine spongiform encephalopathy |
| brain inflammation |
| brain lesions |
| brain neoplasms |
| cardiac arrythmias |
| cerebral amyloid angiopathy (and Icelandic type) |
| cerebral haemorrhage with amyloidosis |
| cerebrovascular disease |
| cognitive impairment due to a history of drug abuse |
| cognitive impairment due to chemotherapy |
| cognitive impairment due to electroconvulsive shock therapy |
| cognitive impairment during waking hours due to sleep apnea |
| complications from intracerebral hemorrhage |
| complications post anoxia |
| congenital SMA with arthrogryposis |
| corticobasal degeneration |
| dementia pugilistica |
| dementia with Lewy bodies |
| dementia |
| dentatorubropallidouysian atrophy |
| depression |
| diabetes mellitus type 2 |
| dialysis related amyloidosis |
| diffuse Lewy body disease |
| Down's syndrome |
| dyslexia |
| epilepsy |
| familial amyloid polyneuropathy |
| Fazio-Londe disease |
| Finnish amyloidosis |
| folic acid deficiency |
| fragile X associated tremor/ataxia syndrome |
| fragile X syndrome |
| fragile XE mental retardation |
| Friedrich's ataxia |
| frontal lobe syndrome |
| frontotemporal dementia with Parkinsonism linked to chromosome 17 |
| frontotemporal lobar degeneration |
| ganglioglioma |
| glaucoma |
| hallervorden-spatz disease |
| hepatic conditions |
| hereditary non-neuropathic systemic amyloidosis |
| Huntington's disease |
| hydrocephalus |
| hyperalgesia or sensory disorders |
| hypercalcemia |
| hyperkinesias |
| hypoglycemia |
| hypothyroidism |
| inclusion body myositis |
| infectious vasculitis |
| isolated atrial amyloidosis |
| Kufor Rakeb disease |
| Kufs' disease |
| lattice corneal dystrophy |
| lead enphalapathy |
| lewy body disease |
| lewy body mutant of Alzheimer' s disease |
| lipofuscinosis |
| Lyme disease |
| malnutrition |
| mania |
| maple syrup urine disease |
| medullary carcinoma of the thyroid |
| meningioangiomatosis |
| metabolic diseases |
| mild cognitive impairment |
| mild cognitive impairment |
| morbus Parkinson |
| multi-infarct dementia |
| multiple sclerosis |
| multiple system atrophy |
| myasthenia gravis |
| myotonic dystrophy |
| nerve trauma |
| neurodegeneration with brain iron accumulation type I |
| neurofibromatosis |
| neurological disorder is generalized anxiety disorders |
| neurosyphillis |
| niacin deficiency |
| obsessive compulsive disorder |
| Parkinson's disease dementia |
| Parkinson's disease |
| phenylketonuria |
| Pick's disease |
| polymyalgia rheumatica |
| post coronary artery by-pass graft surgery |
| post-polio syndrome (PPS) |
| post-traumatic stress disorder |
| prion disease (Creutzfeldt-Jakob disease) |
| progressive bulbar palsy |
| progressive muscular atrophy |
| progressive supranuclear palsy |
| prolactinomas |
| protein and lipid accumulation due to normal aging |
| pseudobulbar palsy primary lateral sclerosis (PLS) |
| Rett's syndrome |
| rheumatoid arthritis |
| schizophrenia |
| senile dementia |
| sleep disorder |
| spinal muscular atrophy (SMA including SMA type I SMA type II and SMA type III) |
| spinobulbar muscular atrophy (Kennedy's disease) |
| spinocerebellar ataxis (types 1-8 10-14 16-29) |
| sporadic inclusion body myositis |
| steel-Richard syndrome |
| storage diseases |
| stroke |
| subacute sclerosing panencephalitis |
| syphillis |
| systemic AL amyloidosis |
| systemic lupus erythematosus |
| tardive dyskinesia |
| thiamine deficiency |
| Tourette's syndrome |
| transmissible spongiform encephalopathy |
| traumatic brain injury |
| tuberous sclerosis |
| vascular amyloidosis |
| vascular dementia. |

In another embodiment, the present disclosure provides a method of treating a disease, disorder, condition, or syndrome in a subject in need thereof, or treating a symptom of a disease, disorder, condition, or syndrome in a subject in need thereof, the method comprising administering a therapeutically effective amount of a Compound of the Disclosure to the subject, wherein the diseases, disorders, conditions, or syndromes are any one or more of the diseases, disorders, conditions, or syndromes provided in Table 7.

**Table 7**

| |
|---|
| acute confusion disorders |
| adrenoleukodystrophy |
| agryophilic grain disease |
| aortic medial amyloid |
| apathy |
| atherosclerosis |
| attention deficit disorder (ADD) |
| attention deficit hyperactivity disorder (ADHD) |
| autism |
| autoimmune vasculitis |
| bipolar disorder |
| bovine spongiform encephalopathy |
| brain inflammation |
| brain lesions |
| brain neoplasms |
| cerebral amyloid angiopathy (and Icelandic type) |
| cerebral haemorrhage with amyloidosis |
| cerebrovascular disease |
| cognitive impairment due to a history of drug abuse |
| cognitive impairment due to chemotherapy |
| cognitive impairment due to electroconvulsive shock therapy |
| cognitive impairment during waking hours due to sleep apnea |
| complications from intracerebral hemorrhage |
| complications post anoxia |
| congenital SMA with arthrogryposis |
| corticobasal degeneration |
| dementia pugilistica |
| dementia |
| dentatorubropallidouysian atrophy |
| dialysis related amyloidosis |
| Down's syndrome |
| dyslexia |
| epilepsy |
| familial amyloid polyneuropathy |
| Fazio-Londe disease |
| Finnish amyloidosis |
| folic acid deficiency |
| fragile X associated tremor/ataxia syndrome |
| fragile X syndrome |
| fragile XE mental retardation |
| Friedrich's ataxia |
| frontal lobe syndrome |
| frontotemporal dementia with Parkinsonism linked to chromosome 17 |
| frontotemporal lobar degeneration |
| ganglioglioma |
| glaucoma |
| hallervorden-spatz disease |
| hepatic conditions |
| hereditary non-neuropathic systemic amyloidosis |
| Huntington's disease |
| hydrocephalus |
| hyperalgesia or sensory disorders |
| hypercalcemia |
| hyperkinesias |
| hypoglycemia |
| hypothyroidism |
| inclusion body myositis |
| infectious vasculitis |
| isolated atrial amyloidosis |
| Kufor Rakeb disease |
| Kufs' disease |
| lattice corneal dystrophy |
| lead enphalapathy |
| lewy body disease |
| lewy body mutant of Alzheimer' s disease |
| lipofuscinosis |
| Lyme disease |
| mania |
| maple syrup urine disease |
| medullary carcinoma of the thyroid |
| meningioangiomatosis |
| metabolic diseases |
| mild cognitive impairment |
| multi-infarct dementia |
| multiple system atrophy |
| myasthenia gravis |
| myotonic dystrophy |
| nerve trauma |
| neurodegeneration with brain iron accumulation type I |
| neurofibromatosis |
| neurological disorder is generalized anxiety disorders |
| neurosyphillis |
| niacin deficiency |
| obsessive compulsive disorder |
| phenylketonuria |
| Pick's disease |
| polymyalgia rheumatica |
| post coronary artery by-pass graft surgery |
| post-polio syndrome (PPS) |
| post-traumatic stress disorder |
| prion disease (Creutzfeldt-Jakob disease) |
| progressive bulbar palsy |
| progressive muscular atrophy |
| progressive supranuclear palsy |
| prolactinomas |
| protein and lipid accumulation due to normal aging |
| pseudobulbar palsy primary lateral sclerosis (PLS) |
| Rett's syndrome |
| rheumatoid arthritis |
| schizophrenia |
| senile dementia |
| sleep disorder |
| spinal muscular atrophy (SMA including SMA type I SMA type II and SMA type III) |
| spinobulbar muscular atrophy (Kennedy's disease) |
| sporadic inclusion body myositis |
| steel-Richard syndrome |
| storage diseases |
| stroke |
| subacute sclerosing panencephalitis |
| syphillis |
| systemic AL amyloidosis |
| systemic lupus erythematosus |
| tardive dyskinesia |
| thiamine deficiency |
| Tourette's syndrome |
| transmissible spongiform encephalopathy |
| tuberous sclerosis |

In another embodiment, the present disclosure provides a method of treating an autoimmune disease, disorder, condition, or syndrome in a subject in need thereof, or treating a symptom of a autoimmune disease, disorder, condition, or syndrome in a subject in need thereof, the method comprising administering a therapeutically effective amount of a Compound of the Disclosure to the subject.

In another embodiment, the present disclosure provides a method of treating a disease, disorder, condition, or syndrome in a subject in need thereof, or treating a symptom of a disease, disorder, condition, or syndrome in a subject in need thereof, the method comprising administering a therapeutically effective amount of a Compound of the Disclosure to the subject, wherein the diseases, disorders, conditions, or syndromes are any one or more of the diseases, disorders, conditions, or syndromes provided in Table 8.

**Table 8**

| |
|---|
| autoimmune thrombocytopenia |
| diabetes mellitus |
| idiopathic thrombocytopenic purpura (ITP) |
| IgA nephropathy |
| IgM polyneuropathies |
| inflammatory bowel disease |
| juvenile rheumatoid arthritis |
| lupus nephritis |
| multiple sclerosis |
| myasthenia gravis |
| psoriasis |
| Reynaud's syndrome |
| rheumatoid arthritis |
| Sjorgen's syndrome and glomerulonephritis. |
| systemic lupus erythematosus (SLE) |
| thrombotic throbocytopenic purpura (TTP) |
| vasculitis |
| Wegener's disease |

In another embodiment, the present disclosure provides a method of treating a disease, disorder, condition, or syndrome in a subject in need thereof, or treating a symptom of a disease, disorder, condition, or syndrome in a subject in need thereof, the method comprising administering a therapeutically effective amount of a Compound of the Disclosure to the subject, wherein the diseases, disorders, conditions, or syndromes are any one or more of the diseases, disorders, conditions, or syndromes provided in Table 9.

**Table 9**

| |
|---|
| autoimmune thrombocytopenia |
| idiopathic thrombocytopenic purpura (ITP) |
| IgA nephropathy |
| IgM polyneuropathies |
| inflammatory bowel disease |
| lupus nephritis |
| myasthenia gravis |
| psoriasis |
| Reynaud's syndrome |
| rheumatoid arthritis |
| Sjorgen's syndrome and glomerulonephritis. |
| systemic lupus erythematosus (SLE) |
| thrombotic throbocytopenic purpura (TTP) |
| Wegener's disease |

In another embodiment, the present disclosure provides a method of treating a neurodegenerative proteinopathic disease, disorder, condition, or syndrome in a subject in need thereof, or treating a symptom of a neurodegenerative proteinopathic disease, disorder, condition, or syndrome in a subject in need thereof, the method comprising administering a therapeutically effective amount of a Compound of the Disclosure to the subj ect.

In another embodiment, the present disclosure provides a method of treating a disease, disorder, condition, or syndrome in a subject in need thereof, or treating a symptom of a disease, disorder, condition, or syndrome in a subject in need thereof, the method comprising administering a therapeutically effective amount of a Compound of the Disclosure to the subject, wherein the diseases, disorders, conditions, or syndromes are any one or more of the diseases, disorders, conditions, or syndromes provided in Table 10.

**Table 10**

| |
|---|
| adrenoleukodystrophy |
| agryophilic grain disease |
| AIDS and AIDS-related dementia |
| Alzheimer's disease |
| amyotrophic lateral sclerosis (Parkinsonism-dementia complex of Guam or Lytico-Bodig disease) |
| vascular dementia |
| aortic medial amyloid |
| apathy |
| atherosclerosis |
| attention deficit disorder (ADD) |
| attention deficit hyperactivity disorder (ADHD) |
| autism |
| autoimmune vasculitis |
| B12 deficiency |
| bipolar disorder |
| bovine spongiform encephalopathy |
| brain lesions |
| brain neoplasms |
| cardiac arrythmias |
| cerebral amyloid angiopathy (and Icelandic type) |
| cerebrovascular disease |
| cognitive impairment due to a history of drug abuse |
| cognitive impairment due to chemotherapy |
| cognitive impairment due to electroconvulsive shock therapy |
| cognitive impairment during waking hours due to sleep apnea |
| complications from intracerebral hemorrhage |
| complications post anoxia |
| corticobasal degeneration |
| dementia pugilistica |
| dementia with Lewy bodies |
| dentatorubropallidouysian atrophy |
| depression |
| diabetes mellitus type 2 |
| dialysis related amyloidosis |
| diffuse Lewy body disease |
| Down's syndrome |
| dyslexia |
| epilepsy |
| familial amyloid polyneuropathy |
| Finnish amyloidosis |
| folic acid deficiency |
| fragile X associated tremor/ataxia syndrome |
| fragile X syndrome |
| fragile XE mental retardation |
| Friedrich's ataxia |
| frontal lobe syndrome |
| frontotemporal dementia with Parkinsonism linked to chromosome 17 |
| frontotemporal lobar degeneration |
| ganglioglioma |
| hallervorden-spatz disease |
| hepatic conditions |
| hereditary non-neuropathic systemic amyloidosis |
| Huntington's disease |
| hydrocephalus |
| hypercalcemia |
| hypoglycemia |
| hypothyroidism |
| inclusion body myositis |
| infectious vasculitis |
| isolated atrial amyloidosis |
| Kufor Rakeb disease |
| Kufs' disease |
| lattice corneal dystrophy |
| lead enphalapathy |
| lewy body disease |
| lewy body mutant of Alzheimer' s disease |
| lipofuscinosis |
| Lyme disease |
| malnutrition |
| maple syrup urine disease |
| medullary carcinoma of the thyroid |
| meningioangiomatosis |
| metabolic diseases |
| mild cognitive impairment |
| multi-infarct dementia |
| multiple sclerosis |
| multiple system atrophy |
| myasthenia gravis |
| myotonic dystrophy |
| neurodegeneration with brain iron accumulation type I |
| neurofibromatosis |
| neurosyphillis |
| niacin deficiency |
| Parkinson's disease and Parkinson's disease dementia |
| phenylketonuria |
| Pick's disease |
| polymyalgia rheumatica |
| post coronary artery by-pass graft surgery |
| post-traumatic stress disorder |
| prion disease (Creutzfeldt-Jakob disease) |
| progressive supranuclear palsy |
| prolactinomas |
| protein and lipid accumulation due to normal aging |
| Rett's syndrome |
| rheumatoid arthritis |
| schizophrenia |
| spinobulbar muscular atrophy (Kennedy's disease) |
| spinocerebellar ataxis |
| sporadic inclusion body myositis |
| storage diseases |
| stroke |
| subacute sclerosing panencephalitis |
| syphillis |
| systemic AL amyloidosis |
| systemic lupus erythematosus |
| thiamine deficiency |
| Tourette's syndrome |
| transmissible spongiform encephalopathy |
| traumatic brain injury |
| tuberous sclerosis |

In another embodiment, the present disclosure provides a method of treating a disease, disorder, condition, or syndrome in a subject in need thereof, or treating a symptom of a disease, disorder, condition, or syndrome in a subject in need thereof, the method comprising administering a therapeutically effective amount of a Compound of the Disclosure to the subject, wherein the diseases, disorders, conditions, or syndromes are any one or more of the diseases, disorders, conditions, or syndromes provided in Table 11.

**Table 11**

| |
|---|
| adrenoleukodystrophy |
| agryophilic grain disease |
| vascular dementia |
| aortic medial amyloid |
| apathy |
| atherosclerosis |
| attention deficit disorder (ADD) |
| attention deficit hyperactivity disorder (ADHD) |
| autism |
| autoimmune vasculitis |
| B12 deficiency |
| bipolar disorder |
| bovine spongiform encephalopathy |
| brain lesions |
| brain neoplasms |
| cardiac arrythmias |
| cerebral amyloid angiopathy (and Icelandic type) |
| cerebrovascular disease |
| cognitive impairment due to a history of drug abuse |
| cognitive impairment due to chemotherapy |
| cognitive impairment due to electroconvulsive shock therapy |
| cognitive impairment during waking hours due to sleep apnea |
| complications from intracerebral hemorrhage |
| complications post anoxia |
| corticobasal degeneration |
| dementia pugilistica |
| dentatorubropallidouysian atrophy |
| dialysis related amyloidosis |
| diffuse Lewy body disease |
| Down's syndrome |
| dyslexia |
| epilepsy |
| familial amyloid polyneuropathy |
| Finnish amyloidosis |
| folic acid deficiency |
| fragile X associated tremor/ataxia syndrome |
| fragile X syndrome |
| fragile XEmental retardation |
| Friedrich's ataxia |
| frontal lobe syndrome |
| frontotemporal dementia with Parkinsonism linked to chromosome 17 |
| frontotemporal lobar degeneration |
| ganglioglioma |
| hallervorden-spatz disease |
| hereditary non-neuropathic systemic amyloidosis |
| Huntington's disease |
| hydrocephalus |
| hypercalcemia |
| hypoglycemia |
| hypothyroidism |
| inclusion body myositis |
| infectious vasculitis |
| isolated atrial amyloidosis |
| Kufor Rakeb disease |
| Kufs' disease |
| lattice corneal dystrophy |
| lead enphalapathy |
| lewy body mutant of Alzheimer' s disease |
| lipofuscinosis |
| Lyme disease |
| malnutrition |
| maple syrup urine disease |
| medullary carcinoma of the thyroid |
| meningioangiomatosis |
| mild cognitive impairment |
| multi-infarct dementia |
| multiple system atrophy |
| myasthenia gravis |
| myotonic dystrophy |
| neurodegeneration with brain iron accumulation type I |
| neurofibromatosis |
| neurosyphillis |
| phenylketonuria |
| polymyalgia rheumatica |
| post coronary artery by-pass graft surgery |
| post-traumatic stress disorder |
| prion disease (Creutzfeldt-Jakob disease) |
| progressive supranuclear palsy |
| prolactinomas |
| Rett's syndrome |
| rheumatoid arthritis |
| schizophrenia |
| spinobulbar muscular atrophy (Kennedy's disease) |
| sporadic inclusion body myositis |
| subacute sclerosing panencephalitis |
| syphillis |
| systemic AL amyloidosis |
| systemic lupus erythematosus |
| thiamine deficiency |
| Tourette's syndrome |
| transmissible spongiform encephalopathy |

In another embodiment, the present disclosure provides a method of treating a psychiatric disease, disorder, condition, or syndrome in a subject in need thereof, or treating a symptom of a psychiatric disease, disorder, condition, or syndrome in a subject in need thereof, the method comprising administering a therapeutically effective amount of a Compound of the Disclosure to the subject

In another embodiment, the present disclosure provides a method of treating a disease, disorder, condition, or syndrome in a subject in need thereof, or treating a symptom of a disease, disorder, condition, or syndrome in a subject in need thereof, the method comprising administering a therapeutically effective amount of a Compound of the Disclosure to the subject, wherein the diseases, disorders, conditions, or syndromes are any one or more of the diseases, disorders, conditions, or syndromes provided in Table 12.

**Table 12**

| |
|---|
| depression |
| generalized anxiety |
| major depression |
| obsessive-compulsive disorder |
| panic disorder |
| post-traumatic stress |
| social phobia |

In another embodiment, the present disclosure provides a method of treating a disease, disorder, condition, or syndrome in a subject in need thereof, or treating a symptom of a disease, disorder, condition, or syndrome in a subject in need thereof, the method comprising administering a therapeutically effective amount of a Compound of the Disclosure to the subject, wherein the diseases, disorders, conditions, or syndromes are any one or more of the diseases, disorders, conditions, or syndromes provided in Table 13.

**Table 13**

| |
|---|
| generalized anxiety |
| obsessive-compulsive disorder |
| panic disorder |
| post-traumatic stress |
| social phobia |

In another embodiment, the present disclosure provides a method of treating a mitochondrial disease, disorder, condition, or syndrome in a subject in need thereof, or treating a symptom of a mitochondrial disease, disorder, condition, or syndrome in a subject in need thereof, the method comprising administering a therapeutically effective amount of a Compound of the Disclosure to the subject.

In another embodiment, the present disclosure provides a method of treating a disease, disorder, condition, or syndrome in a subject in need thereof, or treating a symptom of a disease, disorder, condition, or syndrome in a subject in need thereof, the method comprising administering a therapeutically effective amount of a Compound of the Disclosure to the subject, wherein the diseases, disorders, conditions, or syndromes are any one or more of the diseases, disorders, conditions, or syndromes provided in Table 14.

**Table 14**

| |
|---|
| Alpers-Huttenlocher syndrome |
| Ataxia neuropathy syndromes (ANS), e.g., mitochondrial recessive ataxia syndrome (MIRAS), spinocerebellar ataxia with epilepsy (SCAE), sensory ataxia neuropathy, dysarthria, ophthalmoplegia (SANDO), myoclonic epilepsy myopathy sensory ataxia (MEMSA) |
| chronic progressive external ophthalmoplegia (CPEO) |
| infantile myopathy and lactic acidosis (fatal & non-fatal forms) |
| Kearns-Sayre syndrome (KSS) |
| Leber hereditary optic neuropathy (LHON) |
| Leigh syndrome |
| mitochondrial encephalomyopathy with lactic acidosis and stroke-like episodes (MELAS) |
| myoclonic epilepsy with ragged-red fibers (MERRF) |
| neurogenic weakness with ataxia and retinitis pigmentosa (NARP) |
| Pearson syndrome |
| Myoneurogenic gastrointestinal encephalopathy (MNGIE) |
| spinal muscular atrophy |
| Friedreich's ataxia |
| Charcot-Marie-Tooth type 2K |
| Wilson's disease |

In another embodiment, the present disclosure provides a method of treating a disease, disorder, condition, or syndrome in a subject in need thereof, or treating a symptom of a disease, disorder, condition, or syndrome in a subject in need thereof, the method comprising administering a therapeutically effective amount of a Compound of the Disclosure to the subject, wherein the diseases, disorders, conditions, or syndromes are any one or more of the diseases, disorders, conditions, or syndromes provided in Table 15.

**Table 15**

| |
|---|
| Alpers-Huttenlocher syndrome |
| Ataxia neuropathy syndromes (ANS), e.g., mitochondrial recessive ataxia syndrome (MIRAS), spinocerebellar ataxia with epilepsy (SCAE), sensory ataxia neuropathy, dysarthria, ophthalmoplegia (SANDO), myoclonic epilepsy myopathy sensory ataxia (MEMSA) |
| chronic progressive external ophthalmoplegia (CPEO) |
| infantile myopathy and lactic acidosis (fatal & non-fatal forms) |
| Kearns-Sayre syndrome (KSS) |
| Leber hereditary optic neuropathy (LHON) |
| Leigh syndrome |
| mitochondrial encephalomyopathy with lactic acidosis and stroke-like episodes (MELAS) |
| myoclonic epilepsy with ragged-red fibers (MERRF) |
| neurogenic weakness with ataxia and retinitis pigmentosa (NARP) |
| Pearson syndrome |
| Myoneurogenic gastrointestinal encephalopathy (MNGIE) |

In another embodiment, the present disclosure provides a method of treating a disease, disorder, condition, or syndrome in a subject in need thereof, or treating a symptom of a disease, disorder, condition, or syndrome in a subject in need thereof, the method comprising administering a therapeutically effective amount of a Compound of the Disclosure to the subject, wherein the diseases, disorders, conditions, or syndromes are any one or more of the diseases, disorders, conditions, or syndrome provided in Table 16.

**Table 16**

| |
|---|
| spinal muscular atrophy |
| Friedreich's ataxia |
| Charcot-Marie-Tooth type 2K |
| Wilson's disease |

In another embodiment, the present disclosure provides a method of treating a disease, disorder, condition, or syndrome in a subject in need thereof, or treating a symptom of a disease, disorder, condition, or syndrome in a subject in need thereof, the method comprising administering a therapeutically effective amount of a Compound of the Disclosure to the subject, wherein the diseases, disorders, conditions, or syndromes are any one or more of the diseases, disorders, conditions, or syndromes provided in Table 17.

**Table 17**

| |
|---|
| acoustic neuroma |
| anencephaly |
| batten disease |
| catatonia |
| cerebral palsy |
| chorea in children and adults |
| chromosome 8P |
| Churg-Struass disease |
| conus and cauda equina medullaris Syndromes |
| dopamine responsive dystonia |
| drop Foot |
| facroscapulohumerol dystrophy |
| infantile scoliosis |
| Kennedy disease |
| Klippel-Feil syndrome |
| labrynthitis |
| Lambert-Eaton myasthenic syndrome (LEMS) |
| Lennox Gastaut syndromes |
| Lesch-Nyhan Disease |
| Menkes Disease |
| Moyamoya Disease |
| olivopontocerebellar atrophy (OPCA) |
| pantothenate kinase associated neurodegeneration |
| pediatric Guillain - Barre syndrome |
| stiff person syndrome |
| West Disease |
| Emory dreifuss muscular dystrophy |

In another embodiment, the present disclosure provides a method of treating a disease, disorder, condition, or syndrome, e.g., a disease, disorder, condition, or syndrome provided in any one of Tables 1-17, in a subject in need thereof, or treating a symptom of a disease, disorder, condition, or syndrome in a subject in need thereof, the method comprising administering about 1 g to about 30 g of a Compound of the Disclosure is administered to the subject per day. In another embodiment, about 2 g to about 15 g of a Compound of the Disclosure is administered to the subject per day. In another embodiment, about 3 g to about 10 g of a Compound of the Disclosure is administered is administered to the subject per day. In another embodiment, about 4 g to about 8 g of a Compound of the Disclosure is administered to the subject per day. In another embodiment, about 4 g to about 5 g of a Compound of the Disclosure is administered to the subject per day. In another embodiment, about 15.1 g to about 30 g of a Compound of the Disclosure is administered to the subject per day. In another embodiment, about 5 g of a Compound of the Disclosure is administered to the subject per day.

In another embodiment, the present disclosure provides a method of treating a disease, disorder, condition, or syndrome, e.g., a disease, disorder, condition, or syndrome provided in any one of Tables 1-17, in a subject in need thereof, or treating a symptom of a disease, disorder, condition, or syndrome in a subject in need thereof, the method comprising administering a therapeutically effective amount of DL-leucine, or a pharmaceutically acceptable salt thereof, is administered to the subject. In another embodiment, a therapeutically effective amount of DL-leucine, or a pharmaceutically acceptable salt thereof, is administered to the subject to treat the disease, disorder, condition, or syndrome. In another embodiment, a therapeutically effective amount of DL-leucine, or a pharmaceutically acceptable salt thereof, is administered to the subject to treat the symptom of the disease, disorder, condition, or syndrome.

In another embodiment, the present disclosure provides a method of treating a disease, disorder, condition, or syndrome, e.g., a disease, disorder, condition, or syndrome provided in any one of Tables 1-17, in a subject in need thereof, or treating a symptom of a disease, disorder, condition, or syndrome in a subject in need thereof, the method comprising administering a therapeutically effective amount of L-leucine, or a pharmaceutically acceptable salt thereof, is administered to the subject. In another embodiment, a therapeutically effective amount of L-leucine, or a pharmaceutically acceptable salt thereof, is administered to the subject to treat the disease, disorder, condition, or syndrome. In another embodiment, a therapeutically effective amount of L-leucine, or a pharmaceutically acceptable salt thereof, is administered to the subject to treat the symptom of the disease, disorder, condition, or syndrome.

In another embodiment, the present disclosure provides a method of treating a disease, disorder, condition, or syndrome, e.g., a disease, disorder, condition, or syndrome provided in any one of Tables 1-17, in a subject in need thereof, or treating a symptom of a disease, disorder, condition, or syndrome in a subject in need thereof, the method comprising administering a therapeutically effective amount of D-leucine, or a pharmaceutically acceptable salt thereof, is administered to the subject. In another embodiment, a therapeutically effective amount of D-leucine, or a pharmaceutically acceptable salt thereof, is administered to the subject to treat the disease, disorder, condition, or syndrome. In another embodiment, a therapeutically effective amount of D-leucine, or a pharmaceutically acceptable salt thereof, is administered to the subject to treat the symptom of the disease, disorder, condition, or syndrome.

In another embodiment, the present disclosure provides a method of treating a disease, disorder, condition, or syndrome, e.g., a disease, disorder, condition, or syndrome provided in any one of Tables 1-17, in a subject in need thereof, or treating a symptom of a disease, disorder, condition, or syndrome in a subject in need thereof, the method comprising administering a therapeutically effective amount of L-leucine ethyl ester, or a pharmaceutically acceptable salt thereof, is administered to the subject. In another embodiment, a therapeutically effective amount of L-leucine ethyl ester, or a pharmaceutically acceptable salt thereof, is administered to the subject to treat the disease, disorder, condition, or syndrome. In another embodiment, a therapeutically effective amount of L-leucine ethyl ester, or a pharmaceutically acceptable salt thereof, is administered to the subject to treat the symptom of the disease, disorder, condition, or syndrome.

In another embodiment, the present disclosure provides a method of treating a disease, disorder, condition, or syndrome, e.g., a disease, disorder, condition, or syndrome provided in any one of Tables 1-17, in a subject in need thereof, or treating a symptom of a disease, disorder, condition, or syndrome in a subject in need thereof, the method comprising administering a therapeutically effective amount of acetyl-DL-leucine, or a pharmaceutically acceptable salt thereof, is administered to the subject. In another embodiment, a therapeutically effective amount of acetyl-DL-leucine, or a pharmaceutically acceptable salt thereof, is administered to the subject to treat the disease, disorder, condition, or syndrome. In another embodiment, a therapeutically effective amount of acetyl-DL-leucine, or a pharmaceutically acceptable salt thereof, is administered to the subject to treat the symptom of the disease, disorder, condition, or syndrome.

In another embodiment, the present disclosure provides a method of treating a disease, disorder, condition, or syndrome, e.g., a disease, disorder, condition, or syndrome provided in any one of Tables 1-17, in a subject in need thereof, or treating a symptom of a disease, disorder, condition, or syndrome in a subject in need thereof, the method comprising administering a therapeutically effective amount of acetyl-D-leucine, or a pharmaceutically acceptable salt thereof, is administered to the subject. In another embodiment, a therapeutically effective amount of acetyl-D-leucine, or a pharmaceutically acceptable salt thereof, is administered to the subject to treat the disease, disorder, condition, or syndrome. In another embodiment, a therapeutically effective amount of acetyl-D-leucine, or a pharmaceutically acceptable salt thereof, is administered to the subject to treat the symptom of the disease, disorder, condition, or syndrome.

In another embodiment, the present disclosure provides a method of treating a disease, disorder, condition, or syndrome, e.g., a disease, disorder, condition, or syndrome provided in any one of Tables 1-17, in a subject in need thereof, or treating a symptom of a disease, disorder, condition, or syndrome in a subject in need thereof, the method comprising administering a therapeutically effective amount of acetyl-L-leucine, or a pharmaceutically acceptable salt thereof, is administered to the subject. In another embodiment, a therapeutically effective amount of acetyl-L-leucine, or a pharmaceutically acceptable salt thereof, is administered to the subject to treat the disease, disorder, condition, or syndrome. In another embodiment, a therapeutically effective amount of acetyl-L-leucine, or a pharmaceutically acceptable salt thereof, is administered to the subject to treat the symptom of the disease, disorder, condition, or syndrome.

In another embodiment, the present disclosure provides a method of modulating the expression of one or more pro-inflammatory mediators, e.g., pro-inflammatory cytokines and pro-inflammatory chemokines, in a subject in a subject in need thereof, the method comprising administering a therapeutically effective amount of a Compound of the Disclosure to the subject. In another embodiment, the pro-inflammatory mediators are any one or more of NOS2, IL-18, IFNb, IL-1β, TNFα, NOX2, NLRP3, SOCS3, ARG1, IL-10, IL-4ra, or YM1.

In another embodiment, the administration of a Compound of the Disclosure reduces or inhibits neuroinflammation in a subject in need thereof. In another embodiment, the reduced or inhibited neuroinflammation comprises a reduction or inhibition of excessive pro-inflammatory cytokines. In another embodiment, the neuroinflammation is inhibited or reduced compared to neuroinflammation in a subject afflicted with a disease, disorder, condition, or syndrome, e.g., a disease, disorder, condition, or syndrome provided in any one of Tables 1-17, e.g., TBI, not administered the Compound of the Disclosure.

In another embodiment, the administration of a Compound of the Disclosure modulates elevated levels of the microtubule-associated protein Tau and/or the microtubule-associated protein 1A/1B-light chain 3-phosphatidylethanolamine conjugate (LC3-II) in a subject in need of treatment for TBI. In one embodiment, the elevated levels of Tau protein and/or LC3-II are modulated compared to elevated levels of Tau protein and/or LC3-II in a subject afflicted with a disease, disorder, condition, or syndrome, e.g., a disease, disorder, condition, or syndrome provided in any one of Tables 1-17, e.g., TBI, not administered the Compound of the Disclosure.

In another embodiment, the administration of a Compound of the Disclosure is effective to improve recovery of the subject in need thereof. In another embodiment, the recovery is improved compared to the recovery of a subject afflicted with a disease, disorder, condition, or syndrome, e.g., a disease, disorder, condition, or syndrome provided in any one of Tables 1-17, e.g., TBI, not administered the Compound of the Disclosure.

In another embodiment, the recovery is evaluated using the Glasgow Outcome Scale (GOS). In one embodiment, the administration of leucine, acetyl-leucine, or any other Compound of the Disclosure, or a pharmaceutically acceptable salt thereof is effective to increase the percentage of subjects with a "favorable" outcome on the GOS by at least 10%, at least 20%, at least 30%, at least 50%, or at least 70% compared to a baseline, such as compared to that of subjects afflicted with TBI not administered the leucine, acetyl-leucine, or pharmaceutically acceptable salt thereof. In one embodiment, the subject is evaluated using the GOS at three months after the infliction of injury. In another embodiment, the subject is evaluated using the GOS at six months after the infliction of injury. In another embodiment, the subject is evaluated using the GOS at 12 months or more after the infliction of injury.

In another embodiment, the administration of a Compound of the Disclosure is effective to improve cognitive function in the subject in need thereof. In another embodiment, the cognitive function is improved compared to the cognitive function of a subject afflicted with a disease, disorder, condition, or syndrome, e.g., a disease, disorder, condition, or syndrome provided in any one of Tables 1-17, e.g., TBI, not administered the Compound of the Disclosure. In one embodiment, the cognitive function is memory. In one embodiment, the memory is long-term memory. In another embodiment, the cognitive function is learning. In another embodiment, the cognitive function is spatial navigation.

In another embodiment, the administration of a Compound of the Disclosure reduces or inhibits at least one symptom of a disease, disorder, condition, or syndrome, e.g., a disease, disorder, condition, or syndrome provided in any one of Tables 1-17, e.g., TBI, in a subject in need thereof. In another embodiment, at least one symptom is reduced or inhibited compared to the symptom(s) in a subject afflicted with a disease, disorder, condition, or syndrome, e.g., a disease, disorder, condition, or syndrome provided in any one of Tables 1-17, e.g., TBI, not administered the Compound of the Disclosure. In another embodiment, the condition is TBI, and at least one symptom of TBI is confusion, dizziness, disorientation, loss of coordination, memory loss, inability to form new memories, sleep disturbances, behavior or mood changes, increased agitation, depression, convulsions, and/or seizures.

In another embodiment, the administration of a Compound of the Disclosure reduces the severity of at least one symptom of a disease, disorder, condition, or syndrome, e.g., a disease, disorder, condition, or syndrome provided in any one of Tables 1-17, e.g., TBI, by at least 10%, at least 20%, at least 30%, at least 40%, at least 50%, at least 70%, at least 90%, or 100% compared to a baseline, such as compared to a subject afflicted with a disease, disorder, condition, or syndrome, e.g., a disease, disorder, condition, or syndrome provided in any one of Tables 1-17, e.g., TBI, not administered the leucine, acetyl-leucine, or pharmaceutically acceptable salt thereof.

In another embodiment, the administration of a Compound of the Disclosure is effective to improve the physical and/or mental activity level of the subject in thereof. In another embodiment, the recovery is improved compared to the recovery of a subject afflicted with a disease, disorder, condition, or syndrome, e.g., a disease, disorder, condition, or syndrome provided in any one of Tables 1-17, e.g., TBI, not administered the Compound of the Disclosure.

In another embodiment, the administration of a Compound of the Disclosure is effective to treat encephalitis. In another embodiment, the encephalitis is infectious encephalitis. In another embodiment, the encephalitis is autoimmune encephalitis. Symptoms of encephalitis include, but are not limited to, headache, mild flu-like symptoms (aches, fatigue, slight fever), sensitivity to light, neck stiffness, sleepiness or lethargy, increased irritability, seizures, changes in alertness, confusion, or hallucinations, loss of energy, loss of appetite, unsteady gait, nausea and vomiting, personality changes, and trouble talking and/or speech changes. In some embodiments, the symptoms of encephalitis are abnormal movements (chorea, incoordination), atypical motor symptoms, e.g., ataxia or hemiparesis, or seizures.

In some embodiments, the encephalitis is caused by an upper respiratory infection, an illness that causes diarrhea, nausea, and vomiting, measles, mumps, rubella, chickenpox, herpes simplex virus, West Nile virus, rabies, Lyme disease, tuberculosis, syphilis, an infection caused by parasites, e.g., toxoplasmosis, or an autoimmune reaction in a subject. Thus, in some embodiments, the present disclosure provides a method of preventing encephalitis in a subject in need thereof, the method comprising administering a therapeutically effective amount of a Compound of the disclosure, to the subject, wherein the subject has an upper respiratory infection, an illness that causes diarrhea, nausea, and vomiting, measles, mumps, rubella, chickenpox, herpes simplex virus, West Nile virus, rabies, Lyme disease, tuberculosis, syphilis, an infection caused by parasites, e.g., toxoplasmosis, or an autoimmune reaction.

In another embodiment, the administration of a Compound of the Disclosure is effective to treat sleep disorder associated with a traumatic brain injury, e.g., difficulty with falling asleep and/or staying asleep, fatigue, drowsiness, sleep apnea, narcolepsy, and/or sleepiness, e.g., excessive daytime sleepiness.

In another embodiment, the administration of a Compound of the Disclosure is effective to treat a metabolic abnormality in the CNS. In another embodiment, the administration of a Compound of the Disclosure is effective to treat symptoms and/or diseases associated with a metabolic abnormality in the CNS. In another embodiment, the metabolic abnormality is an imbalance of glucose metabolism. In another embodiment, the metabolic abnormality is an imbalance of glucose metabolism in the cerebellum. In another embodiment, the administration of a Compound of the Disclosure increases the metabolism of glucose, e.g., in the cerebellum, in the CNS.

The disclosure also provides the following particular embodiments.

Embodiment I: Leucine, acetyl-leucine, or a pharmaceutically acceptable salt thereof for use in a method of treating traumatic brain injury (TBI) in a subject in need thereof.

Embodiment II: Leucine, acetyl-leucine, or a pharmaceutically acceptable salt thereof for use in a method according to Embodiment I, wherein the method comprises administering to the subject a therapeutically effective amount of the leucine, acetyl-leucine, or pharmaceutically acceptable salt thereof.

Embodiment III: Leucine, acetyl-leucine, or a pharmaceutically acceptable salt thereof, for use in a method according to any of Embodiments I and II, wherein the leucine is DL-leucine or the acetyl-leucine is acetyl-DL-leucine.

Embodiment IV: Leucine, acetyl-leucine, or a pharmaceutically acceptable salt thereof, for use in a method according to any of Embodiments I and II, wherein the leucine or acetyl-leucine has an enantiomeric excess of the L-enantiomer or the D-enantiomer.

Embodiment V: Leucine, acetyl-leucine, or a pharmaceutically acceptable salt thereof, for use in a method according to any of Embodiments I-IV, wherein the method comprises administering the acetyl-leucine to the subject in need thereof at a therapeutically effective amount of from about 1 g to about 15 g per day, from about 1 g to about 10 g per day, from about 1.5 g to about 7 g per day, from about 4 g to about 6 g per day, or from about 4 g to about 5 g per day.

Embodiment VI: A method of inhibiting or reducing one or more symptoms of traumatic brain injury (TBI) in a subject in need thereof, comprising administering a therapeutically effective amount of leucine, acetyl-leucine, or a pharmaceutically acceptable salt thereof to the subject.

Embodiment VII: The method according to Embodiment VI, wherein the leucine is DL-leucine or the acetyl-leucine is acetyl-DL-leucine.

Embodiment VIII: The method according to Embodiment VI, wherein the leucine or acetyl-leucine has an enantiomeric excess of the L-enantiomer or the D-enantiomer.

Embodiment IX: The method according to any of Embodiments VI-VIII, wherein therapeutically effective amount is from about 1 g to about 15 g per day, from about 1 g to about 10 g per day, from about 1.5 g to about 7 g per day, from about 4 g to about 6 g per day, or from about 4 g to about 5 g per day.

The disclosure also provides the following particular embodiments.

Embodiment 1. A Compound of the Disclosure for use in treating a disease, disorder, condition, or syndrome in a subject in need thereof, or for use in treating a symptom of a disease, disorder, condition, or syndrome in a subject in need thereof, wherein the diseases, disorders, conditions, or syndromes are any one or more of the diseases, disorders, conditions, or syndrome provided in Table 1.

Embodiment 2. The Compound of the Disclosure for use of Embodiment 1, wherein the diseases, disorders, conditions, or syndromes are any one or more of the diseases, disorders, conditions, or syndrome is provided in Table 2.

Embodiment 3. The Compound of the Disclosure for use of Embodiment 1, wherein the diseases, disorders, conditions, or syndromes are any one or more of the diseases, disorders, conditions, or syndrome provided in Table 3.

Embodiment 4. The Compound of the Disclosure for use of Embodiment 1, wherein the diseases, disorders, conditions, or syndromse are any one or more of the diseases, disorders, conditions, or syndrome is provided in Table 4.

Embodiment 5. The Compound of the Disclosure for use of Embodiment 1, wherein the diseases, disorders, conditions, or syndromes are any one or more of the diseases, disorders, conditions, or syndrome provided in Table 5.

Embodiment 6. The Compound of the Disclosure for use of Embodiment 1, wherein the disease, disorder, condition, or syndrome is traumatic brain injury.

Embodiment 7. The Compound of the Disclosure for use of Embodiment 1, wherein the diseases, disorders, conditions, or syndromes are any one or more of the diseases, disorders, conditions, or syndrome provided in Table 6.

Embodiment 8. The Compound of the Disclosure for use of Embodiment 1, wherein the diseases, disorders, conditions, or syndromes are any one or more of the diseases, disorders, conditions, or syndrome provided in Table 7.

Embodiment 9. The Compound of the Disclosure for use of Embodiment 1, wherein the diseases, disorders, conditions, or syndromes are any one or more of the diseases, disorders, conditions, or syndrome provided in Table 8.

Embodiment 10. The Compound of the Disclosure for use of Embodiment 1, wherein the diseases, disorders, conditions, or syndromes are any one or more of the diseases, disorders, conditions, or syndrome provided in Table 9

Embodiment 11. The Compound of the Disclosure for use of Embodiment 1, wherein the diseases, disorders, conditions, or syndromes are any one or more of the diseases, disorders, conditions, or syndrome provided in Table 10.

Embodiment 12. The Compound of the Disclosure for use of Embodiment 1, wherein the diseases, disorders, conditions, or syndromes are any one or more of the diseases, disorders, conditions, or syndrome provided in Table 11

Embodiment 13. The Compound of the Disclosure for use of Embodiment 1, wherein the diseases, disorders, conditions, or syndromes are any one or more of the diseases, disorders, conditions, or syndrome provided in Table 12.

Embodiment 14. The Compound of the Disclosure for use of Embodiment 1, wherein the diseases, disorders, conditions, or syndromes are any one or more of the diseases, disorders, conditions, or syndrome provided in Table 13.

Embodiment 15. The Compound of the Disclosure for use of Embodiment 1, wherein the diseases, disorders, conditions, or syndromes are any one or more of the diseases, disorders, conditions, or syndrome provided in Table 14.

Embodiment 16. The Compound of the Disclosure for use of Embodiment 1, wherein the diseases, disorders, conditions, or syndromes are any one or more of the diseases, disorders, conditions, or syndrome provided in Table 15.

Embodiment 17. The Compound of the Disclosure for use of Embodiment 1, wherein the diseases, disorders, conditions, or syndromes are any one or more of the diseases, disorders, conditions, or syndrome provided in Table 16.

Embodiment 18. The Compound of the Disclosure for use of any one of Embodiments 1-17, wherein about 1 g to about 30 g of Compound of the Disclosure is administered to the subject per day.

Embodiment 19. The Compound of the Disclosure for use of Embodiment 18, wherein about 2 g to about 15 g of Compound of the Disclosure is administered to the subject per day.

Embodiment 20. The Compound of the Disclosure for use of Embodiment 19, wherein about 3 g to about 10 g of Compound of the Disclosure is administered to the subject per day.

Embodiment 21. The Compound of the Disclosure for use of Embodiment 20, wherein about 4 g to about 8 g of Compound of the Disclosure is administered to the subject per day.

Embodiment 22. The Compound of the Disclosure for use of Embodiment 21, wherein about 4 g to about 5 g of Compound of the Disclosure is administered to the subject per day.

Embodiment 23. The Compound of the Disclosure for use of Embodiment 22, wherein about 5 g of Compound of the Disclosure is administered to the subject per day.

Embodiment 24. The Compound of the Disclosure for use of any one of Embodiments 1-23, wherein a therapeutically effective amount of leucine, or a pharmaceutically acceptable salt thereof, is administered to the subject.

Embodiment 25. The Compound of the Disclosure for use of Embodiment 24, wherein a therapeutically effective amount of leucine, or a pharmaceutically acceptable salt thereof, is administered to the subject to treat the disease, disorder, condition, or syndrome.

Embodiment 26. The Compound of the Disclosure for use of Embodiment 24, wherein a therapeutically effective amount of leucine, or a pharmaceutically acceptable salt thereof, is administered to the subject to treat the symptom of the disease, disorder, condition, or syndrome.

Embodiment 27. The Compound of the Disclosure for use of any one of Embodiments 1-23, wherein a therapeutically effective amount of acetyl-DL-leucine, or a pharmaceutically acceptable salt thereof, is administered to the subject.

Embodiment 28. The Compound of the Disclosure for use of Embodiment 27, wherein a therapeutically effective amount of acetyl-DL-leucine, or a pharmaceutically acceptable salt thereof, is administered to the subject to treat the disease, disorder, condition, or syndrome.

Embodiment 29. The Compound of the Disclosure for use of Embodiment 27, wherein a therapeutically effective amount of acetyl-DL-leucine, or a pharmaceutically acceptable salt thereof, is administered to the subject to treat the symptom of the disease, disorder, condition, or syndrome.

Embodiment 30. The Compound of the Disclosure for use of any one of Embodiments 1-23, wherein a therapeutically effective amount of acetyl-L-leucine, or a pharmaceutically acceptable salt thereof, is administered to the subject.

Embodiment 31. The Compound of the Disclosure for use of Embodiment 30, wherein a therapeutically effective amount of acetyl-L-leucine, or a pharmaceutically acceptable salt thereof, is administered to the subject to treat the disease, disorder, condition, or syndrome.

Embodiment 32. The Compound of the Disclosure for use of Embodiment 30, wherein a therapeutically effective amount of acetyl-L-leucine, or a pharmaceutically acceptable salt thereof, is administered to the subject to treat the symptom of the disease, disorder, condition, or syndrome.

Embodiment 32A. The Compound of the Disclosure for use of any one of Embodiments 1-23, wherein a therapeutically effective amount of acetyl-D-leucine, or a pharmaceutically acceptable salt thereof, is administered to the subject.

Embodiment 32B. The Compound of the Disclosure for use of Embodiment 32A, wherein a therapeutically effective amount of acetyl-D-leucine, or a pharmaceutically acceptable salt thereof, is administered to the subject to treat the disease, disorder, condition, or syndrome.

Embodiment 32C. The Compound of the Disclosure for use of Embodiment 32A, wherein a therapeutically effective amount of acetyl-D-leucine, or a pharmaceutically acceptable salt thereof, is administered to the subject to treat the symptom of the disease, disorder, condition, or syndrome.

Embodiment 33. Use of a Compound of the Disclosure for the manufacture of a medicament for treating a disease, disorder, condition, or syndrome in a subject in need thereof, or for the manufacture of a medicament for treating a symptom of a disease, disorder, condition, or syndrome in a subject in need thereof, wherein the diseases, disorders, conditions, or syndromes are any one or more of the diseases, disorders, conditions, or syndrome provided in Table 1.

Embodiment 34. The use of Embodiment 33, wherein the diseases, disorders, conditions, or syndromes are any one or more of the diseases, disorders, conditions, or syndrome provided in Table 2.

Embodiment 35. The use of Embodiment 33, wherein the diseases, disorders, conditions, or syndromes are any one or more of the diseases, disorders, conditions, or syndrome provided in Table 3.

Embodiment 36. The use of Embodiment 33, wherein the diseases, disorders, conditions, or syndromes are any one or more of the diseases, disorders, conditions, or syndrome is any one or more of the diseases, disorders, conditions, or syndrome provided in Table 4.

Embodiment 37. The use of Embodiment 33, wherein the diseases, disorders, conditions, or syndromes are any one or more of the diseases, disorders, conditions, or syndrome provided in Table 5.

Embodiment 38. The use of Embodiment 37, wherein the disease, disorder, condition, or syndrome is traumatic brain injury.

Embodiment 39. The use of Embodiment 33, wherein the diseases, disorders, conditions, or syndromes are any one or more of the diseases, disorders, conditions, or syndrome provided in Table 6.

Embodiment 40. The use of Embodiment 33, wherein the diseases, disorders, conditions, or syndromes are any one or more of the diseases, disorders, conditions, or syndrome provided in Table 7.

Embodiment 41. The use of Embodiment 33, wherein the diseases, disorders, conditions, or syndromes are any one or more of the diseases, disorders, conditions, or syndrome provided in Table 8.

Embodiment 42. The use of Embodiment 33, wherein the diseases, disorders, conditions, or syndromes are any one or more of the diseases, disorders, conditions, or syndrome provided in Table 9.

Embodiment 43. The use of Embodiment 33, wherein the diseases, disorders, conditions, or syndromes are any one or more of the diseases, disorders, conditions, or syndrome provided in Table 10.

Embodiment 44. The use of Embodiment 33, wherein the diseases, disorders, conditions, or syndromes are any one or more of the diseases, disorders, conditions, or syndrome provided in Table 11.

Embodiment 45. The use of Embodiment 33, wherein the diseases, disorders, conditions, or syndromes are any one or more of the diseases, disorders, conditions, or syndrome provided in Table 12.

Embodiment 46. The use of Embodiment 33, wherein the diseases, disorders, conditions, or syndromes are any one or more of the diseases, disorders, conditions, or syndrome provided in Table 13.

Embodiment 47. The use of Embodiment 33, wherein the diseases, disorders, conditions, or syndromes are any one or more of the diseases, disorders, conditions, or syndrome provided in Table 14.

Embodiment 48. The use of Embodiment 33, wherein the diseases, disorders, conditions, or syndromes are any one or more of the diseases, disorders, conditions, or syndrome provided in Table 15.

Embodiment 49. The use of Embodiment 33, wherein the diseases, disorders, conditions, or syndromes are any one or more of the diseases, disorders, conditions, or syndrome provided in Table 16.

Embodiment 50. The use of any one of Embodiments 33-49, wherein about 1 g to about 30 g of a Compound of the Disclosure is administered to the subject per day.

Embodiment 51. The use of Embodiment 50, wherein about 2 g to about 15 g of a Compound of the Disclosure is administered to the subject per day.

Embodiment 52. The use of Embodiment 51, wherein about 3 g to about 10 g of a Compound of the Disclosure is administered to the subject per day.

Embodiment 53. The use of Embodiment 52, wherein about 4 g to about 8 g of a Compound of the Disclosure is administered to the subject per day.

Embodiment 54. The use of Embodiment 53, wherein about 4 g to about 5 g of a Compound of the Disclosure is administered to the subject per day.

Embodiment 55. The use of Embodiment 54, wherein about 5 g of a Compound of the Disclosure is administered to the subject per day.

Embodiment 56. The use of any one of Embodiments 33-55, wherein a therapeutically effective amount of leucine, or a pharmaceutically acceptable salt thereof, is administered to the subject.

Embodiment 57. The use of Embodiment 56, wherein a therapeutically effective amount of leucine, or a pharmaceutically acceptable salt thereof, is administered to the subject to treat the disease, disorder, condition, or syndrome.

Embodiment 58. The use of Embodiment 56, wherein a therapeutically effective amount of leucine, or a pharmaceutically acceptable salt thereof, is administered to the subject to treat the symptom of the disease, disorder, condition, or syndrome.

Embodiment 59. The use of any one of Embodiments 33-55, wherein a therapeutically effective amount of acetyl-DL-leucine, or a pharmaceutically acceptable salt thereof, is administered to the subject.

Embodiment 60. The use of Embodiment 59, wherein a therapeutically effective amount of acetyl-DL-leucine, or a pharmaceutically acceptable salt thereof, is administered to the subject to treat the disease, disorder, condition, or syndrome.

Embodiment 61. The use of Embodiment 59, wherein a therapeutically effective amount of acetyl-DL-leucine, or a pharmaceutically acceptable salt thereof, is administered to the subject to treat the symptom of the disease, disorder, condition, or syndrome.

Embodiment 62. The use of any one of Embodiments 33-55, wherein a therapeutically effective amount of acetyl-L-leucine, or a pharmaceutically acceptable salt thereof, is administered to the subject.

Embodiment 63. The use of Embodiment 62, wherein a therapeutically effective amount of acetyl-L-leucine, or a pharmaceutically acceptable salt thereof, is administered to the subject to treat the disease, disorder, condition, or syndrome.

Embodiment 64. The use of Embodiment 62, wherein a therapeutically effective amount of acetyl-L-leucine, or a pharmaceutically acceptable salt thereof, is administered to the subject to treat the symptom of the disease, disorder, condition, or syndrome.

Embodiment 64A. The use of any one of Embodiments 33-55, wherein a therapeutically effective amount of acetyl-D-leucine, or a pharmaceutically acceptable salt thereof, is administered to the subject.

Embodiment 64B. The use of Embodiment 64A, wherein a therapeutically effective amount of acetyl-D-leucine, or a pharmaceutically acceptable salt thereof, is administered to the subject to treat the disease, disorder, condition, or syndrome.

Embodiment 64C. The use of Embodiment 64A, wherein a therapeutically effective amount of acetyl-D-leucine, or a pharmaceutically acceptable salt thereof, is administered to the subject to treat the symptom of the disease, disorder, condition, or syndrome.

Embodiment 65. A pharmaceutical composition comprising a Compound of the Disclosure and a pharmaceutically acceptable carrier for use in treating a disease, disorder, condition, or syndrome in a subject in need thereof, or for use in treating a symptom of a disease, disorder, condition, or syndrome in a subject in need thereof, wherein the diseases, disorders, conditions, or syndromes are any one or more of the diseases, disorders, conditions, or syndrome provided in Table 1.

Embodiment 66. The pharmaceutical composition for use of Embodiment 65, wherein the diseases, disorders, conditions, or syndromes are any one or more of the diseases, disorders, conditions, or syndrome provided in Table 2.

Embodiment 67. The pharmaceutical composition for use of Embodiment 65, wherein the diseases, disorders, conditions, or syndromes are any one or more of the diseases, disorders, conditions, or syndrome provided in Table 3.

Embodiment 68. The pharmaceutical composition for use of Embodiment 65, wherein the diseases, disorders, conditions, or syndromes are any one or more of the diseases, disorders, conditions, or syndrome provided in Table 4.

Embodiment 69. The pharmaceutical composition for use of Embodiment 65, wherein the diseases, disorders, conditions, or syndromes are any one or more of the diseases, disorders, conditions, or syndrome provided in Table 5.

Embodiment 70. The pharmaceutical composition for use of Embodiment 65, wherein the disease, disorder, condition, or syndrome is traumatic brain injury.

Embodiment 71. The pharmaceutical composition for use of Embodiment 65, wherein the diseases, disorders, conditions, or syndromes are any one or more of the diseases, disorders, conditions, or syndrome provided in Table 6.

Embodiment 72. The pharmaceutical composition for use of Embodiment 65, wherein the diseases, disorders, conditions, or syndromes are any one or more of the diseases, disorders, conditions, or syndrome provided in Table 7.

Embodiment 73. The pharmaceutical composition for use of Embodiment 65, wherein the diseases, disorders, conditions, or syndromes are any one or more of the diseases, disorders, conditions, or syndrome provided in Table 8.

Embodiment 74. The pharmaceutical composition for use of Embodiment 65, wherein the diseases, disorders, conditions, or syndromes are any one or more of the diseases, disorders, conditions, or syndrome provided in Table 9.

Embodiment 75. The pharmaceutical composition for use of Embodiment 65, wherein the diseases, disorders, conditions, or syndromes are any one or more of the diseases, disorders, conditions, or syndrome provided in Table 10.

Embodiment 76. The pharmaceutical composition for use of Embodiment 65, wherein the diseases, disorders, conditions, or syndromes are any one or more of the diseases, disorders, conditions, or syndrome provided in Table 11.

Embodiment 77. The pharmaceutical composition for use of Embodiment 65, wherein the diseases, disorders, conditions, or syndromes are any one or more of the diseases, disorders, conditions, or syndrome provided in Table 12.

Embodiment 78. The pharmaceutical composition for use of Embodiment 65, wherein the diseases, disorders, conditions, or syndromes are any one or more of the diseases, disorders, conditions, or syndrome provided in Table 13.

Embodiment 79. The pharmaceutical composition for use of Embodiment 65, wherein the diseases, disorders, conditions, or syndromes are any one or more of the diseases, disorders, conditions, or syndrome provided in Table 14.

Embodiment 80. The pharmaceutical composition for use of Embodiment 65, wherein the diseases, disorders, conditions, or syndromes are any one or more of the diseases, disorders, conditions, or syndrome provided in Table 15.

Embodiment 81. The pharmaceutical composition for use of Embodiment 65, wherein the diseases, disorders, conditions, or syndromes are any one or more of the diseases, disorders, conditions, or syndrome provided in Table 16.

Embodiment 82. The pharmaceutical composition for use of any one of Embodiments 65-81, wherein about 1 g to about 30 g of a Compound of the Disclosure is administered to the subject per day.

Embodiment 83. The pharmaceutical composition for use of Embodiment 82, wherein about 2 g to about 15 g of a Compound of the Disclosure is administered to the subject per day.

Embodiment 84. The pharmaceutical composition for use of Embodiment 83, wherein about 3 g to about 10 g of a Compound of the Disclosure is administered to the subject per day.

Embodiment 85. The pharmaceutical composition for use of Embodiment 84, wherein about 4 g to about 8 g of a Compound of the Disclosure is administered to the subject per day.

Embodiment 86. The pharmaceutical composition for use of Embodiment 85, wherein about 4 g to about 5 g of a Compound of the Disclosure is administered to the subject per day.

Embodiment 87. The pharmaceutical composition for use of Embodiment 86, wherein about 5 g of a Compound of the Disclosure is administered to the subject per day.

Embodiment 88. The pharmaceutical composition for use of any one of Embodiments 65-87, wherein a therapeutically effective amount of leucine, or a pharmaceutically acceptable salt thereof, is administered to the subject.

Embodiment 89. The pharmaceutical composition for use of Embodiment 88, wherein a therapeutically effective amount of leucine, or a pharmaceutically acceptable salt thereof, is administered to the subject to treat the disease, disorder, condition, or syndrome.

Embodiment 90. The pharmaceutical composition for use of Embodiment 88, wherein a therapeutically effective amount of leucine, or a pharmaceutically acceptable salt thereof, is administered to the subject to treat the symptom of the disease, disorder, condition, or syndrome.

Embodiment 91. The pharmaceutical composition for use of any one of Embodiments 65-87, wherein a therapeutically effective amount of acetyl-DL-leucine, or a pharmaceutically acceptable salt thereof, is administered to the subject.

Embodiment 92. The pharmaceutical composition for use of Embodiment 91, wherein a therapeutically effective amount of acetyl-DL-leucine, or a pharmaceutically acceptable salt thereof, is administered to the subject to treat the disease, disorder, condition, or syndrome.

Embodiment 93. The pharmaceutical composition for use of Embodiment 91, wherein a therapeutically effective amount of acetyl-DL-leucine, or a pharmaceutically acceptable salt thereof, is administered to the subject to treat the symptom of the disease, disorder, condition, or syndrome.

Embodiment 94. The pharmaceutical composition for use of any one of Embodiments 65-87, wherein a therapeutically effective amount of acetyl-L-leucine, or a pharmaceutically acceptable salt thereof, is administered to the subject.

Embodiment 95. The pharmaceutical composition for use of Embodiment 94, wherein a therapeutically effective amount of acetyl-L-leucine, or a pharmaceutically acceptable salt thereof, is administered to the subject to treat the disease, disorder, condition, or syndrome.

Embodiment 96. The pharmaceutical composition for use of Embodiment 94, wherein a therapeutically effective amount of acetyl-L-leucine, or a pharmaceutically acceptable salt thereof, is administered to the subject to treat the symptom of the disease, disorder, condition, or syndrome.

Embodiment 96A. The pharmaceutical composition for use of any one of Embodiments 65-87, wherein a therapeutically effective amount of acetyl-D-leucine, or a pharmaceutically acceptable salt thereof, is administered to the subject.

Embodiment 96B. The pharmaceutical composition for use of Embodiment 96A, wherein a therapeutically effective amount of acetyl-D-leucine, or a pharmaceutically acceptable salt thereof, is administered to the subject to treat the disease, disorder, condition, or syndrome.

Embodiment 96C. The pharmaceutical composition for use of Embodiment 96A, wherein a therapeutically effective amount of acetyl-D-leucine, or a pharmaceutically acceptable salt thereof, is administered to the subject to treat the symptom of the disease, disorder, condition, or syndrome.

### EXAMPLES

### EXAMPLE 1

A male patient suffering from traumatic brain injury was administered acetyl-DL-leucine at a dose of 3 g per day for the first week, followed by a dose of 5 g per day. The patient's therapists and nurses reported that the patient displayed more active behaviour after treatment with the acetyl-DL-leucine. The medication was later stopped and the patient's level of activity returned to its original level before treatment with acetyl-DL-leucine.

### EXAMPLE 2

### Efficacy of N-acetyl-L-leucine in the CCI mouse model of TBI

### Controlled Cortical Impact Model

Controlled cortical impact (CCI) induced TBI was performed in male *C57BL6*/*J* mice (20-25g). Briefly, after a 10-mm midline incision was made over the skull, the skin and fascia were retracted, and a 4-mm craniotomy was made on the central aspect of the left parietal bone of mice under surgical anesthesia (2-3% isoflurane evaporated in a gas mixture containing 70% N₂O and 30% O₂). Moderate injury was induced in the exposed brain by a custom microprocessor-controlled and compressed air driven pneumatic impactor of 3.5 mm diameter tip with the impact velocity of 6 m/s and a deformation depth of 2 mm.

### NAL Treatment

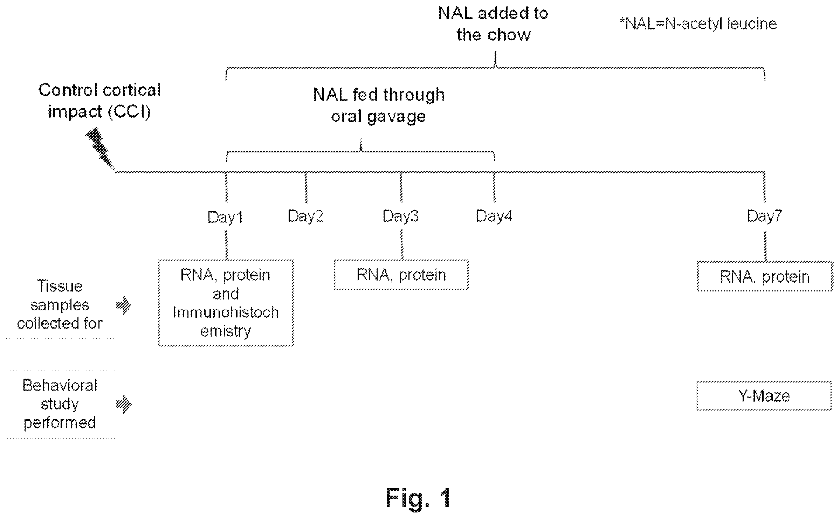

N-Acetyl-L-Leucine ("NAL" or "NALL") was dissolved in ethanol to prepare a 50 mg/ml solution which was then diluted in water to give a 10 mg/ml solution. About 0.25 ml of the NAL solution (10 mg/ml) was orally administered in mice at a 10 mg/kg dose (2.5 mg NAL/25 g mouse) via oral gavage each day after CCI induced TBI for 4 days. Mice were also fed with NAL at 0.5 mg/kg chow powder chow mixed with diet gel for up to 7 days after injury. *See* Fig. 1. No significant difference in food intake was observed between the sham and TBI mice fed with NALL (data not shown). While a slight decrease in body weight was detected in vehicle treated TBI mice, no appreciable change in body weight was observed in NALL treated TBI mice (data not shown).

### Cell Death After TBI

The level of α-fodrin cleavage products in NALL treated, vehicle treated sham, and TBI mouse cortices were determined by Western blot analysis and using the TUNEL assay to assess whether NALL treatment decreases cortical cell death after TBI.

### Western Blot Analysis

Around 5 mm tissue of ipsilateral cortex around the site of injury from TBI mice or the corresponding tissue of same volume around the same cortical region from sham mice were dissected and processed. Tissue lysates were resolved on 4-20% SDS-PAGE gels (Bio-Rad, 5671095) and transferred to PVDF membrane (Millipore, IPVH00010) using semi-dry transfer (Bio-Rad), blocked with 5% nonfat milk in tris buffered saline with 0.05% tween 20 (TBST), probed with primary antibodies in 1% BSA in TBST overnight at 4 °C and incubated with HRP-conjugated secondary antibodies (KPL, 474-1506, 474-1806, 14- 16-06 and 14-13-06) in blocking solution at room temperature for 1h. Protein bands were detected using chemiluminiscence kit (Pierce, 34076) and visualized using Chemi-doc system (Bio-Rad). Band intensity was analyzed using Image Lab software (Bio-Rad) and normalized to loading control (β-Actin). Primary antibodies: LC3 (1: 1000; Novus, NB100-2220), p62/SQSTM1 (1: 1000; BD Bioscience, 610832), β-actin/ACTB (1: 10,000; A1978) and fodrin/spectrin (1:5000; Enzo Life Science International, BMI,-FG6090).

### TUNEL Assay

Frozen sections (20 µm) were cut from vehicle or NAL fed sham and TBI mouse brains fixed with 4% paraformaldehyde (PFA, pH 7.4) and protected in 30% sucrose. TUNEL assay was performed on the frozen brain sections using ApopTag In Situ Apoptosis Detection Kit (Millipore, S7165) as per the manufacturer's protocol. Images following TUNEL assay were acquired using a fluorescent Nikon Ti-E inverted microscope, at 20X (CFI Plan APO VC 20X NA 0.75 WD 1 mm).

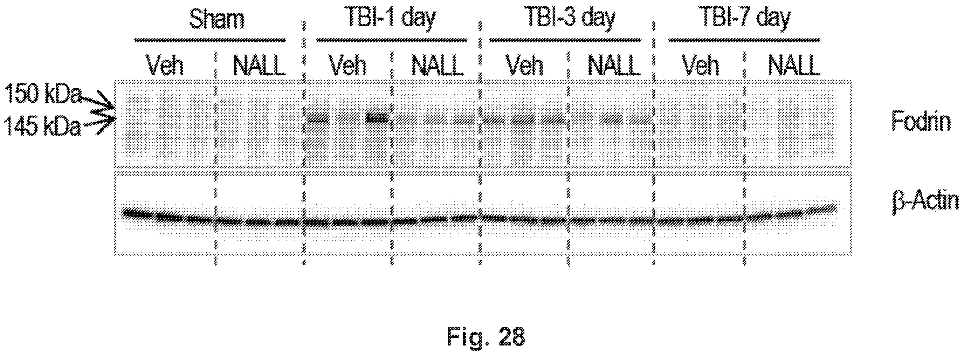

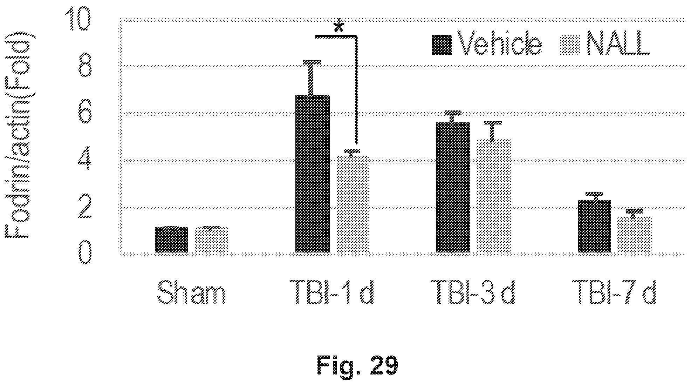

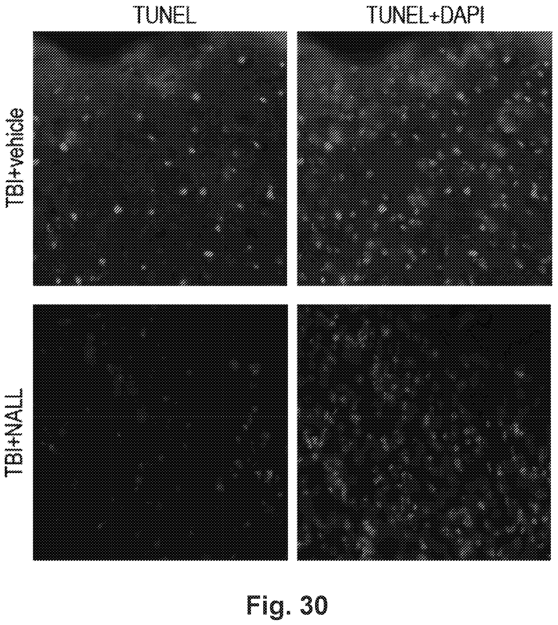

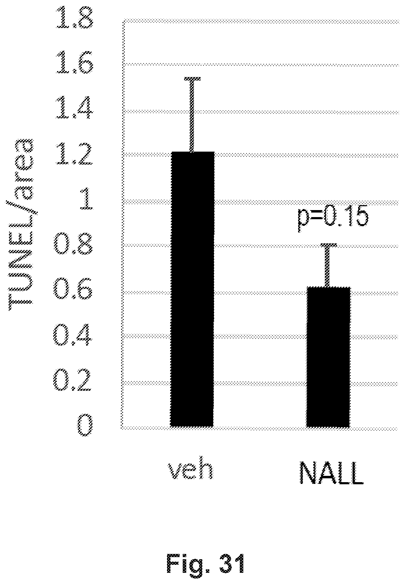

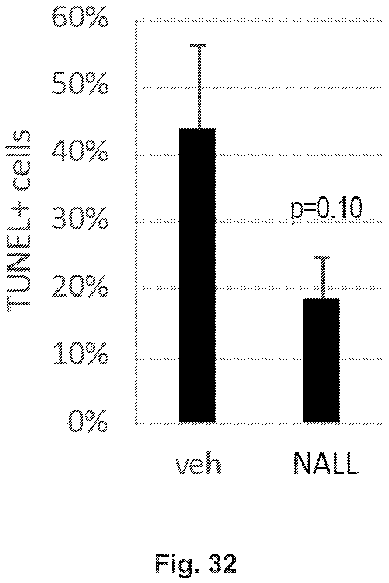

TBI induces both calpain and caspase mediated cleavage of α-fodrin generating 145-150 kDa and 150-120 kDa fragments, respectively. A lower level of 145-150 kDa fragments of α-fodrin in the NALL fed mouse cortices as compared to the cortices of mice fed with vehicle after TBI was observed, indicating the protective benefit of NALL in preventing cortical cell death. *See* Figs. 28 and 29. Lower levels of TUNEL positive cells in the brain sections of injured mice fed with NALL as compared to vehicle fed TBI mice were also detected, indicating the protective role of NALL in attenuating cell death after TBI. *See* Figs. 30, 31, and 32.

### Autophagy Flux After TBI

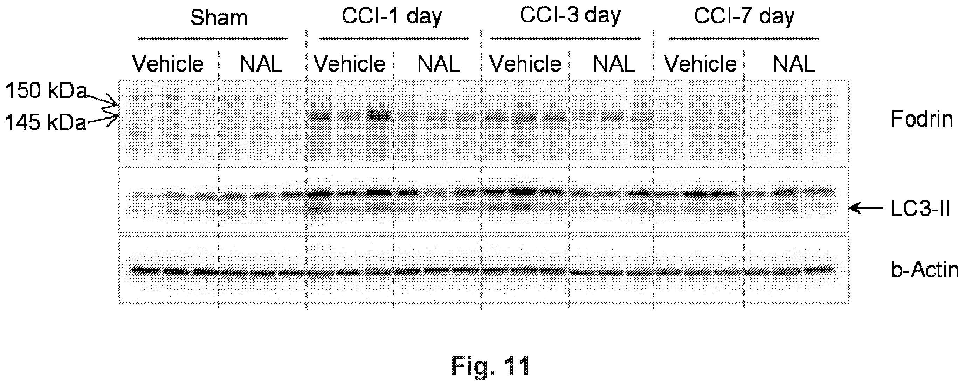

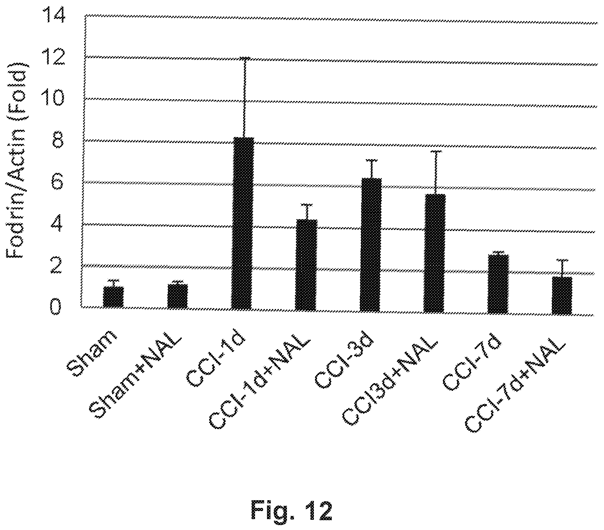

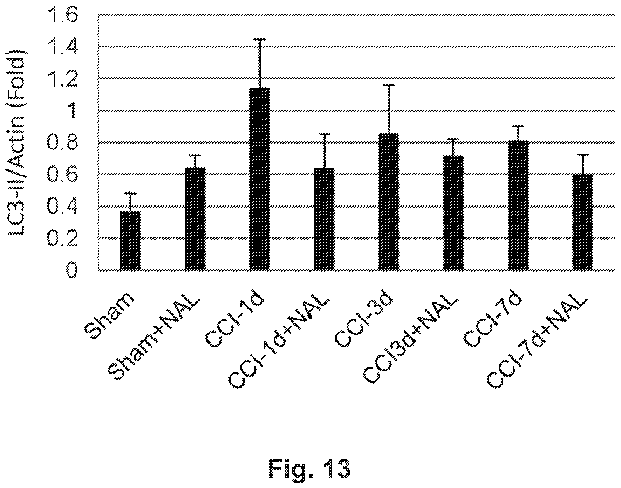

Autophagy is a lysosome dependent cellular degradation process that is disrupted due to lysosomal damage after TBI. *See, e.g.,* Sarkar et al., Autophagy 10:2208-2222 (2014); Sarkar et al., Autophagy, 1-20 (2019). Whether attenuation of cell death following NALL treatment in TBI mice is mediated through the restoration of autophagy flux was assessed. The results are presented in Figs. 11-13 and 33-35.

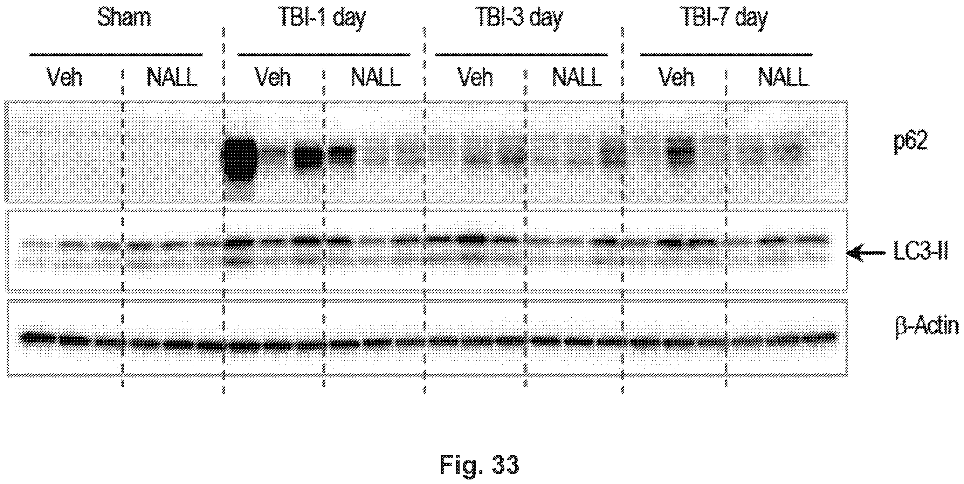

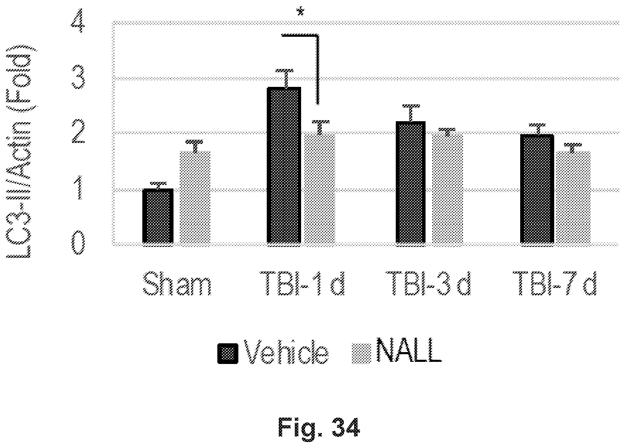

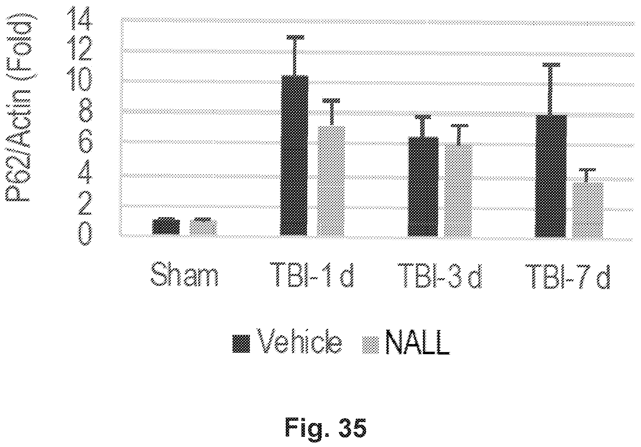

The levels of LC3-II and p62/SQSTM1 in the cortical tissue lysates prepared from vehicle and NAL fed naive and TBI mice were determined by western blot analysis. A decrease in the levels of LC3-II in the brain of NALL fed mice as compared to vehicle treated mice at day 1 after TBI was detected. *See* Figs. 33 and 34. A decrease in p62/SQSTM1 level in the cortex of mice fed with NALL as compared to vehicle treated mice after TBI was also observed. *See* Figs. 33 and 35. These results demonstate that NALL improves autophagy flux and thereby restores its neuroprotective function in the mouse cortices after TBI.

### Inflammatory Cytokines In TBI Mouse Brain

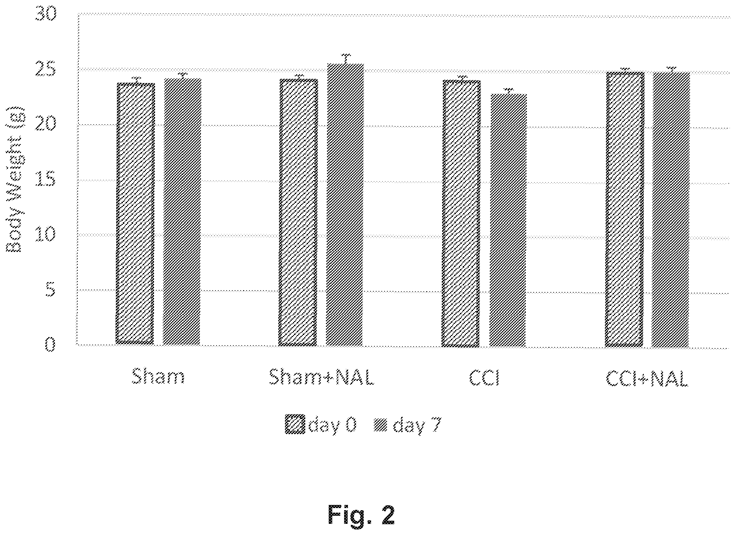

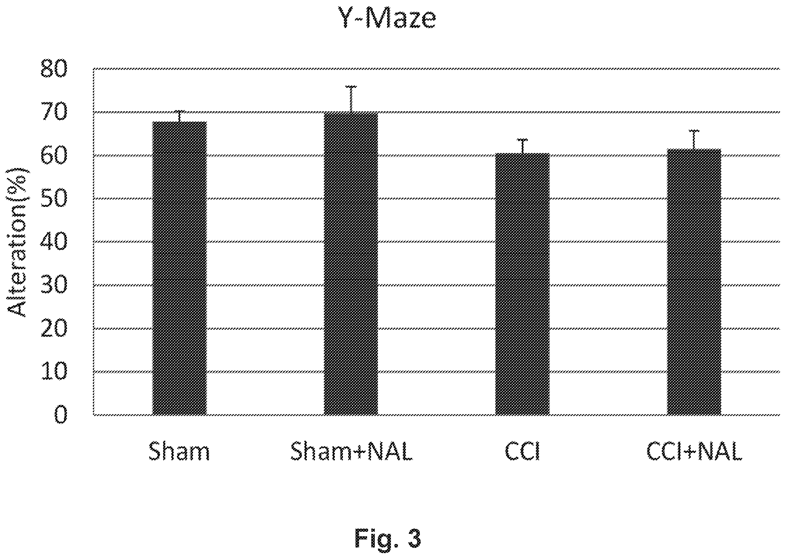

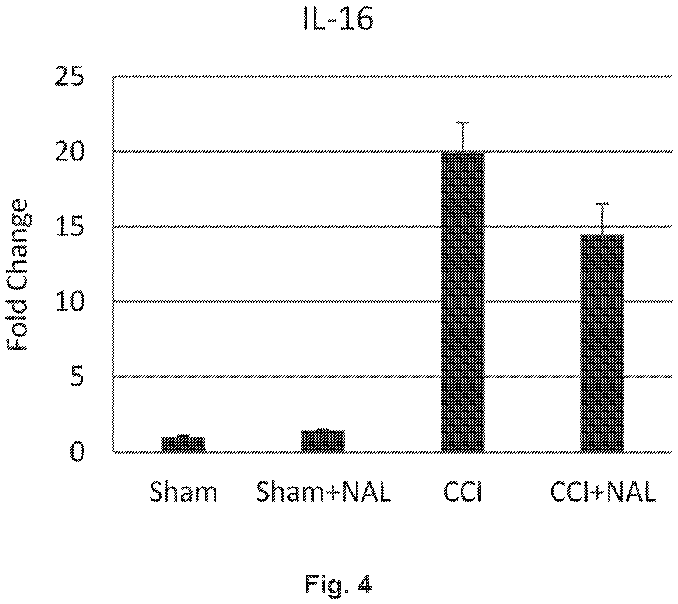

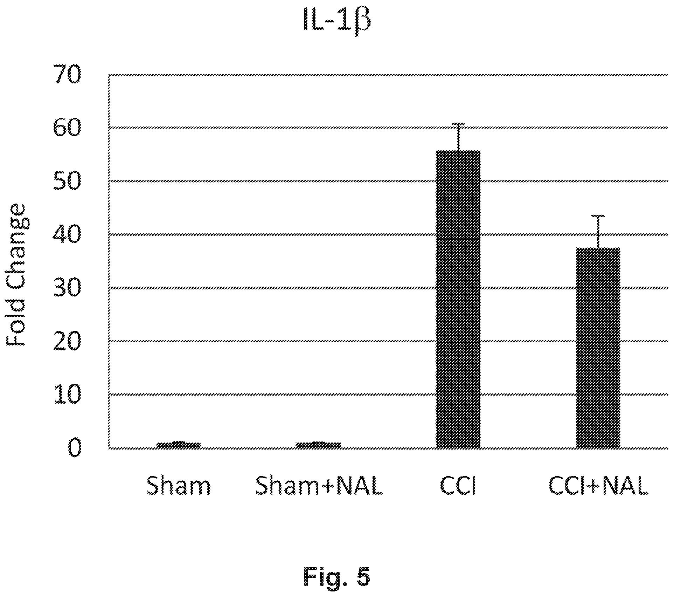

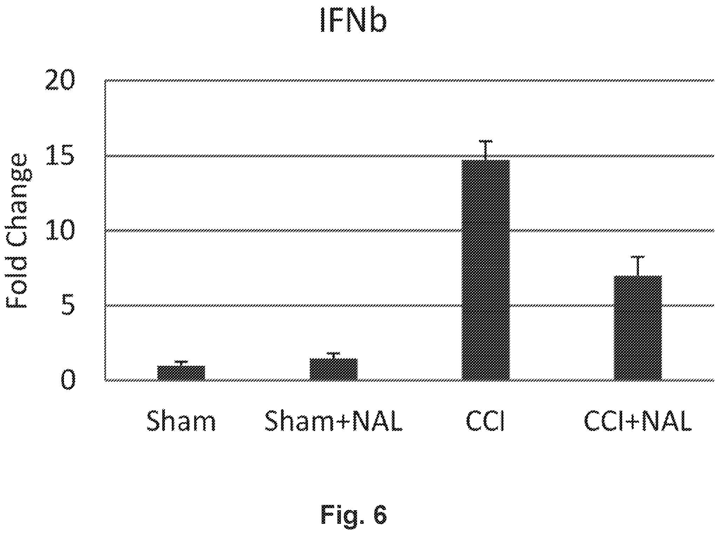

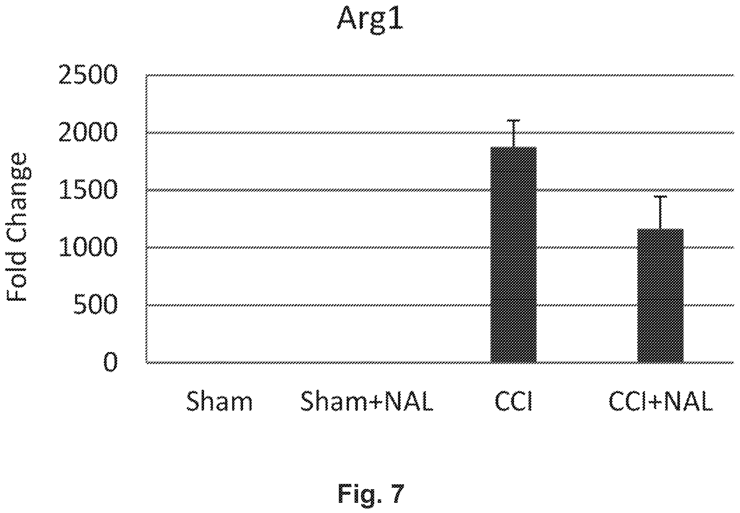

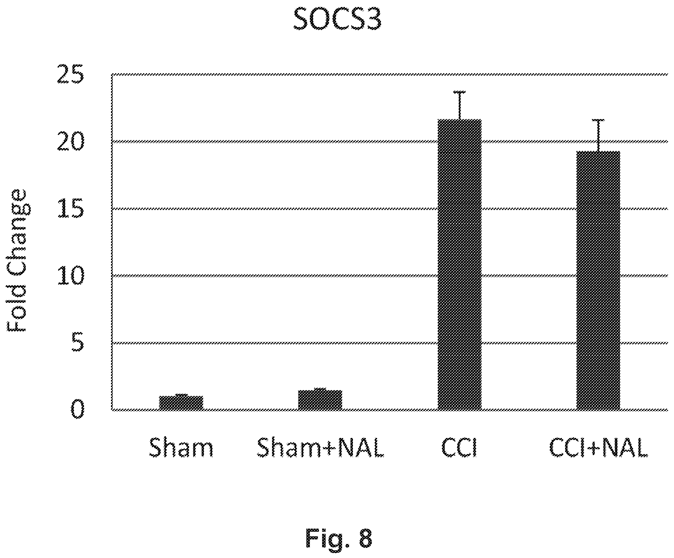

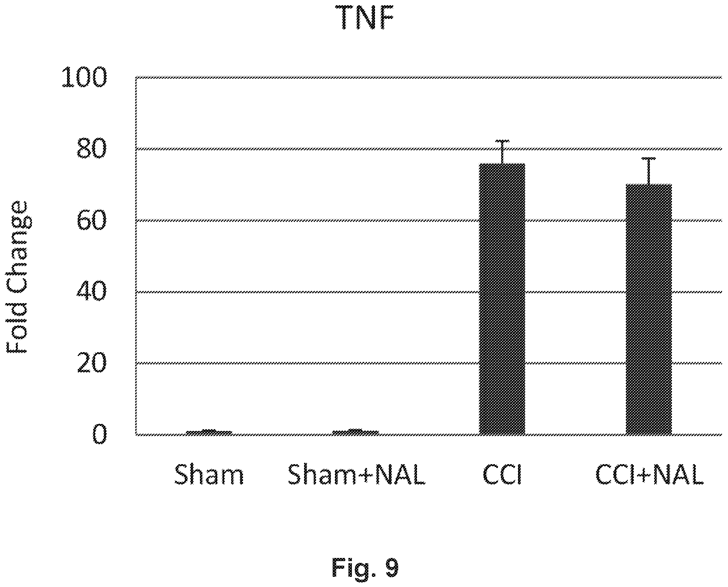

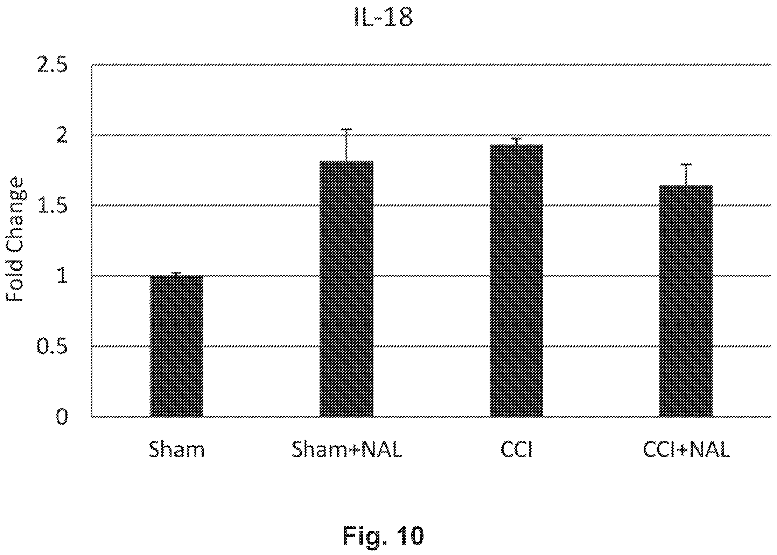

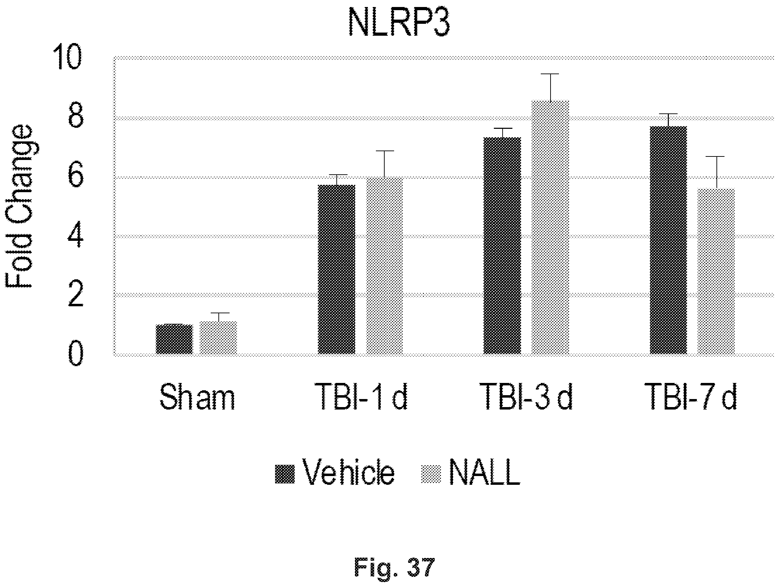

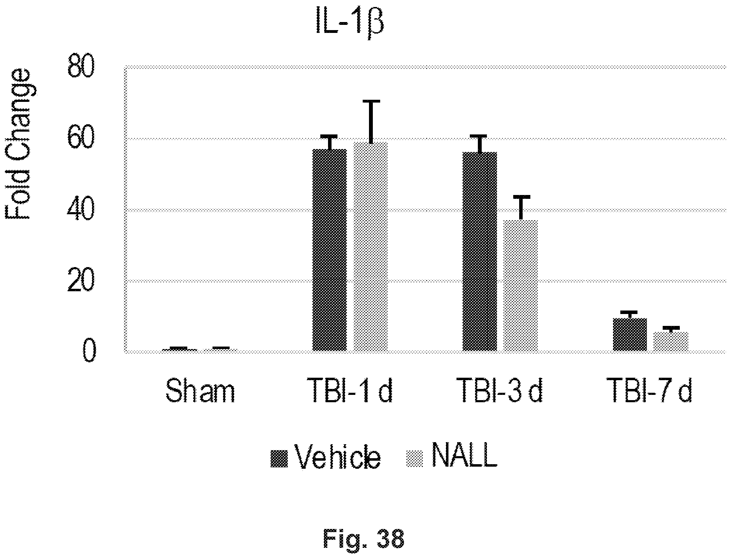

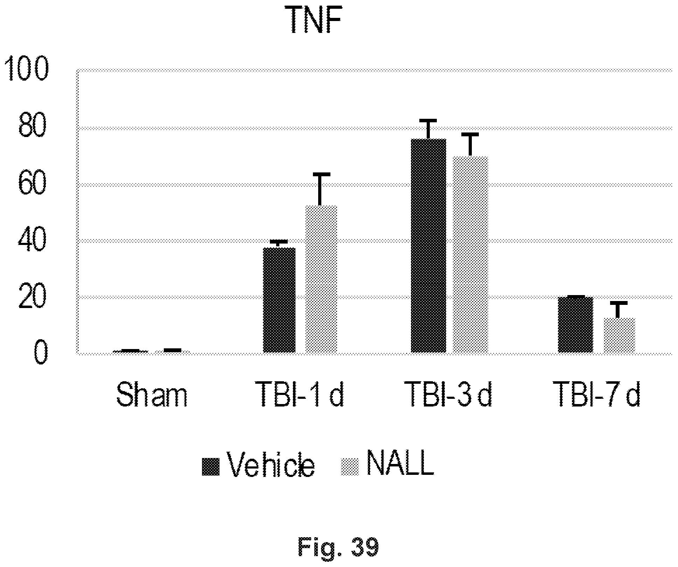

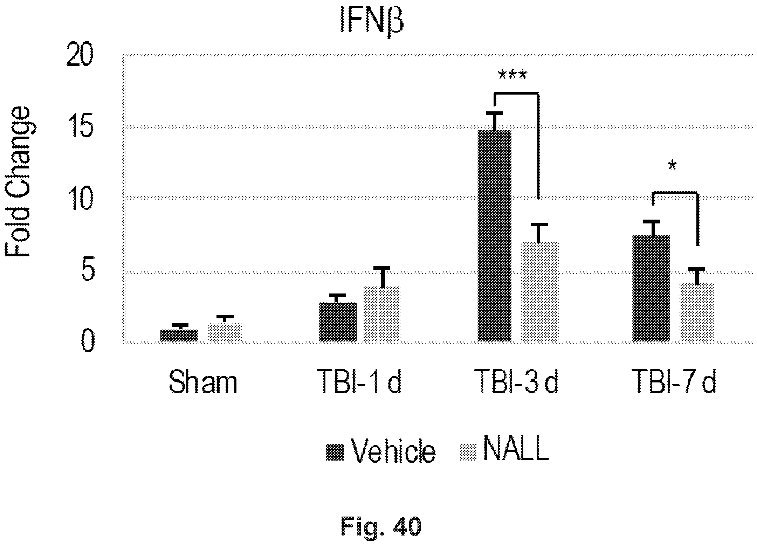

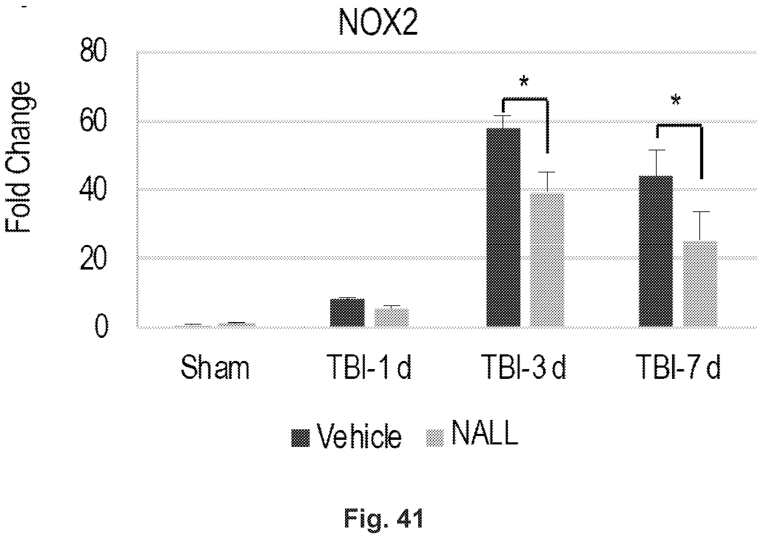

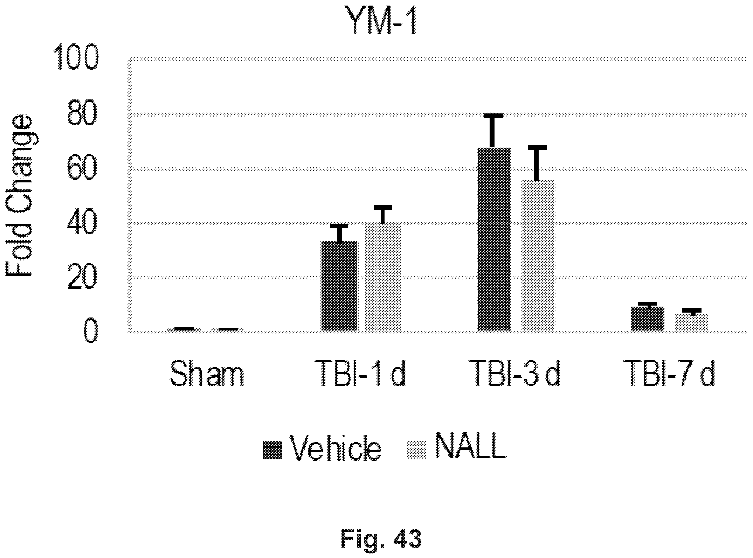

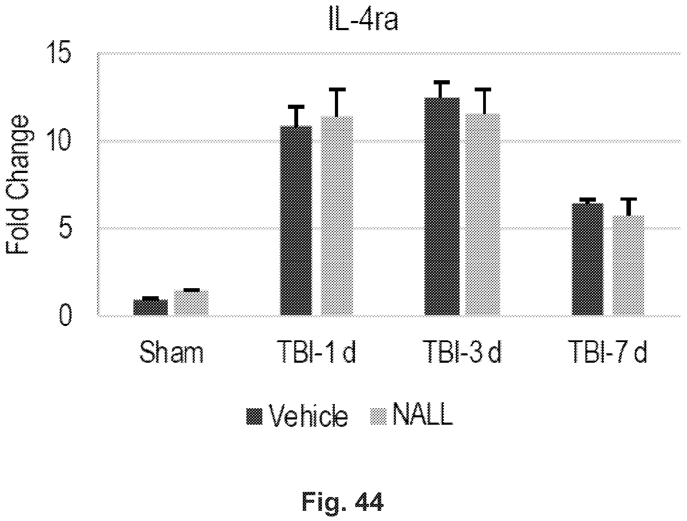

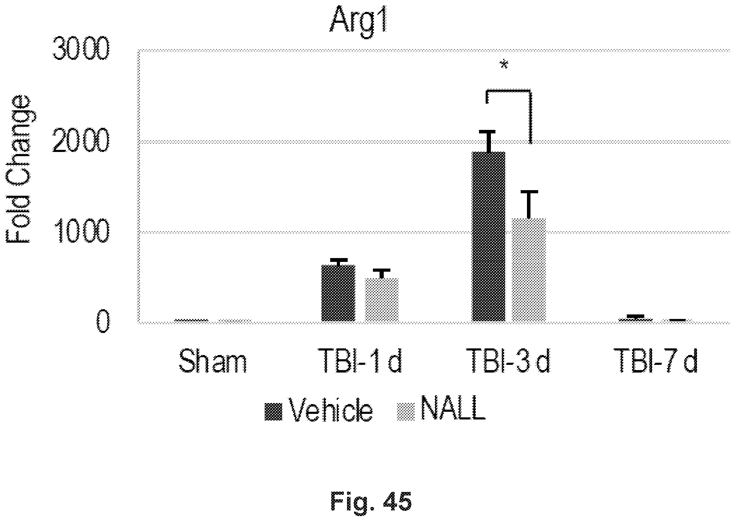

The expression level of M1-type pro-inflammatory markers Nos2, Nlrp3, IL-1β, TNF, IFNβ and Nox2 in the perilesional area in TBI mouse cortices were determined by real time rt-PCR. The expression level of M2-type markers Socs3, YM-1, IL4ra and Arg-1 in cortical tissue was also determined. The results are presented in Figs. 2-10 and 36-45.

### Real Time PCR

Total RNA isolated from the ipsilateral cortex using miRNeasy Mini Kit (Qiagen, Cat No. **217004**) was converted into cDNA using High Capacity RNA to cDNA kit (Applied Biosystem, Cat. No. 4387406) as per manufacturer's instruction. cDNA TaqMan^{®} Universal Master Mix II (Applied Biosystems, Cat. No. 4440040) was used to perform quantitative real-time PCR amplification as described previously using 20 × TaqMan^{®} Gene Expression Assay (Applied Biosystems) for following mouse genes were used: *Gapdh* (Mm99999915_g1), *NLRP3* (Mm00840904_m1), *NOX2* (Mm01287743_m1), *iNOS* (Mm00440502*_*m1*), TNFα* (Mm00443258_m1), *IFnβ1* (Mm00439552_s1), *IL-10* (Mm01288386_m1), *IL1b* (Mm00434228_m1), *Arg1* (Mm00475988_m1), *SOCS3* (Mm00545913_s1), *YM-1* (Mm00657889_mH) and*IL-4ra* (Mm01275139_m1) (Applied Biosystems). Reactions were amplified and quantified using a 7900HT Fast Real-Time PCR System and corresponding software (Applied Biosystems). Relative gene expression normalized to *Gapdh* was calculated based on the comparative Ct method.

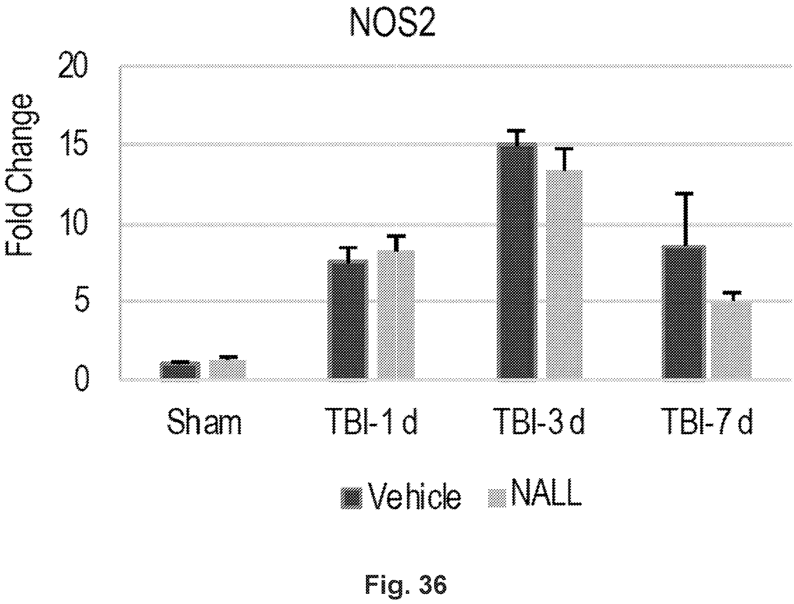

Elevated levels of all M1-type pro-inflammatory markers markers were observed, which started from day 1 and peaked at day 3 after injury in all TBI mouse cortices, irrespective of treatment. *See* Figs. 36-41. But a decrease in the mRNA level of IFNβ and Nox2 in the cortices of NAL fed mice as compared to vehicle fed TBI mouse cortices was observed.

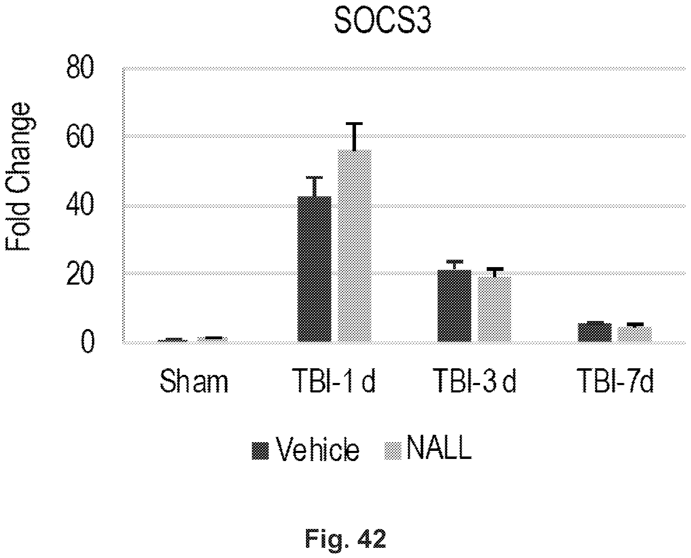

Similar to M1-type markers, a higher expression of M2-type markers Socs3, YM-1, IL4ra and Arg-1 was observed in the cortical tissue of all TBI mice as compared to sham animals. *See* Figs. 42-45. Among these markers, NAL treatment lowered the levels of Arg-1 in the injured mouse cortex as compared to vehicle fed TBI mice. The levels of Socs3, YM-1 and IL-4ra remained unaltered in NAL fed mouse cortices as compared to sham mice.

These results demonstrate that NAL reduces expression of several inflammatory markers thus indicating overall decrease in neuroinflammation following TBI in mice. Statistics

All data are presented as mean ± standard error of the mean (SEM). One-way ANOVA was performed followed by appropriate post-hoc test (Tukey's) as specified in the figure legends. For data with only two groups 2-tailed student t-test with equal variance was used. A *p* value ≤ 0.05 was considered statistically significant.

### EXAMPLE 3

### EAL flux in NPC mice

Amyloid-β deposition and accumulation of autophagic vacuoles are pathologic features of Alzheimer's disease (AD). Dysregulation of the endosomal-autophagic-lysosomal (EAL) pathway, which impairs amyloid precursor protein processing, is one of the earliest changes in AD. Xu and Ren, Annu Rev Physiol. 77:57-80 (2015). Lysosomal storage diseases such as NPC also exhibit autophagic dysfunction with pathological changes reminiscent of features seen in AD, such as dystrophic axons, ectopic dendrites, neurofibrillary tangles, and amyloid aggregation. Boland et al., The Journal of Biological Chemistry 285: 37415-37426 (2010).

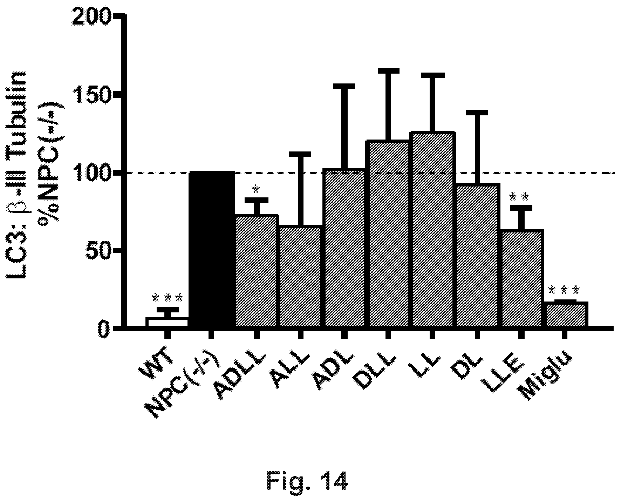

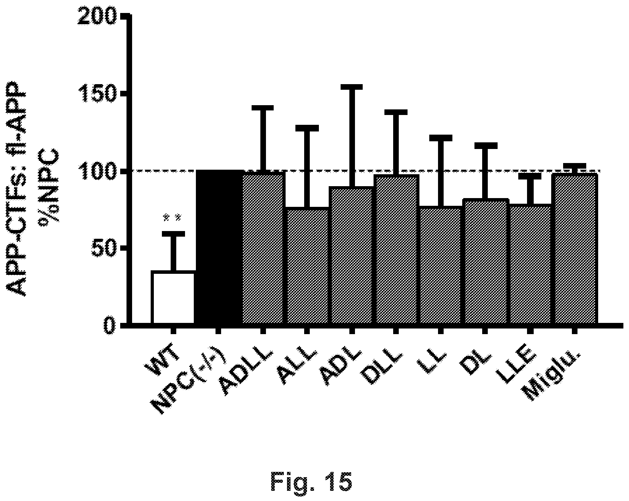

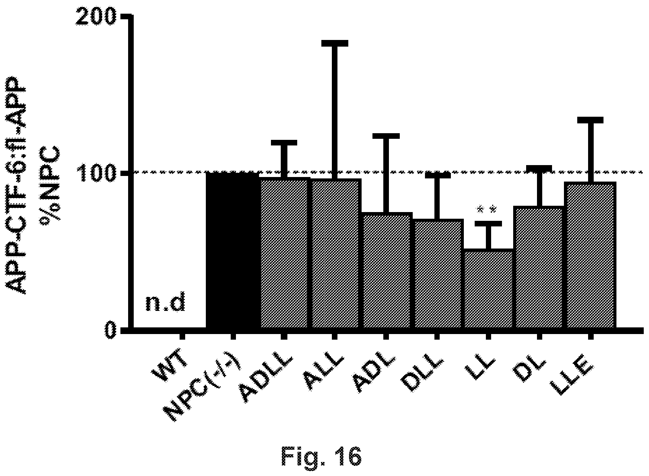

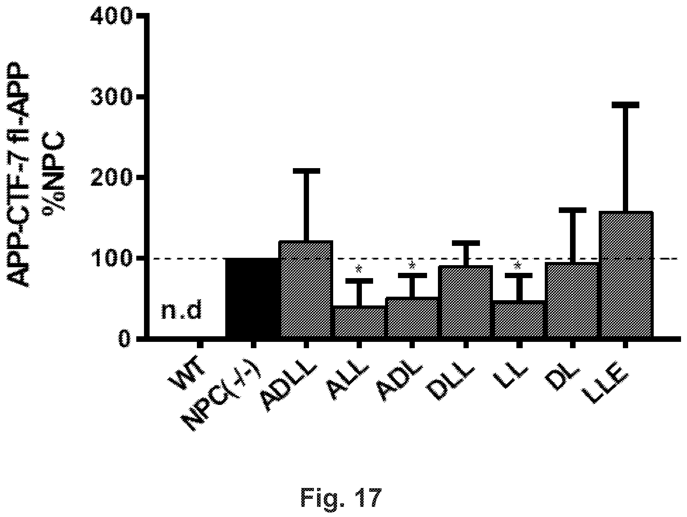

The efficacy of ADLL, ALL, ADL, DLL, LL, DL, and LLE in alleviating the impairment of EAL flux in NPC1 mice was investigated. Acetyl DL-leucine (ADLL) and ethyl ester of L-Leucine (LLE) were effective in restoring autophagic flux with respect to reducing LC3-II levels (Fig. 14). But the reduction in LC3-II levels brought about by ADLL and LLE were not accompanied by a concomitant reduction in APP-CTFs, particularly CTF-6 and -7 (Fig. 15). Without wishing to be bound by any particular theory, it is possible that ADL and LLE reduced autophagic vacuole (AV) build-up by inhibiting autophagosome formation, possibly through the activation mTORC1. Despite their ability to reduce AV accumulation, their inability to reduce elevated levels of APP-CTFs suggests that ADL and LLE do not improve lysosomal digestive capacity itself. Surprisingly, L-leucine did reduce CTF-6 accumulation in NPC1 mice (Fig. 16). Also, acetyl L-leucine (ALL), acetyl D-leucine (ADL) and L-leucine (LL) reduced CTF-7 expression, indicating some improvement in lysosomal proteolysis (Fig. 17). The positive effects of acetyl D-leucine, acetyl L-leucine and D-leucine on CTF-7clearance in NPC1 mice suggest that they may promote endosomal-lysosomal fusion without impacting on autophagosome-lysosome clearance.

### EXAMPLE 4

### Lysosomal fusion in primary cortical neurons

Neurons have a heterogeneous distribution of organelles that belong to the EAL pathway, ranging from the cell soma to distal neurites. These include autophagosomes, endosomes and amphisomes (endosomes fused to autophagosomes). U18666A is a pharmacological agent that induces a NPC1-like phenotype in cells by inhibiting the NPC1 protein and preventing endosomal-lysosomal fusion. U18666A-mediated abrogation of autophagosome-lysosome fusion and endosomal-lysosomal fusion causes an increase in LC3-II and APP-CTFs -1 to -5 while preventing the production of CTFs -6 and -7. Boland et al., The Journal of Biological Chemistry 285: 37415-37426 (2010).

The efficacy of ADLL, ALL, ADL, DLL, LL, DL, and LLE in alleviating the U18666A-mediated impairment of autophagosome-lysosome fusion and endosomal lysosomal fusion was investigated. In this cell-based assay of impaired EAL flux, the reappearance of basal levels of APP CTFs -6 and -7, provided an indication of improved endosomal-lysosomal fusion.

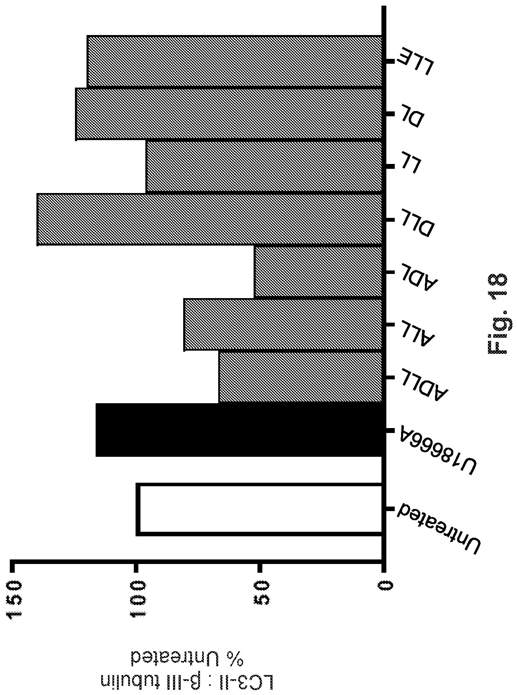

Rat primary cortical neurons (DIV14) that were treated with U18666A (2 µg/mL, 24 hr) had a complete absence of CTFs -6 and 7 (Fig. 18). U18666A treatment for a period of 24 h did not cause an increase in LC3-II accumulation (Fig. 18).

Neurons were co-treated with U18666A (2 µg/mL) along with ADLL, ALL, ADL, DLL, LL, DL, and LLE (1 mM, 24 h). All neuron cultures that were co-treated showed signs of improved endosomal-lysosomal fusion as evidenced by the reappearance of basal amounts of CTF-6 and -7 in these cells (Fig. 18). ADLL, ALL, and ADL reduced LC3-II levels lower than the basal levels (Fig. 18). This suggests that these three compounds enhanced both autophagosome-lysosome fusion endosomal-lysosomal fusion in primary cortical neurons.

### EXAMPLE 5

### Truncated forms of tau in neuron-glia co-cultures

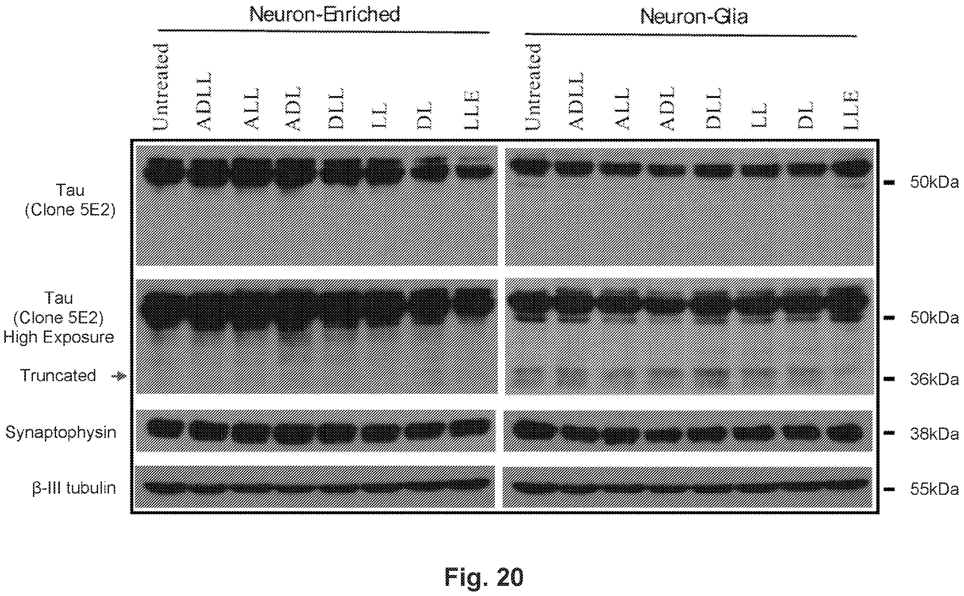

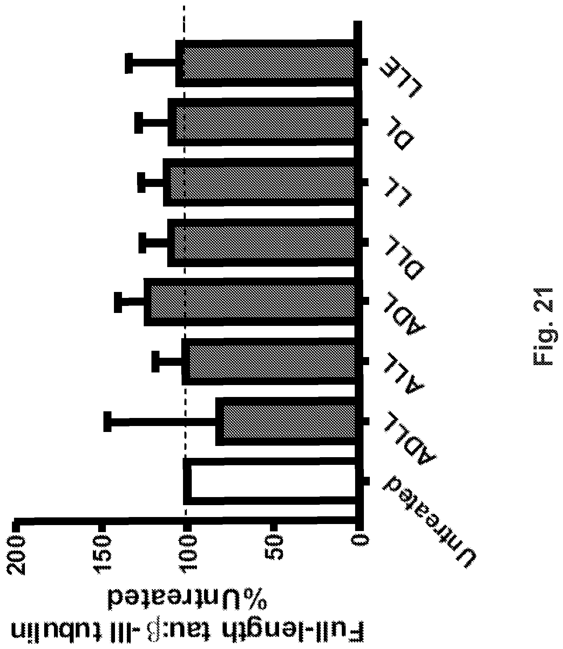

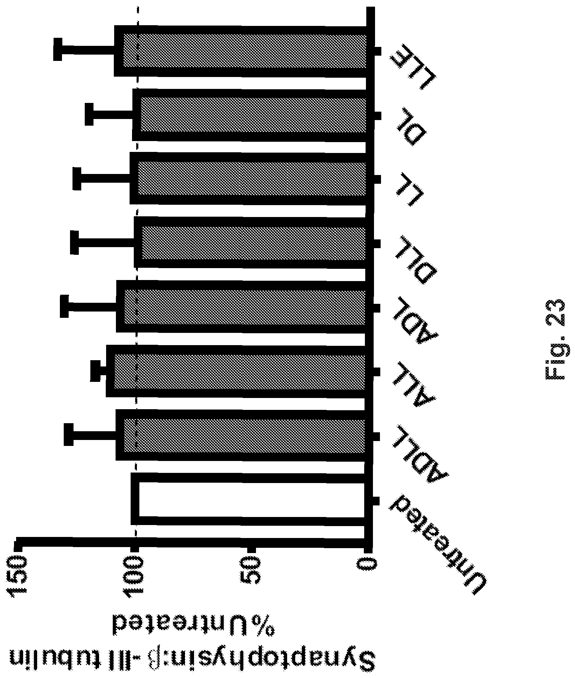

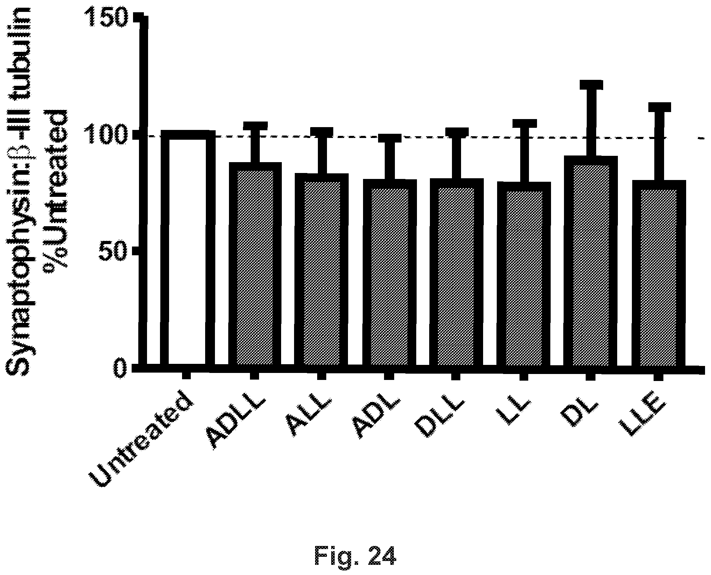

The efficacy of ADLL, ALL, ADL, DLL, LL, DL, and LLE in reducing truncated tau levels in neuron-glia (NG) cultures was investigated. The results of this study are provided in Figs. 20-24 One aim of this study was to determine whether ADLL, ALL, ADL, DLL, LL, DL, and LLE conferred intrinsic neuroprotection by sustaining neuronal function under conditions of cellular overgrowth, or whether they acted indirectly through the non-autonomous modulation of glial cells. Irrespective of whether the compounds acted directly or indirectly on neurons, preventing the generation of truncated tau confers beneficial effects to neuronal integrity.

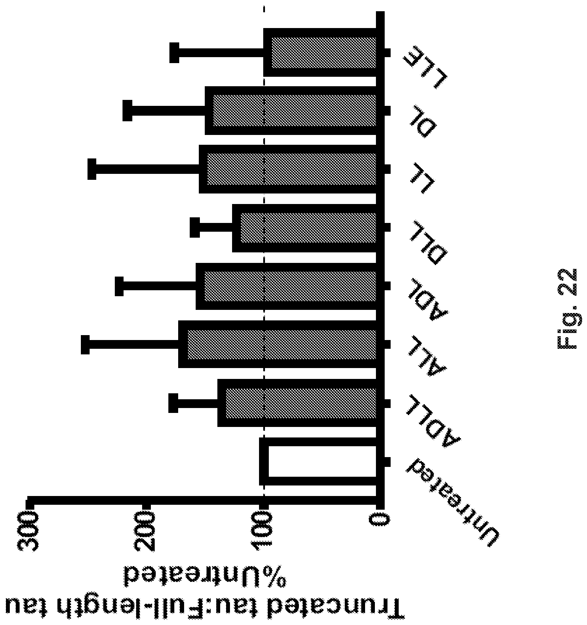

Cultures were treated for five days (DIV 9 - 14) with 1 mM of ADLL, ALL, ADL, DLL, LL, DL, or LLE, and were subsequently assessed for a number of neuron-specific markers that could provide a readout on neuronal health: βIII-tubulin, full-length tau, truncated tau (antibody 5E2) and synaptophysin. ADLL, ALL, ADL, DLL, LL, DL, and LLE were well-tolerated in NE and NG cultures, as evidenced by consistently equal levels of synaptophysin and βIII-tubulin detected across untreated (control) and drug-treated samples (Figs. 20, 23, and 24). None of ADLL, ALL, ADL, DLL, LL, DL, and LLE reduced basal levels of truncated tau in NG cultures at DIV14 (Fig. 20 and Fig. 22). These findings suggest that ADLL, ALL, ADL, DLL, LL, DL, and LLE are particularly useful for treating neurodegenerative conditions that involve substrate accumulation in the EAL network.

### EXAMPLE 6

### Lysosomal Volumes Measured by Flow Cytometry

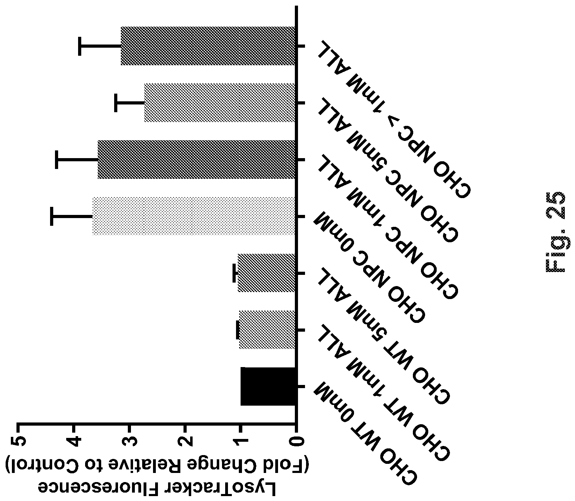

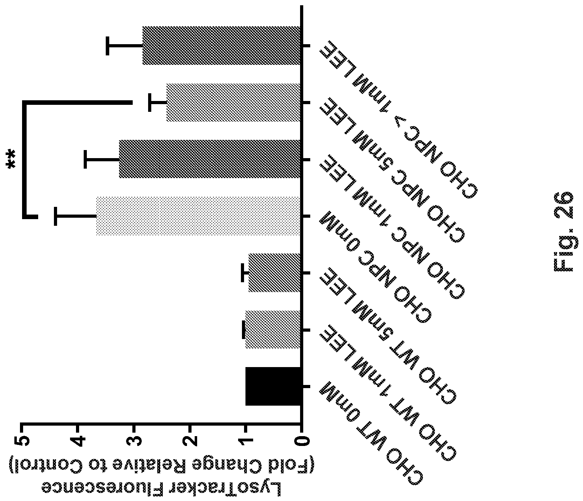

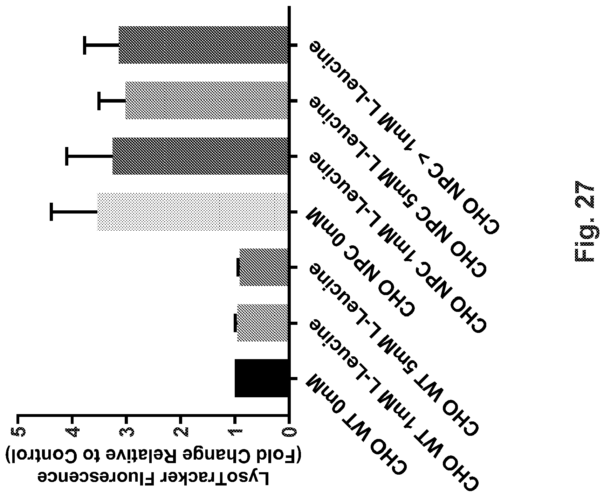

Wild type and NPC1 null Chinese Hamster Ovary (CHO) cells were grown in T75 culture flasks and treated for 7 days with N-Acetyl-L-leucine (ALL), L-Leucine ethyl ester hydrochloride (LEE), or L-leucine (1 or 5 mM purchased from Sigma-Aldrich) prior to extraction. Cells were trypsinized, centrifuged (270 × g, 5 min), washed twice with 1× PBS, centrifuged again and were stained with 100 nM LysoTracker-green DND-26 (Invitrogen) in PBS for 20 min at room temperature. Following incubation, cells were centrifuged (800 × g, 5 min), resuspended in 0.5 ml of FACS buffer (0.1% BSA, 0.02 M NaN3 in 1 × PBS) and kept on ice for flow cytometric analysis (BD Biosciences FACSCanto II or Accuri C6 Plus). The cytometer was calibrated using Cytometer Setup and Tracking beads (BD), and compensation was performed using cells stained with Lysotracker or Propidium Iodide using BD FACSDiva software (BD) or BD Accuri C6 Plus software (BD) before assay. Experiment and analysis were conducted on 10,000 recorded cells to obtain median values for further statistical analysis. The results are shown in Figs. 25-27.

### EXAMPLE 7

### Purkinje Cell Survival

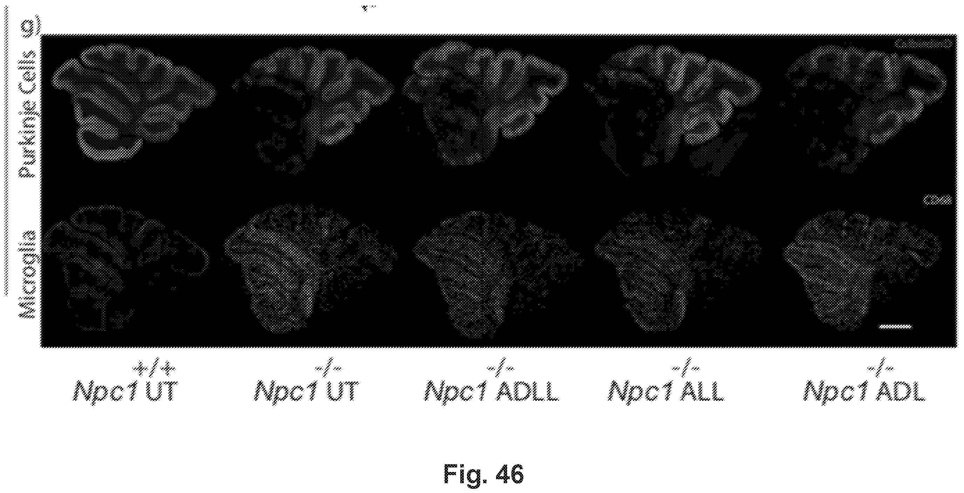

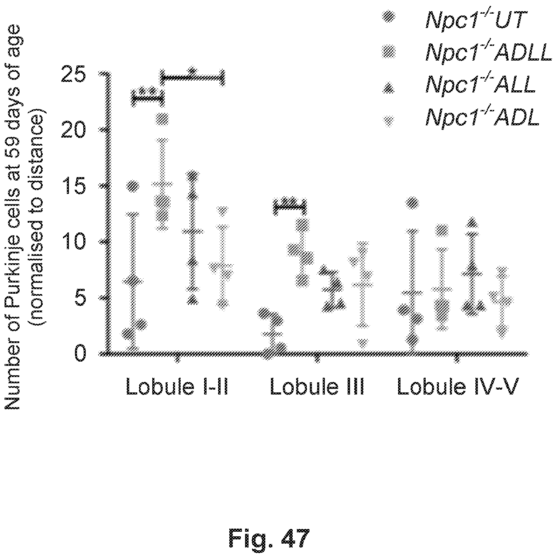

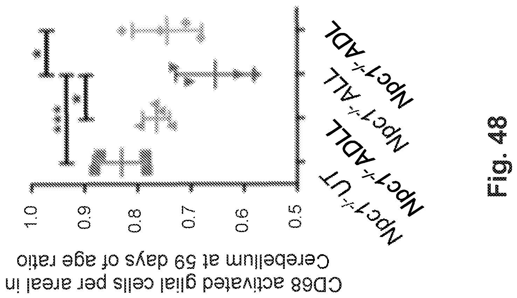

*Npc1*^{*-*/*-*} mice exhibit progressive neurodegeneration with cerebellar Purkinje cell loss progressing from anterior to posterior lobes, accompanied by microglial activation (Fig. 46). ADLL treatment increased Purkinje cell survival at 59 days of age relative to untreated *Npc1*^{*-*/*-*} littermates: 133% more Purkinje cells were present in lobules I and II (*p*=0.0027), and 402% more Purkinje cells in lobule III (*p*=0.0108). Other treatments did not improve Purkinje cell survival in any cerebellar lobules (*Npc1*^{*-*/*-*}ALL Lobule I-II *p*=0.107, Lobule III *p*=0.157, Lobule IV-V *p*=0.533; *Npc1*^{*-*/*-*}ADL Lobule I-II *p*=0.60, Lobule III *p*=0.11, Lobule IV-V *p*=0.766, compared to *Npc1*^{*-*/*-*}UT) (Fig. 46 and 47). In addition, ALL treatment reduced (by 20%, *p*=0.0177) the frequency of CD68 positive activated microglia. Other treatments did not have an impact (*p*=*0.1353 for Npc1*^{*-*/*-*}ADLL, *p*=*0.0553 for Npc1*^{*-*/*-*}ADL compared to *Npc1*^{*-*/*-*}UT) (Fig. 46 and 48). ADL did not mediate any long-term neuroprotective effects. The combination of increased survival of neurons and a less inflammatory environment in the brain could improve brain function, quality of life, and delay the progression of disease in human subjects.

### EXAMPLE 8

### Controlled Cortical Impact Model

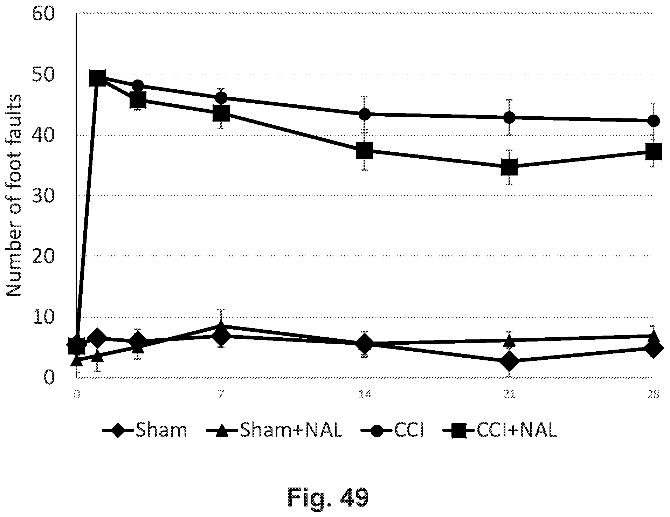

Mice were subjected to moderate controlled cortical impact TBI or sham surgery. NAL mice were treated with oral NAL starting after initial recovery after surgery (via oral gavage, daily for 4 days) and continuing for duration of experiment (in chow, ad lib). Vehicle mice received equivalent administration of oral gavage vehicle followed by standard chow. Group: Sham + Vehicle = 5 mice; Sham + NAL = 4 mice; TBI + Vehicle = 10 mice; and TBA + NAL = 9 mince. Functional outcomes were evaluated up to 28 days after surgery in the beam walk (motor function), Y-maze (spatial memory) and novel object recognition (NOR, non-spatial memory) assessments. The results are provided in Fig. 49, Fig, 50, and Fig, 51, respectively.

It is to be understood that the foregoing embodiments and exemplifications are not intended to be limiting in any respect to the scope of the disclosure, and that the claims presented herein are intended to encompass all embodiments and exemplifications whether or not explicitly presented herein.

All patents and publications cited herein are fully incorporated by reference in their entirety.

### ENUMERATED EMBODIMENTS

Some embodiments of the invention described herein can be defined according to any of the following enumerated embodiments:
1. A method of treating a disease, disorder, condition, or syndrome in a subject in need thereof, or treating a symptom of a disease, disorder, condition, or syndrome in a subject in need thereof, the method comprising administering a therapeutically effective amount of acetyl-DL-leucine, or a pharmaceutically acceptable salt thereof, acetyl-D-leucine, or a pharmaceutically acceptable salt thereof, acetyl-L-leucine, or a pharmaceutically acceptable salt thereof, or L-leucine ethyl ester, or a pharmaceutically acceptable salt thereof, to the subject, wherein the diseases, disorders, conditions, or syndromes are any one or more of the diseases, disorders, conditions, or syndromes provided in Table 1.
2. The method of embodiment 1, wherein the diseases, disorders, conditions, or syndromes are any one or more of the diseases, disorders, conditions, or syndromes provided in Table 2.
3. The method of embodiment 1, wherein the diseases, disorders, conditions, or syndromes are any one or more of the diseases, disorders, conditions, or syndromes provided in Table 3.
4. The method of embodiment 1, wherein the diseases, disorders, conditions, or syndromes are any one or more of the diseases, disorders, conditions, or syndromes provided in Table 4.
5. The method of embodiment 1, wherein the diseases, disorders, conditions, or syndromes are any one or more of the diseases, disorders, conditions, or syndromes provided in Table 5.
6. The method of embodiment 5, wherein the disease, disorder, condition, or syndrome is traumatic brain injury.
7. The method of embodiment 1, wherein the diseases, disorders, conditions, or syndromes are any one or more of the diseases, disorders, conditions, or syndromes provided in Table 6.
8. The method of embodiment 1, wherein the diseases, disorders, conditions, or syndromes are any one or more of the diseases, disorders, conditions, or syndromes provided in Table 7.
9. The method of embodiment 1, wherein the diseases, disorders, conditions, or syndromes are any one or more of the diseases, disorders, conditions, or syndromes provided in Table 8.
10. The method of embodiment 1, wherein the diseases, disorders, conditions, or syndromes are any one or more of the diseases, disorders, conditions, or syndromes provided in Table 9.
11. The method of embodiment 1, wherein the diseases, disorders, conditions, or syndromes are any one or more of the diseases, disorders, conditions, or syndromes provided in Table 10.
12. The method of embodiment 1, wherein the diseases, disorders, conditions, or syndromes are any one or more of the diseases, disorders, conditions, or syndromes provided in Table 11.
13. The method of embodiment 1, wherein the diseases, disorders, conditions, or syndromes are any one or more of the diseases, disorders, conditions, or syndromes provided in Table 12.
14. The method of embodiment 1, wherein the diseases, disorders, conditions, or syndromes are any one or more of the diseases, disorders, conditions, or syndromes provided in Table 13.
15. The method of embodiment 1, wherein the diseases, disorders, conditions, or syndromes are any one or more of the diseases, disorders, conditions, or syndromes provided in Table 14.
16. The method of embodiment 1, wherein the diseases, disorders, conditions, or syndromes are any one or more of the diseases, disorders, conditions, or syndromes provided in Table 15.
17. The method of embodiment 1, wherein the diseases, disorders, conditions, or syndromes are any one or more of the diseases, disorders, conditions, or syndromes provided in Table 16.
18. The method of embodiment 1, wherein the diseases, disorders, conditions, or syndromes are any one or more of the diseases, disorders, conditions, or syndromes provided in Table 17.
19. The method of any one of embodiments 1-18, wherein about 1 g to about 30 g of acetyl-DL-leucine, or a pharmaceutically acceptable salt thereof, acetyl-D-leucine, or a pharmaceutically acceptable salt thereof, acetyl-L-leucine, or a pharmaceutically acceptable salt thereof, or L-leucine ethyl ester, or a pharmaceutically acceptable salt thereof, is administered to the subject per day.
20. The method of embodiment 19, wherein about 2 g to about 15 g of acetyl-DL-leucine, or a pharmaceutically acceptable salt thereof, acetyl-D-leucine, or a pharmaceutically acceptable salt thereof, acetyl-L-leucine, or a pharmaceutically acceptable salt thereof, or L-leucine ethyl ester, or a pharmaceutically acceptable salt thereof, is administered to the subject per day.
21. The method of embodiment 20, wherein about 3 g to about 10 g of acetyl-DL-leucine, or a pharmaceutically acceptable salt thereof, acetyl-D-leucine, or a pharmaceutically acceptable salt thereof, acetyl-L-leucine, or a pharmaceutically acceptable salt thereof, or L-leucine ethyl ester, or a pharmaceutically acceptable salt thereof, is administered to the subject per day.
22. The method of embodiment 21, wherein about 4 g to about 8 g of acetyl-DL-leucine, or a pharmaceutically acceptable salt thereof, acetyl-D-leucine, or a pharmaceutically acceptable salt thereof, or acetyl-L-leucine, or a pharmaceutically acceptable salt thereof, or L-leucine ethyl ester, or a pharmaceutically acceptable salt thereof, is administered to the subject per day.
23. The method of embodiment 22, wherein about 4 g to about 5 g of acetyl-DL-leucine, or a pharmaceutically acceptable salt thereof, acetyl-D-leucine, or a pharmaceutically acceptable salt thereof, acetyl-L-leucine, or a pharmaceutically acceptable salt thereof, or L-leucine ethyl ester, or a pharmaceutically acceptable salt thereof, is administered to the subject per day.
24. The method of embodiment 23, wherein about 5 g of acetyl-DL-leucine, or a pharmaceutically acceptable salt thereof, acetyl-D-leucine, or a pharmaceutically acceptable salt thereof, or acetyl-L-leucine, or a pharmaceutically acceptable salt thereof, or L-leucine ethyl ester, or a pharmaceutically acceptable salt thereof, is administered to the subject per day.
25. The method of any one of embodiments 1-24, wherein a therapeutically effective amount of L-leucine ethyl ester, or a pharmaceutically acceptable salt thereof, is administered to the subject.
26. The method of embodiment 25, wherein a therapeutically effective amount of L-leucine ethyl ester, or a pharmaceutically acceptable salt thereof, is administered to the subject to treat the disease, disorder, condition, or syndrome.
27. The method of embodiment 25, wherein a therapeutically effective amount of L-leucine ethyl ester, or a pharmaceutically acceptable salt thereof, is administered to the subject to treat the symptom of the disease, disorder, condition, or syndrome.
28. The method of any one of embodiments 1-24, wherein a therapeutically effective amount of acetyl-DL-leucine, or a pharmaceutically acceptable salt thereof, is administered to the subj ect.
29. The method of embodiment 28, wherein a therapeutically effective amount of acetyl-DL-leucine, or a pharmaceutically acceptable salt thereof, is administered to the subject to treat the disease, disorder, condition, or syndrome.
30. The method of embodiment 28, wherein a therapeutically effective amount of acetyl-DL-leucine, or a pharmaceutically acceptable salt thereof, is administered to the subject to treat the symptom of the disease, disorder, condition, or syndrome.
31. The method of any one of embodiments 1-24, wherein a therapeutically effective amount of acetyl-L-leucine, or a pharmaceutically acceptable salt thereof, is administered to the subj ect.
32. The method of embodiment 31, wherein a therapeutically effective amount of acetyl-L-leucine, or a pharmaceutically acceptable salt thereof, is administered to the subject to treat the disease, disorder, condition, or syndrome.
33. The method of embodiment 31, wherein a therapeutically effective amount of acetyl-L-leucine, or a pharmaceutically acceptable salt thereof, is administered to the subject to treat the symptom of the disease, disorder, condition, or syndrome.
34. The method of any one of embodiments 1-24, wherein a therapeutically effective amount of acetyl-D-leucine, or a pharmaceutically acceptable salt thereof, is administered to the subj ect.
35. The method of embodiment 34, wherein a therapeutically effective amount of acetyl-D-leucine, or a pharmaceutically acceptable salt thereof, is administered to the subject to treat the disease, disorder, condition, or syndrome.
36. The method of embodiment 34, wherein a therapeutically effective amount of acetyl-D-leucine, or a pharmaceutically acceptable salt thereof, is administered to the subject to treat the symptom of the disease, disorder, condition, or syndrome.
37. A method of modulating the expression of one or more pro-inflammatory mediators in a subject in a subject in need thereof, the method comprising administering a therapeutically effective amount of acetyl-DL-leucine, or a pharmaceutically acceptable salt thereof, acetyl-D-leucine, or a pharmaceutically acceptable salt thereof, or acetyl-L-leucine, or a pharmaceutically acceptable salt thereof, to the subject.
38. The method of embodiment 37, wherein the one or more pro-inflammatory mediators are NOS2, IL-18, IFNb, IL-1β, TNFα, NOX2, NLRP3, SOCS3, ARG1, IL-10, IL-4ra, or YM1.

## Claims

1. Acetyl-L-leucine, or a pharmaceutically acceptable salt thereof, for use in a method of treating one or more symptoms associated with traumatic brain injury in a subject in need thereof, the method comprising administering a therapeutically effective amount of acetyl-L-leucine, or a pharmaceutically acceptable salt thereof, to the subject, wherein about 1 g to about 30 g of acetyl-L-leucine, or a pharmaceutically acceptable salt thereof, is administered to the subject per day, wherein the one or more symptoms associated with traumatic brain injury comprise a neuroinflammatory, neurological, autoimmune, neurodegenerative proteinopathic, psychiatric, and mitochondrial diseases, disorders, conditions, or syndrome.

2. Acetyl-L-leucine, or a pharmaceutically acceptable salt thereof, for use of claim 1, wherein the one or more symptoms associated with or resulting from traumatic brain injury is aortic medial amyloid, apathy, attention deficit disorder (ADD), attention deficit hyperactivity disorder (ADHD), autism, autism spectrum disorder (ASD), bipolar disorder, brain inflammation, brain lesions, catatonia, cerebral amyloid angiopathy, cerebral haemorrhage with amyloidosis, cerebral palsy, cerebrovascular disease, chorea in children and adults, complications from intracerebral hemorrhage, complications post anoxia, concussions, conus and cauda equina medullaris syndromes, dementia, depression, diabetes mellitus type 1, diffuse Lewy body disease, dopamine responsive dystonia, drop foot, dyslexia, Emory Dreifuss muscular dystrophy, encephalitis (infectious encephalitis or autoimmune encephalitis), Epilepsy, frontal lobe syndrome, frontotemporal lobar degeneration, generalized anxiety, hydrocephalus, hyperalgesia or sensory disorders, hypercalcemia, hyperkinesias, hypoglycemia, hypothyroidism, depression, malnutrition, mania, mild cognitive impairment, mitochondrial disorders, mitochondrial encephalomyopathy with lactic acidosis and stroke-like episodes (MELAS), Moyamoya disease, multi-infarct dementia, nerve trauma, neurodegeneration with brain iron accumulation, neuroinflammation, neuropsychiatric disorders, pain, panic disorder, pantothenate kinase associated neurodegeneration, Pick's disease, post-traumatic stress disorder, progressive bulbar palsy, progressive muscular atrophy, schizophrenia, social phobia, stroke, tardive dyskinesia, Tourette's syndrome, transverse myelitis, traumatic brain injury, vascular amyloidosis, vascular dementia, or vasculitis.

3. Acetyl-L-leucine, or a pharmaceutically acceptable salt thereof, for use of claim 1 or 2, wherein the disease is Tourette's syndrome.

4. Acetyl-L-leucine, or a pharmaceutically acceptable salt thereof, for use of claim 1 or 2, wherein the disease is attention deficit hyperactivity disorder (ADHD).

5. Acetyl-L-leucine, or a pharmaceutically acceptable salt thereof, for use in a method of modulating the expression of one or more pro-inflammatory mediators in a subject with neuroinflammation, the method comprising administering a therapeutically effective amount of acetyl-L-leucine, or a pharmaceutically acceptable salt thereof, to the subject.

6. Acetyl-L-leucine, or a pharmaceutically acceptable salt thereof, for use of claim 5, wherein the subject has increased expression of one or more pro-inflammatory mediators compared to a subject without neuroinflammation.

7. Acetyl-L-leucine, or a pharmaceutically acceptable salt thereof, for use of claims 5 or 6, wherein the one or more pro-inflammatory mediators are NOS2, IL-18, IFN-β, IL-1β, TNFα, NOX2, NLRP3, SOCS3, ARGI, IL-10, IL-4ra, and/or YM-1.

8. Acetyl-L-leucine, or a pharmaceutically acceptable salt thereof, for use of claims any one of claims 5-7, wherein the one or more pro-inflammatory mediators are IL-1β, IL-6, and/or TNFα.

9. Acetyl-L-leucine, or a pharmaceutically acceptable salt thereof, for use of any one of claims 5-8, wherein the neuroinflammation is associated with traumatic brain injury, Alzheimer's disease, Multiple sclerosis, Parkinson's Disease, and/or ALS.

10. Acetyl-L-leucine, or a pharmaceutically acceptable salt thereof, for use of any one of claims 5-9, wherein expression of the one or more pro-inflammatory mediators is decreased after administration of acetyl-L-leucine, or a pharmaceutically acceptable salt thereof.

11. Acetyl-L-leucine, or a pharmaceutically acceptable salt thereof, for use of claim 10, wherein the one or more pro-inflammatory mediators are IFN-β, Nox2, and/or Arg-1.

12. Acetyl-L-leucine, or a pharmaceutically acceptable salt thereof, for use of any one of claims 2-11, wherein about 1 g to about 30 g of acetyl-L-leucine, or a pharmaceutically acceptable salt thereof, is administered to the subject per day, preferably wherein about 2 g to about 15 g, about 3 g to about 10 g, about 4 g to about 8 g, about 4 g to about 5 g, or about 5 g of acetyl-L-leucine, or a pharmaceutically acceptable salt thereof, is administered to the subject per day.

13. Acetyl-L-leucine, or a pharmaceutically acceptable salt thereof, for use of any one of claims 1-12, wherein a therapeutically effective amount of acetyl-L-leucine, or a pharmaceutically acceptable salt thereof, is administered to the subject to treat the disease.

14. Acetyl-L-leucine, or a pharmaceutically acceptable salt thereof, for use of any one of claims 1-13, wherein a therapeutically effective amount of acetyl-L-leucine, or a pharmaceutically acceptable salt thereof, is administered to the subject to treat the symptom of the disease.
